# FASCICULE DE BREVET EUROPEEN

(11) **EP 0 518 960 B1**
(45) Date de publication et mention de la délivrance du brevet: **14.09.1994**
(21) Numéro de dépôt: 91905832.1
(22) Date de dépôt: 05.03.1991
(51) Int. Cl.: C07K 5/06, A61K 37/02

(54) **DERIVES DE N-PHENYL GLYCINAMIDE, LEUR PREPARATION ET LES MEDICAMENTS LES CONTENANT**
N-PHENYLGLYCINDERIVATE, IHRE HERSTELLUNG UND MEDIKAMENTE, DIE SIE ENTHALTEN
DERIVATIVES OF N-PHENYL GLYCINAMIDE, THEIR PREPARATION AND MEDICAMENTS CONTAINING THEM

(30) Priorité: 07.03.1990 FR 9002889; 16.10.1990 FR 9012727
(43) Date de publication de la demande: 23.12.1992
(73) Titulaire: RHONE-POULENC RORER S.A., 92165 Antony Cédex (FR)
(72) Inventeur: BOURZAT, Jean-Dominique, F-94300 Vincennes (FR); CAPET, Marc, F-94320 Thiais (FR); COTREL, Claude, F-75012 Paris (FR); GUYON, Claude, F-94100 Saint-Maur-des-Fossés (FR); MANFRE, Franco, F-94400 Vitry-sur-Seine (FR); ROUSSEL, Gérard, F-91450 Soisy-sur-Seine (FR)
(74) Mandataire: Pilard, Jacques (FR)
(86) Numéro de dépôt international: FR9100174
(87) Numéro de publication internationale: WO9113907

(56) Documents cités:
- EP-A- 0 166 355
- EP-A- 0 175 498
- EP-A- 0 397 556

## Description

La présente invention concerne des dérivés de N-phényl glycinamide de formule : 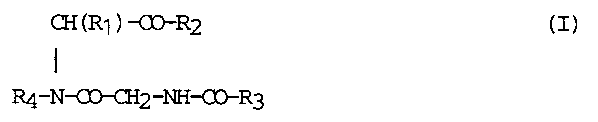
leur préparation et les médicaments les contenant.

Des antagonistes de la cholécystokinine sont décrits dans les brevets EP 397 556, EP 166 355 et EP 175 498.

Dans la formule (I),
- R₁ représente un atome d'hydrogène, un radical alkyle, alcoxycarbonyle ou phényle (éventuellement substitué par un ou plusieurs substituants choisis parmi les atomes d'halogène et les radicaux alkyle, alcoxy, alkylthio, nitro et amino),
- R₂ représente un radical alcoxy, cycloalkyloxy (éventuellement substitué par au moins un radical alkyle), cycloalkylalkyloxy, phénylalkyloxy, polyfluoroalkyloxy, cinnamyloxy, ou -NR₅R₆,
- R₃ représente un radical phénylamino (dont le noyau phényle est éventuellement substitué par un ou plusieurs substituants choisis parmi les atomes d'halogène et les radicaux alkyle, alcoxy, alkylthio, carboxy, hydroxy, mono ou polyhydroxyalkyle, nitro, amino, acyle, cyano, sulfamoyle, carbamoyle, benzoyle, alcoxycarbonyle, phénylhydroxyméthyle, pipéridino, hydroxyiminoalkyle, alcoxyiminoalkyle, alkylsulfinyle, tétrazolyl-5, tétrazolyl-5 alkyle, sulfo, -alk-O-CO-alk, -alk-O-alk, -alk-COOX, -O-alk-COOX, -alk'-COOX, -CH=CH-COOX, -CO-COOX, -alk-SO₃H et -S-alk-COOX), phényle (éventuellement substitué par un ou plusieurs substituants choisis parmi les atomes d'halogène et les radicaus alkyle, alcoxy et alkylthio), naphtyle, indolyle ou quinolyle,
- R₄ représente un radical phényle substitué par un ou plusieurs substituants choisis parmi les atomes d'halogène et les radicaux alkyle, alcoxy, hydroxy, trifluorométhyle, nitro, alkylthio, alcoxycarbonyle, carboxy, acylamino, méthylènedioxy, trifluorométhoxy, trifluorométhylthio, phénoxy, phényle, benzyle, phénylamino et -CO-NR₁₂R₁₃,
- R₅ et R₆ identiques ou différents représentent un atome d'hydrogène, un radical alkyle, phényle (éventuellement substitué par un ou plusieurs substituants choisis parmi les atomes d'halogène et les radicaux alkyle, alcoxy et alkylthio), indanyle, cycloalkylalkyle, cycloalkyle, phénylalkyle ou bien R₅ et R₆ forment ensemble avec l'atome d'azote auquel ils sont rattachés un hétérocycle mono polycyclique saturé ou insaturé contentant 4 à 9 atomes de carbone et un ou plusieurs hétéroatomes (O,N,S) et éventuellement substitué par un ou plusieurs radicaux alkyle, alcoxy, alcoxycarbonyle, dialkylcarbamoyle, phényle ou en combinaison avec un atome de carbone de l'hétérocycle par un cycle spiromonocyclique à 4 ou 5 chaînons et contenant éventuellement un ou plusieurs hétéroatomes (O,S,N),
- R₁₂ représente un atome d'hydrogène ou un radical alkyle,
- R₁₃ représente un radical phényle,
ou bien NR₁₂R₁₃ représente un radical pipéridino éventuellement substitué par un radical alkyle, tétrahydro-1,2,3,4 quinolyl-1, pyrrolidinyl-1 éventuellement substitué par au moins un radical phényle ou pipérazinyl-1 éventuellement substitué en -4 par un radical alkyle,
- alk représente un radical alkyle ou alkylène,
- alk' représente un radical hydroxyalkylène,
- X représente un atome d'hydrogène ou un radical alkyle.

Dans les définitions qui précèdent et celles qui seront citées ci-après, sauf mention contraire, les radicaux alkyle, alkylène et alcoxy et les portions alkyle, alkylène et alcoxy contiennent 1 à 4 atomes de carbone en chaîne droite ou ramifiée, les radicaux et portions cycloalkyle contiennent 3 à 6 atomes de carbone et les radicaux acyle contiennent 2 à 4 atomes de carbone.

Dans la formule (I), les atomes d'halogène sont de préférence des atomes de chlore, de brome et de fluor.

Lorsque R₅ et R₆ forment ensemble avec l'atome d'azote auquel ils sont rattachés un hétérocycle celui-ci est de préférence un cycle pipéridino (éventuellement substitué par au moins un radical alkyle, phényle, alcoxycarbonyle ou dialkylcarbamoyle), un cycle perhydroazépinyl-1, indolinyl-1, tétrahydro-1,2,3,6 pyridyl-1, tétrahydro-1,2,3,4 quinolyl-1, pyrrolidinyl-1, dihydro-3,4 2H-benzoxazine-1,4 yl-4, dihydro-3,4 2H-benzothiazine-1,4 yl-4, N-alkyl tétrahydro-1,2,3,4 quinoxalinyl-1, perhydroquinolyl-1, tétrahydro-1,2,3,4 isoquinolyl-2, aza-8 spiro [4,5] décanyl-8, aza-8 dioxa-1,4 spiro [4,5] décanyl-8, phényl-2 ou -3 pyrrolidinyl-1 ou thiomorpholino (éventuellement substitué par au moins un radical alkyle).

Les composés de formule (I) contentant un ou plusieurs centres asymétriques présentent des formes isomères. Les racémiques et les énantiomères de ces composés font également partie de l'invention.

Les composés de formule (I) pour lesquels R₃ représente un radical phénylamino (dont le noyau phényle est éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, -alk-O-alk, tétrazolyl-5 et tétrazolyl-5 alkyle) peuvent être obtenus par action d'un isocyanate de formule :

OCN - R₉ (II)

dans laquelle R₉ représente un radical phényle (éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, -alk-O-alk, tètrazolyl-5 et tétrazolyl-5 alkyle) sur un dérivé aminé de formule : 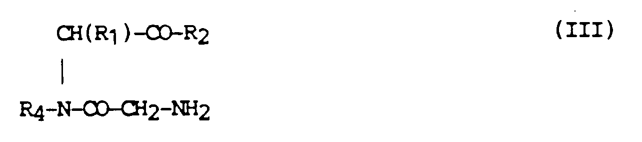
dans laquelle R₁, R₂ et R₄ ont les mêmes significations que dans la formule (I).

Cette réaction s'effectue généralement au sein d'un solvant inerte tel que le tétrahydrofuranne, le diméthylformamide, un solvant chloré (chloroforme, dichloro-1,2 éthane par exemple), un solvant aromatique (benzène, toluène par exemple), à une température comprise entre 10°C et la température d'ébullition du solvant.

Les isocyanates de formule (II) sont commercialisés ou décrite par R. RICHTER et coll., The Chemistry of Cyanate and their thio derivatives, S. PATAI, part 2, Wiley New York (1977).

Les dérivés de formule (III) peuvent être obtenus par applications ou adaptation de la méthode décrite dans les exemples ou de la méthode décrite par T. WIELAND et coll., Justus Liebigs Ann. Chem., 613, 84 (1958) ou par adaptation de la méthode de GABRIEL (GIBSON et coll., Angew Chem. Int. Ed., 7, 919 (1968) qui consiste à faire réagir une hydrazine de formule :

H₂N-NH R₁₀ (IV)

dans laquelle R₁₀ représente un atome d'hydrogène ou un radical méthyle, sur un dérivé de formule : 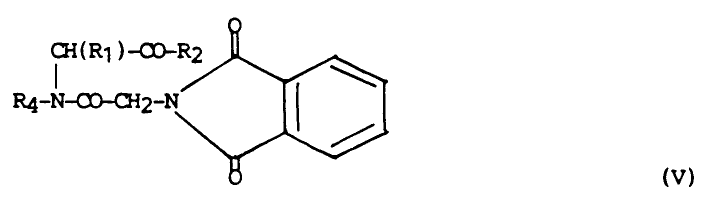
dans laquelle R₁, R₂ et R₄ ont les mêmes significations que dans la formule (I).

Cette réaction s'effectue de préférence au sein d'un solvant inerte tel qu'un alcool (méthanol, éthanol par exemple) ou un solvant chloré (dichlorométhane, chloroforme par exemple), à une température comprise entre 0°C et la température d'ébullition du solvant.

Les dérivés de formule (V) peuvent être obtenus par action d'une amine de formule :

R₄-NH-CH(R₁)-CO-R₂ (VI)

dans laquelle R₁, R₂ et R₄ ont les mêmes significations que dans la formule (I), sur le chlorure de phtalimido-2 acétyle.

Cette réaction s'effectue généralement au sein d'un solvant inerte (chloroforme, dichloro-1, 2 éthane par exemple), en présence d'une base telle qu'une amine tertiaire comme une trialkylamine, un carbonate ou un bicarbonate de métal alcalin, à une température voisine de 20°C.

Le chlorure de phtalimido-2 acétyle peut être préparé par application de la méthode décrite par W. GRASSMANN et coll., Chem. Ber., 83, 244 (1950).

Les amines de formule (VI) peuvent être obtenues par action d'un dérivé aminé de formule :

R₄-NH₂ (VII)

dans laquelle R₄ a les mêmes significations que dans la formule (I) sur un dérivé halogéné de formule :

Hal-CH(R₁)-CO-R₂ (VIII)

dans laquelle R₁ et R₂ ont les mêmes significations que dans la formule (I) et Hal représente un atome d'halogène (chlore ou brome de préférence).

Cette réaction s'effectue généralement au sein d'un solvant inerte tel que l'acétonitrile, le diméthylformamide, le tétrahyrofuranne ou un solvant chloré, éventuellement en présence d'une base telle qu'un hydrure de métal alcalin ou un bicarbonate de métal alcalin, à la température d'ébullition du solvant.

Les anilines substituées de formule (VII) peuvent être obtenues par application ou adaptation de la méthode décrite par R. SCHRÖTER, Methoden der Organischen Chemie, Houben Weil, Band XI/1, p. 360.

Les dérivés halogénés de formule (VIII) peuvent être obtenus par halogénation d'un dérivé de formule :

HCH(R₁)-CO-R₂ (IX)

dans laquelle R₁ et R₂ ont les mêmes significations que dans la formule (I).

Cette réaction s'effectue généralement au moyen de brome ou de chlore éventuellement en présence d'acétamide.

Les dérivés de formule (IX) pour lesquels R₂ représente un radical alcoxy, cycloalkyloxy éventuellement substitué, cycloalkylalcoxy, phénylalkloxy, polyfluoroalkyloxy ou cinnamyloxy peuvent être obtenus par estérification d'un acide de formule :

HCH(R₁)-COOH (X)

dans laquelle R₁ a les mêmes significations que dans la formule (I).

Cette estérification s'effectue par toute méthode connue de l'homme du métier pour passer d'un acide à un ester. On peut par exemple fair réagir l'alcool correspondant en présence d'un acide tel que l'acide sulfurique.

Les acides de formule (X) pour lesquels R₁ représente un radical alcoxycarbonyle peuvent être obtenus par application ou adaptation de la méthode décrite dans Acta. Chem. Scand., B29, 687 (1975).

Les dérivés de formule (IX) pour lesquels R₂ représente un radical -NR₅R₆ peuvent être obtenus par action d'un acide de formule (X) ou un dérivé réactif de cet acide, sur une amine de formule :

HNR₅R₆ (XI)

dans laquelle R₅ et R₆ ont les mêmes significations que dans la formule (I).

Lorsque l'on met en oeuvre l'acide on opère en présence d'un agent de condensation peptidique tel qu'un carbodiimide (par exemple le dicyclohexylcarbodiimide) ou le N,N'-diimidazole carbonyle, dans un solvant inerte tel qu'un éther (tétrahydrofuranne, dioxanne par exemple), un amide (diméthylformamide par exemple) ou un solvant chloré (chlorure de méthylène, dichloro-1,2 éthane, chloroforme par exemple) à une température comprise entre 0°C et la température de reflux mélange réactionnel.

Lorsque l'on met en oeuvre en dérivé réactif de l'acide, il est possible de faire réagir l'anhydride, un anhydride mixte ou un halogènure d'acide ou un ester (qui peur être choisi parmi les esters activés ou non de l'acide).

On opère alors soit en milieu organique éventuellement en présence d'un accepteur d'acide tel qu'une base organique azotée (trialkylamine, pyridine, diaza-1,8 bicyclo[5.4.0] undécène-7 ou dizaz-1,5 bicyclo[4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les dérivés de formule (V) peuvent également être obtenus par action d'un dérivé de formule (VIII) sur un dérivé de formule : 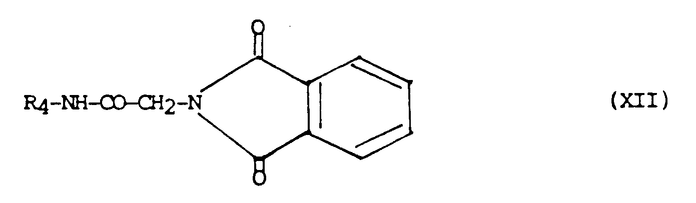
dans laquelle R₄ a les mêmes significations que dans la formule (I).

Cette réaction s'effectue généralement au sein d'un solvant inerte tel que l'acétonitrile, le diméthylformamide ou le tétrahydrofuranne, en présence d'une base telle qu'un hydrure de métal alcalin ou d'un carbonate ou bicarbonate d'un métal alcalin ou alcalino-terreux, à une température comprise entre 15°C et la température de reflux du milieu réactionnel.

Les dérivés de formule (XII) peuvent être obtenus par action d'une amine de formule (VII) sur le chlorure de phtalimido-2 acétyle.

Cette réaction s'effectue généralement au sein d'un solvant inerte tel qu'un solvant chloré (chloroforme, dichloro-1,2 éthane par exemple), en présence d'une base telle qu'une amine tertiaire comme la triéthylamine, à une température voisine de 20°C.

Les dérivés de formule (V) peuvent également être obtenus par action de phtalimide potassé sur un dérivé de formule : 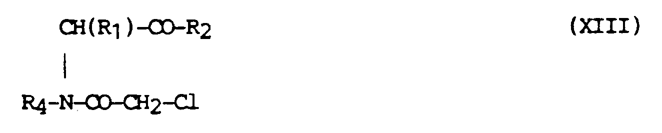
dans laquelle R₁, R₂ et R₄ ont les mêmes significations que dans la formule (I).

Cette réaction s'effectue au sein d'un solvant inerte tel que le diméthylformamide, à une température voisine de 100°C.

Les dérivés de formule (XIII) peuvent être obtenus par action d'une amine de formule (VI) sur le chlorure de chloroacétyle.

Cette réaction s'effectue au sein d'un solvant inerte tel que le diméthylformamide, le tétrahydrofuranne ou un solvant chloré en présence d'une amine tertiaire telle que la triéthylamine, à une température comprise entre 10°C et 80°C.

Les dérivés formule (V) pour lesquels R₂ représente un radical -NR₅R₆ peuvent également être obtenus par action d'une amine de formule (XI) sur un acide de formule : 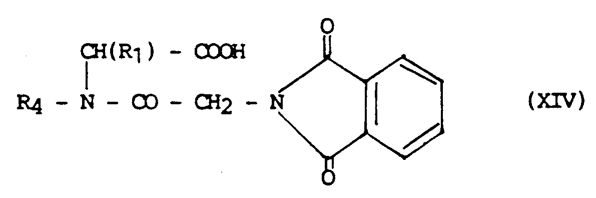
dans laquelle R₁ et R₄ ont les mêmes significations que dans la formule (I) ou un dérivé réactif de cet acide.

Cette réaction s'effectue généralement dans les conditions mentionnées précédemment pour la réaction des acides de formule (X) et des amines de formule (XI).

Les acides de formule (XIV) peuvent être obtenus par hydrolyse des esters correspondants.

Cette hydrolyse s'effectue par toute méthode connue de l'homme de l'art permettant de passer d'un ester à un acide. De préférence, on utilise l'acide trifluoroacétique, à une température voisine de 20°C.

Les dérivés de formule (V) pour lesquels R₄ représente un radical phényle substitué par un radical hydroxy peuvent également être obtenus par désalkylation des dérivés correspondants pour lesquels R₄ représente un radical phényle substitué par un radical alcoxy.

Cette désalkylation s'effectue de préférence au moyen de tribromure de bore, au sein d'un solvant inerte tel qu'un solvant chloré, à une température voisine de 20°C.

Les dérivés de formule (V) pour lesquels R₄ représente un radical phényle substitué par un radical alcoxy peuvent également être obtenus par action d'un agent alkylant tel qu'un halogènure d'alkyle sur les dérivés correspondants pour lesquels R₄ représente un radical phényle substitué par un radical hydroxy.

Cette réaction s'effectue de préférence au moyen d'hydrure de sodium dans un solvant organique inerte tel que le diméthylformamide, à une température comprise entre 10°C et 50°C.

Les composés de formule (I) pour lesquels R₃ représente un radical phénylamino (dont le noyau phényle est éventuellement substitué par un ou plusieurs substituants choisis parmie les atomes d'halogène et les radicaux alkyle, alcoxy, alkylthio, nitro, acyle, cyano, sulfamoyle, benzoyle, alcoxycarbonyle, tétrazolyl-5, tétrazolyl-5 alkyle et -alk-O-alk peuvent également être préparés par action d'une amine de formule (VI) sur un acide de formule :

HOOC-CH₂-NH-CO-R₃ (XV)

dans laquelle R₃ a les mêmes significations que ci-dessus ou un dérivé réactif de cet acide.

Cette réaction s'effectue généralement dans les conditions mentionnées précédemment pour la réaction des acides de formule (X) sur une amine de formule (XI).

Les acides de formule (XV) peuvent être obtenus par action d'un isocyanate de formule (II) sur la glycine.

Cette réaction s'effectue généralement en solution aqueuse, en présence d'une base telle qu'un bicarbonate de métal alcalin, à une température voisine de 20°C.

Les composés de formule (I) pour lesquels R₃ représente un radical phénylamino éventuellement substitué peuvent également être préparés par action d'un dérivé de formule : 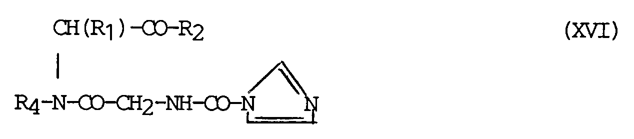
dans laquelle R₁, R₂ et R₄ ont les mêmes significations que dans la formule (I), sur un dérivé de formule :

H₂N-R₁₁ (XVII)

dans laquelle R₁₁ représente un radical phényle (éventuellement substitué par un ou plusieurs substituants choisis parmi les atomes d'halogène, les radicaux alkyle, alcoxy, alkylthio, carboxy, hydroxy, mono ou polyhydroxyalkyle, nitro, amino, acyle, cyano, sulfamoyle, carbamoyle, benzoyle, alcoxycarbonyle, tétrazolyl-5, tétrazolyl-5 alkyle, phénylhydroxyméthyle, pipéridino, hydroxyiminoalkyle, alcoxyiminoalkyle, alkylsulfinyle, sulfo, -alk-O-CO-alk, -alk-O-alk, -alk-COOX, -O-alk-COOX, -alk'-COOX, -CH=CH-COOX, -CO-COOX, -alk-SO₃H et -S-alk-COOX.

Cette réaction s'effectue généralement au sein d'un solvant inerte tel que le tétrahydrofuranne, le diméthylformamide, un solvant chloré ou un solvant aromatique, à une température comprise entre 20°C et la température d'ébullition du solvant.

Les anilines substituées de formule (XVII) peuvent être obtenues par application ou adaptation des méthodes décrites par R. SCHRÖTER, Methoden der Orgnischen Chemie, Houber Weil, Band XI/1, p. 360, G. J. ESSELEN et coll., J. Am. Chem, Soc., 36, 322 (1914) ; G. ADRIANT et coll.,Bull. Soc. Chim. Fr, 1511 (1970) ; W. A. JACOBS et coll., J. Am. Chem. Soc., 39, 2438 (1917) et J. Am. Chem Soc., 39, 1438 (1917) et dans les exemples.

Les dérivés de formule (XVI) peuvent être obtenus par action d'un dérivé de formule (III) sur le N,N-diimidazole carbonyle.

Cette réaction s'effectue généralement au sein d'un solvant inerte tel que le tétrahydrofuranne, le diméthylformamide, un solvant chloré ou un solvant aromatique, à une température comprise entre 20°C et la température d'ébullition du solvant.

Les composés de formule (I) pour lesquels R₃ représente un radical phénylamino dont le noyau phényle est éventuellement substitué peuvent également être préparés par action d'une amine de formule (XVII) sur un isocyanate de formule : 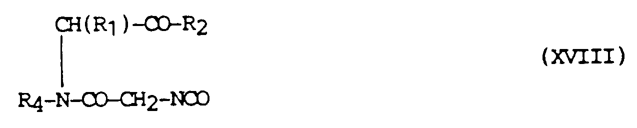
dans laquelle R₁, R₂ et R₄ ont les mêmes significations que dans la formule (I).

Cette réaction s'effectue généralement au sein d'un solvant inerte tel qu'un éther (tétrahydrofuranne par exemple), un solvant chloré (chloroforme, chlorure de méthylène par exemple) ou un solvant aromatique (benzène, toluène par exemple), à une température comprise entre 10°C et la température d'ébullition du solvant.

Les isocyanates de formule (XVIII) peuvent être obtenus par action d'une amine de formule (VI) sur le chlorure d'isocyanatoacétyle.

Cette réaction s'effectue au sein d'un solvant inerte tel qu'un éther (éther diéthylique par exemple) en présence d'une base organique azotée telle qu'une trialkylamine ou la pyridine, à une température voisine de 20°C.

Les composés de formule (I) pour lesquels R₃ représente un radical pnénylamino dont le noyau phényle est substitué par au moins un radical carboxy, -alk-COOH, -O-alk-COOH, -alk'-COOH, -CH=CH-COOH, -CO-COOH ou -S-alk-COOH et/ou R₄ représente un radical phényle substitué par un radical carboxy à l'exception des composés comportant un radical alcoxycarbonyle peuvent également être obtenus par hydrolyse d'un ester correspondant.

Cette hydrolyse s'effectue généralement au moyen d'une base telle que la soude, la potasse, au sein d'un solvant inerte tel que l'eau, le tétrahydrofuranne, le dioxanne ou un mélange de ces solvants, à une température voisine de 25°C.

Les composés de formule (I) pour lesquels R₄ représente un radical phényle substitué par un radical hydroxy à l'exception de ceux comportant un radical alcoxy, alcoxycarbonyle, alkylthio, cycloalkyloxy, cycloalkylalkyloxy, phénlalkyloxy, trifluorométhoxy, cinnamyloxy peuvent également être obtenus par hydrolyse des composés correspondants pour lesquels R₄ représente un radical phényle substitué par un radical alcoxy.

Cette hydrolyse s'effectue de préférence au moyen de tribromure de bore, au sein d'un solvant inerte tel qu'un solvant chloré (chloroforme, dichlorométhane par exemple) à une température comprise entre -55°C et 30°C.

Les composés de formule (I) pour lesquels R₃ représente un radical phényle éventuellement substitué, naphtyle, indolyle ou quinolyle peuvent être préparés par action d'un dérivé de formule (III) sur un acide de formule :

HOOC-R₃ (XIX)

dans laquelle R₃ a les mêmes significations que précédemment, ou un dérivé réactif de cet acide.

Cette réaction s'effectue généralement dans les conditions décrites précédemment pour la réaction d'un acide de formule (X) sur une amine de formule (XI).

Il est entendu pour l'homme du métier que, pour la mise en oeuvre des procédés selon l'invention décrite précédemment, il peut être nécessaire d'introduire des groupes protecteurs des fonctions amino afin d'éviter des réactions secondaires. Ces fonctions peuvent par exemple être bloquées sous forme de trifluorométhylacétamide puis régénérées par action de méthanol ammoniacal après avoir mis en oeuvre le procédé selon l'invention.

De même, lorqu'il existe une fonction hydroxy, il peut être nécessaire de bloquer la dite fonction, par exemple sous forme d'éthers de tert-butyldiméthylsilyle ou de triméthylsilyle puis de régénérer la fonction par hydrolyse en milieu acide ou au moyen d'ions fluorure après avoir mis en oeuvre le procédé adéquat.

De même, lorsqu'il existe une fonction carboxy, il peut être nécessaire de bloquer la dite fonction par exemple sous forme de diméthyl-4,4 oxazoline-1,3 puis de régénérer la fonction par hydrolyse en milieu acide aqueux ou hydroalcoolique après avoir mis en oeuvre le procédé adéquat ou sous forme d'ester benzylique puis de régénerer la fonction par hydrogénation après avoir mis en oeuvre le procédé adéquat.

Les énantiomères des composés de formule (I) contenant au moins un centre asymétrique peuvent être obtenus par dédoublement des racémiques par exemple par chromatographie sur colonne chirale selon W.H. PIRCKLE et coll., asymetric synthesis, vol. 1, Academic Press (1983) ou par synthèse à partir des précurseurs chiraux.

Les composés de formule (I) peuvent être purifiés par les méthodes connues habituelles, par exemple, par cristallisation, chromatographie, extraction...

Les composés de formule (I) présentent des propriétés pharmacolgiques intéressantes. Ces composés possèdent une forte affinité pour les récepteurs de la cholécystokinine (CCK) et de la gastrine et sont donc utiles dans le traitement et la prévention des désordres liés à la CCK et à la gastrine au niveau du système nerveux et de l'appareil gastrointestinal.

C'est ainsi que ces composés peuvent être utilisés pour le traitement ou la prévention des psychoses, des troubles anxieux, de la maladie de Parkinson, de la diskinésie tardive, du syndrôme du colon irritable, de la pancréatite aiguë, des ulcères, des désordres de la motilité intestinale, de certaines tumeurs de l'oesphage inférieur, du colon et de l'intestin et comme régulateur de l'appétit.

Ces composés ont également un effet de potentialisation sur l'activité analgésique des médicaments narcotiques et non narcotiques.

L'affinité des composés de formule (I) pour les récepteurs CCK a été déterminée selon une technique inspirée de celle de S. SAITO et coll., (J. Neuro. Chem., 37, 483-490 (1981)) au niveau du cortex cérébral et au niveau du pancréas.

Dans ces tests la CI₅₀ des composés de formule (1) est généralement égale ou inférieure à 1000 nM.

Par ailleurs, il est connu que les produits qui reconnaissent les récepteurs centraux de la CCK ont une spécificité similaire pour les récepteurs de la gastrine dans le tractus gastrointestinal (BOCK et coll., J. Med. Chem., 32; 16-23 (1989) ; REYFELD et coll., Am. J. Physiol., 240, G255-266 (1981) ; BEINFELD et coll., Neuropeptides, 3, 411-427 (1983)).

Les composés de formule (I) présentent une faible toxicité. Leur DL₅₀ est généralement supérieure à 40 mg/kg par voie sous cutanée chez la souris.

D'un intérêt particulier sont les composés de formule (I) pour lesquels:
- R₁ représente un atome d'hydrogène,
- R₂ représente un radical alcoxy ou -NR₅R₆,
- R₃ représente un radical phénylamino (dont le noyau phényle est substitué par un ou plusieurs substitutants choisis parmi les radicaux alkyle, moohydroxyalkyle, carboxy et -alk-COOH),
- R₄ représente un radical phényle substitué par un ou plusieurs substituants choisis parmi les atomes d'halogène et les radicaux alcoxy, hydroxy, alcoxycarbonyle et -CO-NR₁₂R₁₃.

Les composés préférés sont les suivants:
- [N-(méthoxy-3 phényl)[(méthyl-3 phényl)-3 uréido]-2 acétamido]-2 N-méthyl N-phényl-acétamide
- [N-(chloro-2 phényl)[(méthyl-3 phényl)-3 uréido]-2 acétamido]-2 N-méthyl N-phényl-acétamide
- {[[(hydroxy-1 éthyl)-3 phényl]-3 uréido]-2 N-(méthoxy-3 phényl) acétamido]-2 N-méthyl N-phényl-acétamide-(RS)
- {N-[(diméthyl-3,3 pipéridino) carbonyl-2 phényl] [(méthyl-3 phényl)-3 uréido]-2 acétamido}-2 acétate de tert-butyle
- {N-[(N-méthyl-anilino) carbonyl-2 phényl] [(méthyl-3 phényl)-3 uréido]-2 acétamido}-2 acétate de tert-butyle
- acide {[N-(méthoxy-3 phényl) N-(N-méthyl N-phényl-carbamoylméthyl) carbamoylméthyl]-3 uréido}-3 benzoïque
- acide {[N-(méthoxy-3 phényl) N-(N-méthyl N-phényl-carbamoylméthyl) carbamoylméthyl]-3 uréido}-3 phénylacétique
- acide {[N-(hydroxy-3 phényl) N-(N-méthyl N-phényl-carbamoylméthyl) carbamoylméthyl]-3 uréido}-3 phénylacétique
- acide {{N-(méthoxy-3 phényl) N-[(tétrahydro-1,2,3,4 quinolyl-1)-2 oxo-2 éthyl] carbamoylméthyl}-3 uréido}-3 benzoïque
- {[(méthyl-3 phényl)-3 uréido]-2 N-(tert-butoxycarbonyl-2 phényl) acétamido}-2 acètate de tert-butyle
- acide {{[N-(méthoxy-3 phényl) N-(N-méthyl N-phényl-carbamoylméthyl) carbamoylméthyl]-3 uréido}-3 phényl}-2 propionique-(RS)
Les exemples suivants illustrent l'invention sans la limiter.

### EXEMPLE 1

A une solution de 1,25 g d'[amino-2 N-(chloro-3 phényl) acétamido]-2 acétate de tert-butyle dans 20 cm3 de tétrahydrofuranne anhydre, on ajoute, à une température voisine de 20°C, 0,6 g d'isocyanate de méthyl-3 phényle. La solution obtenue est agitée pendant 4 heures à une température voisine de 20°C puis concentrée à sec sous pression réduite (2,7 kPa) à 40°C. L'huile résiduelle est purifiée par chromatographie sur 150 g de silice (0,063-0,2 mm) contenus dans une colonne de 2 cm de diamètre [éluant : cyclohexaneacétane d'éthyle (75-25 en volumes)] en recueillant des fractions de 20 cm3. Les fractions ne contenant que le produit cherché sont réunies et concentrées à sec sous pression réduite (2,7 kPa) à 40°C. On obtient après cristallisation dans l'oxyde de diisopropyle, 0,8 g de [N-(chloro-3 phényl)[(méthyl-3 phényl)-3 uréido]-2 acétamido]-2 acétate de tert-butyle fondant à 110°C.

L'[amino-2 N-(chloro-3 phényl) acétamido]-2 acétate de tert-butyle peut être préparé de la maniére suivante : à une solution de 2,4 g de [N-(chloro-3 phényl) phtalimido-2 acétamido]-2 acétate de tert-butyle dans 40 cm³ de méthanol, on ajoute 0,75 g d'hydrate d'hyrazine. Le mélange réactionnel est agité à reflux penant 3 heures puis concentré à sec sous pression réduite (2,7 kPa) à 40°C. Le résidu est agité avec 100 cm3 d'éther diéthylique puis le produit insoluble est séparé par filtration et le filtrat est concentré à sec sous pression réduite (2,7 kPa) à 40°C. On obtient ainsi 1,3 g d'[amino-2 N-(chloro-3 phényl) acétamido]-2 acétate de tert-butyle sous forme d'une huile utilisée telle quelle dans les synthèses ultérieures.

Le [N-(chloro-3 phényl) phtalimido-2 acétamido]-2 acétate de tert-butyle peut être préparé de la manière suivante : à une solution de 4,8 g de (chloro-3 phényl) amino-2 acétate de tert-butyle dans 60 cm3 de dichloro-1,2 éthane, maintenue sous atmosphère d'argon, on ajoute 2,8 g de triéthylamine puis goutte à goutte, à une température voisine de 20°C, une solution de 6,2 g de chlorure de phtalimido-2 acétyle dans 20 cm3 de dichloro-1,2 éthane. La solution obtenue est agitée pendant 3 heures à une température voisine de 20°C, puis additionnée de 50 cm³ d'eau. La phase aqueuse est séparée par décantation puis réextraite par 2 fois 50 cm3 de dichloro-1,2 éthane. Les phases organique sont réunies, lavées par 2 fois 10 cm3 d'eau, séchées sur sulfate de magnésium, filtrées puis concentrées à sec sous pression réduite (2,7 kPa) à 40°C. L'huile obtenu est purifiée par chromatographie sur 200 g de silice (0,063-0,2 mm) contenus dans une colonne de 2,5 cm de diamètre [éluant : cyclohexane-acétate d'éthyle (50-50 en volumes)] en recueillant des fractions de 25 cm3. Les fractions 3 à 7 sont réunies et concentrées à sec sous pression réduite (2,7 kPa) à 40°C. On obtient ainsi 2,6 g de [N-(chloro-3 phényl) phtalimido-2 acétamido]-2 acétate de tert-butyle sous forme d'une huile utilisée telle quelle dans les synthèses ultérieures.

Le (chloro-3 phényl) amino-2 acétate de tert-butyle peut être préparé de la manière suivante : à une solution de 7,6 g de chloro-3 aniline dans 60 cm³ d'acétonitrile, on ajoute 5,9 g de bromoacétate de tert-butyle. La solution obtenue est agitée à reflux pendant 4 heures. Après refroidissement, le produit insoluble est séparé par filtration et lavé par 30 cm3 d'acétonitrile. Les filtrats sont réunis et concentrés à sec sous pression réduite (2,7 kPa) à 40°C. L'huile résiduelle est dissoute dans 150 cm3 de dichlorométhane et la solution obtenue est lavée par 4 fois 15 cm3 d'eau. La phase organique est séchée sur sulfate de magnésium, filtrée, puis concentrée à sec sous pression réduite (2,7 kPa) à 40°C. On obtient ainsi 8,1 g de (chloro-3 phényl) amino-2 acétate de tert-butyle sous forme d'une huile utilisée telle quelle dans les synthèses ultérieures.

Le chlorure de phtalimido-2 acétyle peut être préparé selon la méthode décrite par W. GRASSMANN et E. SCHULTE-UEBBING, Chem. Ber., 83, 244-247, (1950).

### EXEMPLE 2

En opérant d'une manière analogue à celle décrite à l'exemple 1, mais à partir de 2,5 g d'[amino-2 N-(fluoro-2 phényl) acétamido]-2 acétate de tert-butyle et de 1,2 g d'isocyanate de méthyl-3 phényle, on obtient après recristallisation dans l'oxyde de diisopropyle, 1,75 g de [N-(fluoro-2 phényl) [(méthyl-3 phényl)-3 uréido]-2 acétamido]-2 acétate de tert-butyle fondant à 148°C.

L'[amino-2 N-(fluoro-2 phényl) acétamido]-2 acétate de tert-butyle peut être préparé d'une manière analogue à celle décrite à l'exemple 1 pour la préparation de l'[amino-2 N-(chloro-3 phényl) acétamido]-2 acétate de tert-butyle, mais à partir de 4,9 g de [N-(fluoro-2 phényl) phtalimido-2 acétamido]-2 acétate de tert-butyle et de 0,77 g d'hydrate d'hydrazine. On obtient ainsi 2,7 g d'[amino-2 N-(fluro-2 phényl) acétamido]-2 acétate de tert-butyle sous forme d'une huile utilisée telle quelle dans les synthèses ultérieures.

Le [N-(fluoro-2 phényl) phtalimido-2 acétamido]-2 acétate de tert-butyle peut être préparé de la manière suivante : à une solution de 3,3 g de (fluoro-2 phénylamino)-2 acétate de tert-butyle dans 60 cm3 de dichloro-1,2 éthane, maintenue sous atmosphère d'argon, on ajoute 1,3 g d'hydrogénocarbonate de sodium puis goutte à goutte, à une température voisine de 20°C, une solution de 3,1 g de chlorure de phtalimido-2 acétyle dans 10 cm3 de dichloro-1,2 éthane. La solution obtenue est agitée pendant 3 heures à une température voisine de 20°C, puis additionée de 20 cm3 d'eau. La phase aqueuse est séparée par décantation puis réextraite par 2 fois 100 cm³ de dichloro-1,2 éthane. Les phases organiques sont réunies, séchées sur sulfate de magnésium, filtrées puis concentrées à sec sous pression réduite (2,7 kPa) à 40°C. On obtient ainsi, après recristallisation dans l'éther de pétrole, 4,9 g de [N-(fluoro-2 phényl) phtalimido-2 acétamido]-2 acétate de tert-butyle fondant à 140°C.

Le (fluoro-2 phényl)amino-2 acétate de tert-butyle peut être préparé d'une manière analogue à celle décrite à l'exemple 1 pour la préparation du (chloro-3 phénylamino)-2 acétate de tert-butyle, mais à partir de 2,45 g de fluoro-2 aniline et de 1,95 g de bromoacétate de tert-butyle. On obtient ainsi, après recristallisation dans l'éther de pétrole, 1,1 g de (fluoro-2 phényl)amino-2 acétate de tert-butyle fondant à 70°C.

### EXEMPLE 3

En opérant d'une manière analogue à celle décrite à l'exemple 1, mais à partir de 6,6 g d'[amino-2 N-(méthoxy-4 phényl) acétamido]-2 acétate de tert-butyle et de 3 g d'isocyanate de méthyl-3 phényle, on obtient après recristallisation dans l'acétontrile, 1,7 g de [N-(méthoxy-4 phényl) [(méthyl-3 phényl)-3 uréido]-2 acétamido]-2 acétate de tert-butyle fondant à 158°C.

L'[amino-2 N-(méthoxy-4 phényl) acétamido]-2 acétate de tert-butyle peut être préparé d'une manière analogue à celle décrite à l'exemple 1 pour la préparation de l'[amino-2 N-(chloro-3 phényl) acétamido]-2 acétate de tert-butyle, mais à partir de 11,7 g de [N-(méthoxy-4 phényl) phtalimido-2 acétamido]-2 acétate de tert-butyle et de 1,75 g d'hydrate d'hydrazine. On obtient ainsi 7 g d'[amino-2 N-(méthoxy-4 phényl) acétamido]-2 acétate de tert-butyle sous forme d'une huile utilisée telle quelle dans les synthèses ultérieures.

Le [N-(méthoxy-4 phényl) phtalimido-2 acétamido]-2 acétate de tert-butyle peut être préparé d'une manière analogue à celle décrite à l'exemple 2 pour la préparation du [phtalimido-2 N-(fluoro-2 phényl) acétamido]-2 acétate de tert-butyle, mais à partir de 6,7 g de (méthoxy-4 phénylamino)-2 acétate de tert-butyle de 2,5 g d'hydrogénocarbonate de sodium et de 6,25 g de chlorure de phtalimido-2 acétyle. On obtient ainsi 11,7 g de [phtalimido-2 N-(méthoxy-4 phényl) acétamido]-2 acétate de tert-butyle sous forme d'une huile utilisée telle quelle dans les synthèses ultérieures.

Le (méthoxy-4 phényl)amino-2 acétate de tert-butyle peut être préparé d'une manière analogue à celle décrite à l'exemple 1 pour la préparation du (chloro-3 phényl)amino-2 acétate de tert-butyle, mais à partir de 7,3 g de méthoxy-4 aniline et de 5,95 g de bromoacétate de tert-butyle. On obtient ainsi 7,2 g de (méthoxy-4 phényl)amino-2 acétate de tert-butyle sous forme d'une huile utilisée telle quelle dans les synthèses ultérieures.

### EXEMPLE 4

En opérant d'une manière analogue à celle décrite à l'exemple 1, mais à partir de 2 g d'[amino-2 N-(trifluorométhoxy-2 phényl) acétamino]-2 acétate de tert-butyle et de 0,8 g d'isocyanate de méthyl-3 phényle, on obtient après recristallisation dans l'acétate d'éthyle, 1,35 g de [[(méthyl-3 phényl)-3 uréido]-2 N-(trifluométhoxy-2 phényl) acétamido]-2 acétate de tert-butyle fondant à 163°C.

L'[amino-2 N-(trifluorométhoxy-2 phényl) acétamido]-2 acétate de tert-butyle peut être préparé de la manière suivante : à une solution de 4,4 g de [phtalimido-2 N-triflurométhoxy-2 phényl) acétamido]-2 acétate de tert-butyle dans 50 cm3 d'éthanol, on ajoute 1,5 g d'hydrate d'hydrazine. Le mélange réactionnel est agité pendant 3 heures à une température voisine de 20°C puis concentré à sec sous pression réduite (2,7 kPa) à 40°C. Le résidu est agité avec 200 cm³ d'éther diéthylique et le produit insoluble est séparé par filtration. Le filtrat est concentré à sec sous pression réduite (2,7 kPa) à 40°C. On obtient ainsi 2,1 g d'[amino-2 N-(trifluorométhoxy-2 phényl) acétamino]-2 acétate de tert-butyle sous forme d'une huile utilisée telle quelle dans les synthèses ultérieures.

Le [phtalimido-2 N-(trifluorométhoxy-2 phényl) acétamido]-2 acétate de tert-butyle peut être préparé de la manière suivante : à une solution de 5 g de phtalimido-2 N-(trifluorométhoxy-2 phényl) acétamide dans 50 cm3 de tétrahydrofuranne anhydre maintenue sous atmosphère d'argon, on ajoute à une température voisine de 10°C, 0,7 g d'une suspension huileuse (50 % en poids) d'hydrure de sodium et agite la suspension obtenue pendant 1 heure à une température voisine de 20°C. On ajoute alors une solution de 2,75 g de bromoacétate de tert-butyle dans 10 cm³ de tétrahydrofuranne anhydre et l'on poursuit l'agitation pendant 3 heures à une température voisine de 20°C. Le mélange réactionnel est ensuite versé dans un mélange refroidi à une température voisine de 0°C, de 20 cm3 d'eau et de 200 cm3 d'acetate d'éthyle. Le phase aqueuse est séparée par décantation et réextraire par 2 fois 20 cm3 d'acétate d'éthyle. Les phases organiques sont réunies, lavées par 3 fois 25 cm3 d'eau, séchées sur sulfate de magnésium, filtrées puis concentrées à sec sous pression réduite (2,7 kPa) à 40°C. on obtient ainsi 4,4 g de [phtalimido-2 N-(trifluorométhoxy-2 phényl) acétamido-2 acétate de tert-butyle sous forme d'une huile utilisée telle quelle dans les synthèses ultérieures.

Le phtalimidi-2 N-(trifluorométhoxy-2 phényl) acétamide peut être préparé de la manière suivante : à une solution de 3,6 g de trifluorométhoxy-2 aniline dans 50 cm3 de dichlorométhane maintenue sous atmosphère d'argon, on ajoute 2,2 g de triéthylamine puis en maintenant la température au voisinage de 20°C, une solution de 4,6 g de chlorure de phtalimido-2 acétyle dans 25 cm3 de dichlorométhane. La solution obtenue est agitée pendant 3 heures à une température voisine de 20°C, puis additionnée de 25 cm3 d'eau. Le solide formé est séparé par filtration, lavé par 3 fois 5 cm3 de dichlorométhane puis par 3 fois 10 cm3 d'eau séché à l'air. La phase organique du filtrat est séparée par décantation, lavée par 2 fois 10 cm3 d'eau distillée, séchée sur sulfate de magnésium, filtrée puis concentrés à sec sous pression réduite (2,7 kPa) à 40°C. Le solide obtenu est réuni au prédédent et l'ensemble est recristallisé dans l'acétate d'éthyle. On obtient ainsi 5,1 g de phtalimido-2 N-(trifluorométhoxy-2 phényl) acétamide fondant à 192°C.

### EXEMPLE 5

En opérant d'une manière analogue à celle décrite à l'exemple 1, mais à partir de 3,4 g d'[amino-2 N-(trifluorométhoxy-3 phényl) acétamido]-2 acétate de tert-butyle et de 1,4 g d'isocyanate de méthyl-3 phényle, on obtient après recristallisation dans l'oxyde de diisopropyle, 1,75 g de [[(méthyl-3 phényl)-3 uréido]-2 N-(trifluorométhoxy-3 phényl) acétamido]-2 acétate de tert-butyle fondant à 125°C.

L'[amino-2 N-(trifluorométhoxy-3 phényl) acétamido]-2 acétate de tert-butyle peut être préparé d'une manière analogue à celle décrite à l'exemple 4 pour la préparation de l'[amino-2 N-(triflurométhoxy-2 phényl) acétamido]-2 acétate de tert-butyle, mais à partir de 3 g de [phtalimido-2 N-(trifluorométhoxy-3 phényl) acétamido]-2 acétate de tert-butyle et de 3,2 g d'hydrate d'hyrazine. On obtient ainsi 3,5 g d'[amino-2 N-(trifluorométhoxy-3 phényl) acétamido]-2 acétate de tert-butyle sous forme d'une huile utilisée telle quelle dans les synthèses ultérieures.

Le [phtalimido-2 N-(trifluorométhoxy-3 phényl) acétamido]-2 acétate de tert-butyle peut être préparé d'une manière analogue à celle décrite à l'exemple 4 pour la préparation du [phtalimido-2 (trifluorométhoxy-2 phényl) acétamido]-2 acétate de tert-butyle, mais à partir de 4,8 g de phtalimido-2 N-(trifluorométhoxy-3 phényl) acétamide, de 0,7 g d'une suspension huileuse (50 % en poids) d'hydrure de sodium et de 2,75 g de bromoacétate de tert-butyle. On obtient ainsi 5,1 g de [phtalimido-2 N-(trifluorométhoxy-3 phényl) acétamido]-2 acétate de tert-butyle sous forme d'une huile utilisée telle quelle dans les synthèses ultérieures.

Le phtalimido-2 N-(trifluorométhoxy-3 phényl) acétamide peut être préparé d'une manière analogue à celle décrite à l'exemple 4 pour la préparation du phtalimido-2 N-(trifluorométhoxy-2 phényl) acétamide, mais à partir de 3,6 de trifluorométhoxy-3 aniline, de 2,2 g de triéthylamine et de 4,6 de chlorure de phtalimido-2 acétyle. On obtient ainsi, après recristallisation dans l'acétate d'éthyle, 4,8 g de phtalimido-2 N-(trifluorométhoxy-3 phényl) acétamide fondant à 170°C.

### EXEMPLE 6

En opérant d'une manière analogue à celle décrite à l'exemple 1, mais à partir de 3,1 g d'[amino-2 N-(méthyl-3 phényl) acétamido]-2 acétate de tert-butyle et de 0,52 g d'isocyante de méthyl-3 phényle, on obtient après recristallisation dans l'oxyde de diisopropyle, 1,2 g de [[(méthyl-3 phényl)-3 uréido]-2 N-(méthyl-3 phényl) acétamido]-2 acétate de tert-butyle fondant à 97°C.

L'[amino-2 N-(méthyl-3 phényl) acétamido]-2 acétate de tert-butyle peut être préparé d'une manière analogue à celle décrite à l'exemple 4 pour la préparation de l'[amino-2 N-(triflurométhoxy-2 phényl) acétamido]-2 acétate de tert-butyle, mais à partir de 7,5 g de [N-(méthyl-3 phényl) phtalimido-2 acétamido]-2 acétate de tert-butyle et de 1,84 g d'hydrate d'hydrazine. On obtient ainsi 3,5 g d'[amino-2 N-(méthyl-3 phényl) acétamido]-2 acétate de tert-butyle sous forme d'une huile utilisée telle quelle dans les synthèses ultérieures.

Le [N-(méthyl-3 phényl) phtalimido-2 acétamido]-2 acétate de tert-butyle peut être préparé d'une manière analogue à celle décrite à l'exemple 4 pour la préparation du [phtalimido-2 N-(trifluorométhoxy-2 phényl) acétamido]-2 acétate de tert-butyle, mais à partir de 12,5 g de N-(méthyl-3 phényl) phtalimido-2 acétamide, de 2,45 g d'une suspension huileuse (50 % en poids) d'hydrure de sodium et de 8,3 g de bromoacétate de tert-butyle. On obtient ainsi, après recristallisation dans l'oxyde de diisopropyle, 7,6 g de [N-(méthyl-3 phényl) phtalimido-2 acétamido]-2 acétate de tert-butyle fondant à 166°C.

Le N-(méthyl-3 phényl) phtalimido-2 acétamide peut être préparé d'une manière analogue à celle décrite à l'exemple 4 pour la préparation du phtalimido-2 N-(trifluorométhoxy-2 phényl)-acétamide, mais à partir de 5,36 g de méthyl-3 aniline, de 5,1 g de triéthylamine et de 11,2 g de chlorure de phtalimido-2 acétyle. On obtient ainsi 12,7 g de N-(méthyl-3 phényl) phtalimido-2 acétamide fondant à 207°C.

### EXEMPLE 7

En opérant d'une manière analogue à celle décrite à l'exemple 1, mais à partir de 1,6 g d'[amino-2 N-(méthoxy-3 phényl) acétamido]-2 N-méthyl N-phényl-acétamide et de 0,67 g d'isocyanate de méthyl-3 phényle, on obtient après recristallisation dans l'acétonitrile, 1,2 g de [N-(méthoxy-3 phényl) [(méthyl-3 phényl)-3 uréido]-2 acétamido]-2 N-méthyl N-phényl-acétamide fondant à 179°C.

L'[amino-2 N-(méthoxy-3 phényl) acétamido]-2 N-méthyl N-phényl-acétamide peut être préparé d'une manière analogue à celle décrite à l'exemple 4 pour préparation de l'[amino-2 N-(trifluorométhoxy-2 phényl) acétamido]-2 acétate de tert-butyle, mais à partir de 2,3 g de [N-(méthoxy-3 phényl) phtalimido-2 acétamido]-2 N-méthyl N-phényl-acétamide, et de 0,75 g d'hydrate d'hydrazine. On obtient ainsi 1,6 g d'[amino-2 N-(méthoxy-3 phényl) acétamido]-2 N-méthyl N-phényl acétamide sous forme d'une huile utilisée telle quelle dans les synthèses ultérieures.

Le [N-(méthoxy-3 phényl) phtalimido-2 acétamido]-2 N-méthyl N-phényl-acétamide peut être préparé d'une manière analogue à celle décrite à l'exemple 4 pour la préparation du [phtalimido-2 N-(trifluorométhoxy-2 phényl) acétamido]-2 acétate de tert-butyle, mais à partir de 9,15 g de N-(méthoxy-3 phényl) phtalimido-2 acétamide, de 1,6 g d'une suspension huileuse (50 % en poids) d'hydrure de sodium et de 7,4 g de bromo-2 N-méthyl N-phényl-acétamide. On obtient ainsi 5,7 g de [N-(méthoxy-3 phényl) phtalimido-2 acétamido]-2 N-méthyl N-phényl-acétamide sous forme d'une résine utilisée telle quelle dans les synthèses ultérieures.

Le N-(méthoxy-3 phényl) phtalimido-2 acétamide peut être préparé d'une manière analogue à celle décrite à l'exemple 4 pour la préparation du phtalimdo-2 N-(trifluorométhoxy-2 phényl) acétamide, mais à partir de 6,15 g de méthoxy-3 aniline, de 5,6 g de triéthylamine et de 11,2 g de chlorure de phtalimido-2 acétyle. On obtient ainsi, après recristallisation dans l'acétonitrile, 12,3 g de phtalimido-2 N-(méthoxy-3 phényl) acétamide fondant à 186°C.

### EXEMPLE 8

En opérant d'une manière analogue à celle décrite à l'exemple 1, mais à partir de 4,9 g d'amino-2 N-(trifluorométhyl-3 phényl) acétamido]-2 acétate de tert-butyle et de 2 g d'isocyanate de méthyl-3 phényle, on obtient après recristallisation dans l'oxyde de diisopropyle, 1,56 g de [[(méthyl-3 phényl)-3 uréido]-2 N-(trifluorométhyl-3 phényl) acétamido]-2 acétate de tert-butyle fondant à 140°C.

L'[amino-2 N-(trifluorométhyl-3 phényl) acétamido]-2 acétate de tert-butyle peut être préparé d'une manière analogue à celle décrite à l'exemple 4 pour la préparation de l'[amino-2 N-(trifluorométhoxy-2 phényl) acétamido]-2 acétate de tert-butyle, mais à partir de 9 g de [phtalimido-2 N-(trifluorométhyl-3 phényl) acétamido]-2 acétate de tert-butyle et de 2,9 g d'hydrate d'hydrazine. On obtient ainsi 5,2 g d'[amino-2 N-(trifluorométhyl-3 phényl) acétamido]-2 acétate de tert-butyle sous forme d'une huile utilisée telle quelle dans les synthèses ultérieures.

Le [phtalimido-2 N-(trifluorométhyl-3 phényl) acétamido]-2 acétate de tert-butyle peut être préparé d'une manière analogue à celle décrite à l'exemple 4 pour la préparation du [phtalimido-2 N-(trifluorométhoxy-2 phényl) acétamido]-2 acétate de tert-butyle, mais à partir de 16,5 g de phtalimido-2 N-(trifluorométhyl-3 phényl) acétamide, de 2,3 g d'une suspension huileuse (50 % en poids) d'hydrure de sodium et de 9,2 g de bromoacétate de tert-butyle. On obtient ainsi 9,1 g de [phtalimido-2 N-(trifluorométhyl-3 phényl) acétamido]-2 acétate de tert-butyle sous forme d'une huile utilisée telle quelle dans les synthèses ultérieures.

Le phtalimido-2 N-(trifluorométhyl-3 phényl)acétamide peut être préparé d'une manière analogue à celle décrite à l'exemple 4 pour la préparation du phtalimido-2 N-(trifluorométhoxy-2 phényl) acétamide, mais à partir de 8,1 g de trifluorométhyl-3 aniline, de 5,1 g de triéthylamine et de 11,2 g de chlorure de phtalimido-2 acétyle. On obtient ainsi 16,7 g de phtalimido-2 N-(trifluorométhyl-3 phényl) acétamide fondant à 235°C.

### EXEMPLE 9

En opérant d'une manière analogue à celle décrite à l'exemple 1 mais à partir de 3,6 g d'[amino-2 N-(éthoxycarbonyl-2 phényl) acétamido]-2 acétate de tert-butyle et de 1,42 g d'isocyanate de méthyl-3 phényle, on obtient après recristallisation dans l'oxyde de diisopropyle, 1,35 g de [N-(éthoxycarbonyl-2 phényl) [(méthyl-3 phényl)-3 uréido]-2 acétamido]-2 acétate de tert-butyle fondant à 142°C.

L'[amino-2 N-(éthoxycarbonyl-2 phényl) acétamido]-2 acétate de tert-butyle peut être préparé d'une manière analogue à celle décrite à l'exemple 4 pour la préparation de l'[amino-2 N-(trifluorométhoxy-2 phényl) acétamido]-2 acétate de tert-butyle, mais à partir de 6,13 g de [N-(éthoxycarbonyl-2 phényl) phtalimido-2 acétamido]-2 acétate de tert-butyle et de 1,98 g d'hydrate d'hydrazine. On obtient ainsi 3,6 g d'[amino-2 N-(éthoxycarbonyl-2 phényl) acétamido]-2 acétate de tert-butyle sous forme d'une huile utilisée telle quelle dans les synthèses ultérieures.

Le [N-(éthoxycarbonyl-2 phényl) phtalimido-2 acétamido]-2 acétate de tert-butyle peut être préparé d'une manière analogue à celle décrite à l'exemple 4 pour la préparation du [phtalimido-2 N-(trifluorométhoxy-2 phényl) acétamido]-2 acétate de tert-butyle, mais à partir de 7,7 g de N-(éthoxycarbonyl-2 phényl) phtalimido-2 acétamide, de 1,26 g d'une suspension huileuse (50 % en poids) d'hydrure de sodium et de 4,3 g de bromoacétate de tert-butyle. On obtient ainsi, après recristallisation dans l'oxyde de diisopropyle, 6,1 g de [N-(éthoxycarbonyl-2 phényl) phtalimido-2 acétamido]-2 acétate de tert-butyle fondant à 127°C.

Le N-(éthoxycarbonyl-2 phényl) phtalimido-2 acétamide peut être préparé d'une manière analogue à celle décrite à l'exemple 4 pour la préparation du phtalimido-2 N-(trifluorométhoxy-2 phényl) acétamide, mais à partir de 4,13 g d'amino-2 benzoate d'éthyle, de 2,8 g de triéthylamine et de 5,6 g de chlorure de phtalimido-2 acétyle. On obtient ainsi 7,7 g de N-(éthoxycarbonyl-2 phényl) phtalimido-2 acétamide fondant à 187°C.

### EXEMPLE 10

En opérant d'une manière analogue à celle décrite à l'exemple 1, mais à partir de 4,7 g d'[amino-2 N-(acétylamino-2 phényl) acétamido]-2 acétate de tert-butyle et de 2 g d'isocyanate de méthyl-3 phényle, on obtient après recristallisation dans l'acétate d'éthyle, 3,6 g de [N-(acétylamino-2 phényl) [(méthyl-3 phényl)-3 uréido]-2 acétamido]-2 acétate de tert-butyle fondant à 185°C.

L'[amino-2 N-(acétylamino-2 phényl) acétamido]-2 acétate de tert-butyle peut être préparé d'une manière analogue à celle décrite à l'exemple 4 pour la préparation de l'[amino-2 N-(trifluorométhoxy-2 phényl) acétamido]-2 acétate de tert-butyle, mais à partir de 7,2 g de [N-(acétylamino-2 phényl)phtalimido-2 acétamido]-2 acétate de tert-butyle et de 2,4 g d'hydrate d'hyrazine. On obtient ainsi 4,8 g d'[amino-2 N-(acétylamino-2 phényl) acétamido]-2 acétate de tert-butyle sous forme d'une huile utilisée telle quelle dans les synthèses ultérieures.

Le [N-(acétylamino-2 phényl) phtalimido-2 acétamido]-2 acétate de tert-butyle peut être préparé d'une manière analogue à celle décrite à l'exemple 4 pour la préparation du [phtalimido-2 N-(trifluorométhoxy-2 phényl) acétamido]-2) acétate de tert-butyle, mais à partir de 9,7 g de N-(acétylamino-2 phényl) phtalimido-2 acétamide, de 1,6 g d'une suspension huileuse (50 % en poids) d'hydrure de sodium et de 5,85 g de bromoacétate de tert-butyle. On obtient ainsi, après recristallisation dans l'acétate d'éthyle, 8 g de [N-(acétylamino-2 phényl) phtalimido-2 acétamido]-2 acétate de tert-butyle fondant à 170°C.

Le N-(acétylamino-2 phényl) phtalimido-2 acétamide peut être préparé d'une manière analogue à celle décrite à l'exemple 4 pour la préparation du phtalimido-2 N-(trifluorométhoxy-2 phényl) acétamide, mais à partir de 6 g d'acétylamino-2 aniline, de 4,05 g de triéthylamine et de 9,6 g de chlorure de phtalimido-2 acétyle. On obtient ainsi 12,5 g de N-(acétylamino-2 phényl) phtalimido-2 acétamide fondant à 270°C.

### EXEMPLE 11

En opérant d'une manière analogue à celle décrite à l'exemple 1, mais à partir de 4,8 g d'[amino-2 N-(chloro-2 phényl) acétamido]-2 acétate de tert-butyle et de 2,35 g d'isocyanate de méthyl-3 phényle, on obtient après recristallisation dans un mélange d'acétate d'éthyle et de méthanol (80-20 en volumes), 1,4 g de [N-(chloro-2 phényl) [(méthyl-3 phényl)-3 uréido]-2 acétamido]-2 acétate de tert-butyle fondant à 148°C.

L'[amino-2 N-(chloro-2 phényl acétamido]-2 acétate de tert-butyle peut être préparé d'une manière analogue à celle décrite à l'exemple 1 pour la préparation de l'[amino-2 N-(chloro-3 phényl) acétamido]-2 acétate de tert-butyle, mais à partir de 9,12 g de [N-chloro-2 phényl) phtalimido-2 acétamido]-2 acétate de tert-butyle et de 1 g d'hydrate d'hyrazine. On obtient ainsi 5,2 g d'[amino-2 N-(chloro-2 phényl) acétamido]-2 acétate de tert-butyle sous forme d'une huile utilisée telle quelle dans les synthèses ultérieures.

Le [N-(chloro-2 phényl) phtalimido-2 acètamido]-2 acétate de tert-butyle peut être préparé d'une manière analogue à celle décrite à l'exemple 2 pour la préparation du [N-(fluoro-2 phényl) phtalimido-2 acétamido]-2 acétate de tert-butyle, mais à partir de 8 g de (chloro-2 phényl)amino-2 acétate de tert-butyle, de 3,1 g d'hydrogénocarbonate de sodium et de 7,4 g de chlorure de phtalimido-2 acétyle. On obtient, après recristallisation dans un mélange d'acétate d'éthyle et de cyclohexane (70-30 en volumes), 10,6 g de [N-(chloro-2 phényl) phtalimido-2 acétamido]-2 acétate de tert-butyle fondant à 164°C.

Le (chloro-2 phényl)amino-2 acétate de tert-butyle peut être préparé d'une manière analogue à celle décrite à l'exemple 1 pour la préparation du (chloro-3 phényl)amino-2 acétate de tert-butyle, mais à partir de 19,1 g de chloro-2 aniline et de 9,75 g de bromoacétate de tert-butyle. On obtient ainsi 9,3 g de (chloro-2 phényl)amino-2 acétate de tert-butyle sous forme d'une huile utilisée telle quelle dans les synthèses ultérieures.

### EXEMPLE 12

En opérant d'une manière analogue à celle décrite à l'exemple 1, mais à partir de 2,4 g d'[amino-2 N-(méthylènedioxy-3,4 phényl) acétamido]-2 acétate de tert-butyle et de 1 g d'isocyanate de méthyl-3 phényle, on obtient après recristallisation dans l'acétate d'éthyle, 1,65 g de [N-(méthylènedioxy-3,4 phényl) [(méthyl-3 phényl)-3 uréido]-2 acétamido]-2 acétate de tert-butyle fondant à 142°C.

L'[amino-2 N-(méthlènedioxy-3,4 phényl) acétamido]-2 acétate de tert-butyle peut être préparé de la manière suivante : à une solution de 4,6 g de [N-(méthylènedioxy-3,4 phényl) phtalimido-2 acétamido]-2 acétate de tert-butyle dans 70 cm3 de dichlorométhane, on ajoute, à une température voisine de 0°C, 1,45 g de méthylhydrazine. Le mélange réactionnel est agité pendant 16 heures à une température voisine de 20°C puis pendant 2 heures au reflux du dichlorométhane et refroidi à une température voisine de 20°C. On ajoute 50 cm3 d'eau, agite et sépare par décantation la phase aqueuse qui est réextraite par 2 fois 40 cm3 de dichlorométhane. Les phases organiques sont réunies, séchées sur sulfate de magnésium, filtrées puis concentrées à sec sous pression réduite (2,7 kPa) à 40°C. L'huile obtenu est purifiée par chromatographie sur 50 g de silice (0,063-0,2 mm) contenus dans une colonne de 2 cm de diamètre [éluant : acétate d'éthyle-méthanol (90-10 en volumes)] en recueillant des fractions de 20 cm3. Les fractions 11 à 22 sont réunies et concentrées à sec sous pression réduite (2,7 kPa) à 40°C. On obtient ainsi 1,85 g d'[amino-2 N-(méthylènedioxy-3,4 phényl) acétamido]-2 acétate de tert-butyle sous forme d'une huile utilisée telle quelle dans les synthèses ultérieures.

Le [N-(méthylènedioxy-3,4 phényl) phtalimido-2 acétamido]-2 acétate de tert-butyle peut être préparé d'une manière analogue à celle décrite à l'exemple 2 pour la préparation du [N-(fluoro-2 phényl) phtalimido-2 acétamido]-2 acétate de tert-butyle, mais à partir de 5,15 g de (méthylènedioxy-3,4 phényl)amino-2 acétate de tert-butyle, de 1,9 g d'hydrogénocarbonate de sodium et de 4,6 g de chlorure de phtalimido-2 acétyle. On obtient après recristallisation dans l'acétate d'éthyle, 8 g de [N-(méthylènedioxy-3,4 phényl) phtalimido-2 acétamido]-2 acétate de tert-butyle fondant à 166°C.

Le (méthylènedioxy-3,4 phényl)amino-2 acètate de tert-butyle peut être préparé d'une manière analogue à celle décrite à l'exemple 1 pour la préparation du (chloro-3 phényl)amino-2 acétate de tert-butyle, mais à partir de 9 g de méthylènedioxy-3,4 aniline et de 5,9 g de bromoacétate de tert-butyle. On obtient ainsi, après recristallisation dans l'éther de pétrole, 5,2 g de (méthylènedioxy-3,4 phènyl)amino-2 acétate de tert-butyle fondant à 80°C.

### EXEMPLE 13

A une solution de 0,7 g d'[amino-2 N-(diméthlamino-4 phényl) acétamido]-2 acétate de tert-butyle dans 15 cm³ de tétrahydrofuranne anhydre, on ajoute, à une température voisine de 20°C, 0,35 g d'isocyanate de méthyl-3 phényle. La solution obtenue est agitée pendant 3 heures à une température voisine 20°C puis concentrée à sec sous pression réduite (2,7 kPa) à 40°C. On obtient après recristallisation du solide résiduel dans l'acétate d'éthyle, 0,7 g de [N-(diméthyamino-4 phényl)[(méthyl-3 phényl)-3 uréidol]-2 acétamido]-2 acétate de tert-butyle fondant à 105°C.

L'[amino-2 N-(diméthylamino-4 phényl) acétamido]-2 acétate de tert-butyle peut être préparé de la manière suivante : à une solution de 1 g de [ N-(diméthylamino-4 phényl) phtalimido-2 acétamido]-2 acétate de tert-butyle dans 20 cm3 de méthanol, on ajoute 0,15 g d'hydrate d'hyrazine. Le mélange réactionnel est agité à reflux pendant 3 heures puis concentré à sec sous pression réduite (2,7 kPa) à 40°C. Le résidu est agité avec 75 cm3 d'éther diéthylique et le produit insoluble est séparé par filtration. Le filtrat est concentré à sec sous pression réduite (2,7 kPa) à 40°C. On obtient ainsi 0,7 g d'[amino-2 N-(diméthylamino-4 phényl) acétamido]-2 acétate de tert-butyle sous forme d'une huile utilisée telle quelle dans les synthèses ultérieures.

Le [N-(diméthlamino-4 phényl) phtalimido-2 acétamido]-2 acétate de tert-butyle peut être préparé de la manière suite : à une solution de 2,6 g de (diméthylamino-4 phényl)amino-2 acétate de tert-butyle dans 20 cm3 de dichloro-1,2 éthane, maintenue sous atmosphère d'argon, on ajoute 1,4 g de triéthylamine puis goutte à goutte, à une température voisine de 20°C, une solution de 3,1 g de chlorure de phtalimido-2 acétyle dans 20 cm3 de dichloro-1,2 éthane. La solution obtenue est agitée pendant 3 heures à une température voisine de 20°C, puis additionnée de 25 cm3 d'eau. La phase aqueuse est séparée par décantation puis réextraite par 2 fois 50 cm3 de dichloro-1,2 éthane. Les phases organiques sont réunies, lavées par 2 fois 10 cm³ d'eau, séchées sur sulfate de magnésium, filtrées puis concentrées à sec sous pression réduite (2,7 kPa) à 40°C. L'huile obtenue est purifiée par chromatographie sur 100 g de silice (0,063-0,2 mm) contenus dans une colonne de 2 cm de diamètre [éluant : dichlorométhane-méthanol (98-2 en volumes)] en recueillant des fractions de 15 cm3. Les fractions ne contenant que le produit cherché sont réunies et concentrées à sec sous pression réduite (2,7 kPa) à 40°C. On obtient ainsi 1,1 g de [N-(diméthlamino-4 phényl) phtalimido-2 acétamido]-2 acétate de tert-butyle fondant à 170°C.

Le (diméthylamino-4 phényl)amino-2 acétate de tert-butyle peut être préparé de la manière suivante : à une suspension de 6,3 g de dichlorhydrate de diméthylamino-4 aniline dans 50 cm3 d'acétonitrile, on ajoute, à une température voisine de 10°C, 6,1 g de triéthylamine. La suspension obtenue est agitée pendant 30 minutes à une température voisine de 20°C, puis on ajoute 3 g de bromoacétate de tert-butyle et le mélange réactionnel est agité à reflux pendant 4 heures. Après refroidissement, le produit insoluble est séparé par filtration et lavé par 15 cm3 d'acétonitrile. Les filtrats sont réunis et concentrés à sec sous pression réduite (2,7 kPa) à 40°C. L'huile résiduelle est dissoute dans 100 cm³ de dichlorométhane et la solution obtenue est lavée par 4 fois 10 cm3 d'eau. La phase organique est séchée sur sulfate de magnésium, filtrée, puis concentrée à sec sous pression réduite (2,7 kPa) à 40°C. On obtient ainsi 2,6 g de (diméthylamino-4 phényl)amino-2 acétate de tert-butyle sous forme d'une huile utilisée telle quelle dans les synthèses ultérieures.

### EXEMPLE 14

En opérant d'une manière analogue à celle décrite à l'exemple 11, mais à partir de 5,1 g d'[amino-2 N-(méthylthio-3 phényl) acétamido]-2 acétate de tert-butyle et de 2,2 g d'isocyanate de méthyl-3 phényle, on obtient après recristallisation dans un mélange d'oxyde de diisopropyle et d'acétate d'éthyle (80-20 en volumes), 2,6 g de [[(méthyl-3 phényl)-3 uréido]-2 N-(méthylthio-3 phényl) acétamido]-2 acétate de tert-butyle fondant à 135°C.

L'[amino-2 N-(méthylthio-3 phényl) acétamido]-2 acétate de tert-butyle peut être préparé d'une manière analogue à celle décrite à l'exemple 1 pour la préparation de l'[amino-2 N-(chloro-3 phényl) acétamido]-2 acétate de tert-butyle, mais à partir de 7,6 g de [N(méthylthio-3 phényl) phtalimido-2 acétamido]-2 acétate de tert-butyle, de 2,6 g d'hydrate d'hydrazine et en opérant à une température voisine de 20°C. on obtient ainsi 5,1 g d'[amino-2 M-(méthylthio-3 phényl) acétamido]-2 acétate de tert-butyle sous forme d'une huile utilisée telle quelle dans les synthèses ultérieures.

Le [N-(méthylthio-3 phényl) phtalimido-2 acétamido]-2 acétate de tert-butyle peut être préparé d'une manière analogue à celle décrite à l'exemple 1 pour la préparation du [N-(chloro-3 phényl) phtalimido-2 acétamido]-2 acétate de tert-butyle, mais à partir de 6,8 g de (méthylthio-3 phényl)amino-2 acétate de tert-butyle, de 3 g de triéthylamine et de 6 g de chlorure de phtalimido-2 acétyle. On obtient ainsi après cristallisation dans l'oxyde de diisopropyle, 7,6 g de [N-(méthylthio-3 phényl) phtalimido-2 acétamido]-2 acétate tert-butyle fondant à 141°C.

Le (méthylthio-3 phényl)amino-2 acétate de tert-butyle peut être préparé d'une manière analogue à celle décrite à l'exemple 1 pour la préparation du (chloro-3 phényl)amino-2 acétate de tert-butyle, mais à partir de 8,4 g de méthylthio-3 aniline et de 5,9 g de bromoacétate de tert-butyle. On obtient ainsi 6,8 g de (méthylthio-3 phényl)amino-2 acétate de tert-butyle sous forme d'une huile utilisée telle quelle dans les synthèses ultérieures.

### EXEMPLE 15

En opérant d'une manière analogue à celle décrite à l'exemple 11, mais à partir de 3,4 g d'[amino-2 N-(chloro-2 phényl) acétamido]-2 N-méthyl N-phényl-acétamide et de 1,4 g d'isocyanate de méthyl-3 phényle, on obtient après recristallisation dans l'acétonitrile, 2,2 g de [N-chloro-2 phényl) [(méthyl-3 phényl)-3 uréido]-2 acétamido]-2 N-méthyl N-phényl-acétamide fondant à 180°C.

L'[amino-2 N-(chloro-2 phényl) acétamido]-2 N-méthyl N-phényl-acétamide peut être préparé d'une manière analogue à celle décrite à l'exemple 4 pour la préparation de l'[amino-2 N-(trifluorométhoxy-2 phényl) acétamido]-2 acétate de tert-butyle, mais à partir de 5,6 g de [N-(chloro-2 phényl) phtalimido-2 acétamido]-2 N-méthyl N-phényl-acétamide et de 1,9 g d'hydrate d'hydrazine. On obtient ainsi 3,3 g d'[amino-2 N-(chloro-2 phényl) acétamido]-2 N-méthyl N-phényl-acétamide sous forme d'une huile utilisée telle quelle dans les synthèses ultérieures.

Le [N-(chloro-2 phényl) phtalimido-2 acétamido]-2 N-méthyl N-phényl-acétamide peut être préparé d'une manière analogue à celle décrite à l'exemple 4 pour la préparation du [phtalimido-2 N-(trifluorométhoxy-2 phényl) acétamido]-2 acétate de tert-butyle, mais à partir de 7,4 g de phtalimido-2 N-(chloro-2 phényl) acétamide, de 1,3 g d'une suspension huileuse (50 % en poids) d'hydrure de sodium et de 5,9 g de bromo-2 N-méthyl N-phényl acétamide. On obtient ainsi, après recristallisation dans l'acétate d'éthyle, 5,7 g de [N-(chloro-2 phényl) phtalimido-2 acétamido]-2 N-méthyl N-phényl-acétamide fondant à 168°C.

Le phtalimido-2 N-(chloro-2 phényl) acétamide peut être préparé d'une manière analogue à celle décrite à l'exemple 4 pour la préparation du phtalimido-2 N-(trifluorométhoxy-2 phényl) acétamide, mais à partir de 3,8 g de chloro-2 aniline, de 3,3 g de triéthylamine et de 7,2 g de chlorure de phtalimido-2 acétyle. On obtient ainsi 7,5 g de N-(chloro-2 phényl) phtalimido-2 acétamide fondant à 250°C.

Le bromo-2 N-méthyl N-phényl-acétamide peut être préparé selon la méthode décrite par C.A. BISCHOFF, Chem. Ber., 34, 2125 (1901).

### EXEMPLE 16

En opérant d'une manière analogue à celle décrite à l'exemple 11 mais à partir de 4,2g d'[amino-2 N-(méthoxy-2 phényl) acétamido]-2 acétate de tert-butyle et de 1,9 g d'isocyanate de méthyl-3 phényle, on obtient après recristallisation dans l'acétate d'éthyle, 3 g de [N-(méthoxy-2 phényl) [(méthyl-3 phényl)-3 uréido]-2 acétamido]-2 acétate de tert-butyle fondant à 171°C.

L'[amino-2 N-(méthoxy-2 phényl) acétamido]-2 acétate de tert-butyle peut être préparé d'une manière analogue à celle décrite à l'exemple 1 pour la préparation de l'[amino-2 N-(chloro-3 phényl) acétamido]-2 acétate de tert-butyle, mais à partir de 8,4 g de [N-(méthoxy-2 phényl) phtalimido-2 acétamido]-2 acétate de tert-butyle, de 3 g d'hydrate d'hyrazine et en opérant à une température voisine de 20°C. On obtient ainsi 4,2 g d'amino-2 N-(méthoxy-2 phényl) acétamido]-2 acétate de tert-butyle sous forme d'une huile utilisée telle quelle dans les synthèses ultérieures.

Le [N-(méthoxy-2 phényl) phtalimido-2 acétamido]-2 acétate de tert-butyle peut être préparé d'une manière analogue à celle décrite à l'exemple 1 pour la préparation du [N-(chloro-3 phényl) phtalimido-2 acétamido]-2 acétate de tert-butyle, mais à partir de 6,4 g de (méthoxy-2 phényl)amino-2 acétate de tert- butyle, de 2,7 g de triéthylamine et de 6 g de chlorure de phtalimido-2 acétyle. On obtient ainsi après recristallisation dans l'oxyde de diisopropyle, 8,4 g de [N-(méthoxy-2 phényl) phtalimido-2 acétamido]-2 acétate de tert-butyle fondant à 144°C.

Le (méthoxy-2 phényl)amino-2 acétate de tert-butyle peut être préparé d'une manière analogue à celle décrite à l'exemple 1 pour la préparation du (chloro-3 phényl) amino-2 acétate de tert-butyle, mais à partir de 7,4 g de méthoxy-2 aniline et de 5,9 g de bromoacétate de tert-butyle. On obtient ainsi, 6,4 g de (méthoxy-2 phényl)amino-2 acétate de tert-butyle sous forme d'une huile utilisée telle quelle dans les synthèses ultérieures.

### EXEMPLE 17

A une suspension de 3,7 g d'acide [(méthyl-3 phényl)-3 uréido]-2 acétique dans 230 cm³ de dichloro-1,2 étane, maintenue sous atmosphère d'argon, on ajoute à une température voisine de 20°C, 4,5 g de (nitro-4 phényl)amino-2 acétate de tert-butyle. Le mélange réactionnel est chauffé sous agitation jusqu'au reflux du solvant. On ajoute alors, gouttes à goutte, en maintenant le reflux, 2,12 g de chlorure de sufinyle. L'addition terminée, on poursuit le chauffage à reflux pendant 10 minutes, puis le mélange réactionnel est refroidi à une température vosine de 10°C et versé dans une solution de 15 g d'hyrogénocarbonate de sodium dans 300 cm³ d'eau. La phase aqueuse est séparée par décantation et réextraite par 2 fois 50 cm3 de dichloro-1,2 éthane. Les phases organiques sont réunies, lavées par 3 fois 20 cm3 d'eau, séchées sur sulfate de magnésium, filtrées puis concentrées à sec sous pression réduite (2,7 kPa) à 40°C. Le résidu est purifié par chromatographie sur 200 g de silice (0,063-0,2 mm) contenus daas une colonne de 3,5 cm de diamètre [éluant : cyclohexane-acétate d'éthyle (50-50 en volumes)] en recueillant des fractions de 30 cm3. Les fractions 16 à 25 sont réunies et concentrées à sec sous pression réduite (2,7 kPa) à 40°C. On obtient après recristallisation dans l'acétate d'éthyle, 2,9 g de [[(méthyl-3 phényl)-3 uréido]-2 N-(nitro-4 phényl)-acétamido]-2 acétate de tert-butyle fondant à 152°C.

Le (nitro-4 phényl)amino-2 acétate de tert-butyle peut être préparé de la manière suivante : un mélange de 6,9 g de nitro-4 aniline, de 19,5 g de bromoacétate de tert-butyle et de 9,24 g d'hydrogénocarbonate de sodium est chauffé sous agitation et sous atmosphère d'argon, à une température de 160°C, pendant 2 heures et 30 minutes puis refroidi à une température voisine de 20°C et versé dans un mélange de 150 cm3 d'eau et 150 cm3 d'acétate d'éthyle. La phase aqueuse est séparée par décantation et réextraite par 3 fois 50 cm3 d'acétate d'éthyle. Les phases organiques sont réunies, lavées par 3 fois 20 cm3 d'eau, séchées sur sulfate de magnésium, filtrées puis concentrées à sec sous pression réduite (2,7 kPa) à 40°C. On obtient après recristallisation dans un mélange d'acétate d'éthyle et de cyclohexane (50-50 en volumes), 7,7 g de (nitro-4 phényl)amino-2 acétate de tert-butyle fondant à 124°C.

L'acide [(méthyl-3 phényl)-3 uréido]-2 acétique peut être préparé de la manière suivante : à une solution de 7,5 g de glycine et de 8,4 g d'hydrogénocarbonate de sodium dans 250 cm3 d'eau, on ajoute, à une température voisine de 20°C, 13,3 g d'isocyanate de méthyl-3 phényle. Le mélange réactionnel est agité pendant 18 heures à une température voisine de 20°C puis le produit insoluble est séparé par filtration et lavé par 2 fois 30 cm3 d'eau puis par 2 fois 30 cm3 d'acétate d'éthyle. Les filtrats sont réunis, la phase aqueuse est séparée par décantation et acidifiée par une solution aqueuse d'acide chlorhydrique 5 N jusqu'à un pH voisin de 1. Le solide formé est séparé par filtration, lavé par 2 fois 30 cm3 d'eau puis par 2 fois 30 cm3 d'acétate d'éthyle et séché à l'air. On obtient ainsi 16,3 g d'acide [(méthyl-3 phényl-3 uréido]-2 acétique fondant à 225°C.

### EXEMPLE 18

En opérant d'une manière analogue à celle décrite à l'exemple 17 mais à partir de 1,04 g d'aide [(méthyl-3 phényl)-3 uréido]-2 acétique, de 1,4 g de (dichloro-2,3 phényl)amino-2 acétate de tert-butyle et de 0,6 g de chlorure de sulfinyle, on obtient après recristallisation dans l'acétonitrile, 0,3 g de [N-(dichloro-2,3 phényl) [(méthyl-3 phényl)-3 uréido]-2 acétamido]-2 acétate de tert-butyle fondant à 135°C.

Le (dichloro-2,3 phényl)amino-2 acétate tert-butyle peut être préparé de la manière suivante : à une solution de 32,4 g de dichloro-2,3 aniline dans 100 cm³ d'acétonitrile on ajoute, à une température voisine de 20°C, 16,8 g d'hydrogénocarbonate de sodium, puis une solution de 39 g de bromoacétate de tert-butyle dans 50 cm3 d'acétonitrile. Le mélange réactionnel est agité à reflux pendant 48 heures puis refroidi à une température voisine de 20°C. Le produit insoluble est séparé par filtration et lavé par 50 cm3 d'acétonitrile. Les filtrats sont réunis et concentrés à sec sous pression réduite (2,7 kPa) à 40°C. Le résidu est purifié par chromatographie sur 300 g de silice (0,063-0,2 mm) contenus dans une colonne de 4 cm de diamètre [éluant : cyclohexane-acétate d'éthyle (90-10 en volumes)] en recueillent des fractions de 50 cm3. Les fractions ne contenant que le produit recherché sont réunies et concentrées à sec sous pression réduite (2,7 kPa) à 40°C. On obtient ainsi 33 g de (dichloro-2,3 phényl)amino-2 acétate de tert-butyle sous forme d'une huile utilisée telle quelle dans les synthèses ultérieures.

### EXEMPLE 19

En opérant d'une manière analogue à celle décrite à l'exemple 17, mais à partir de 3,12 g d'acide [(méthyl-3 phényl)-3 uréido]-2 acétique, de 4,3 g de (bromo-2 phényl)amino-2 acétate de tert-butyle et de 2 g de chlorure de sulfinyle, on obtient après recristallisation dans un mélange d'oxyde de diispropyle et d'acétonitrile (80-20 en volumes), 0,6 g de [N-(bromo-2 phényl) [(méthyl-3 phényl)-3 uréido]-2 acétamido]-2 acétate de tert-butyle fondant à 158°C.

Le (bromo-2 phényl)amino-2 acétate de tert-butyle peut être préparé d'une manière analogue à celle décrite à l'exemple 18 pour la préparation du (dichoro-2,3 phényl)amino-2 acétate de tert-butyle, mais à partir de 34,4 g de bromo-2 aniline, de 8,4 g d'hydrogénocarbonate de sodium et de 19,5 g de bromoacétate de tert-butyle. On obtient ainsi 17,4 g de (bromo-2 phényl)amino-2 acétate de tert-butyle sous forme d'une huile utilisée telle quelle dans les synthèses ultérieures.

### EXEMPLE 20

En opérant d'une manière analogue à celle décrite à l'exemple 17, mais à partir de 2,08 g d'acide [(méthyl-3 phényl)-3 uréido]-2 acétique, de 3,07 g de (trifluorométhylthio-3 phényl)amino-2 acétate de tert-butyle et de 1,3 g de chlorure de sulfinyle, on obtient après recristallisation dans l'oxyde de diisopropyle, 0,8 g de [[(méthyl-3 phényl)-3 uréido]-2 N-(trifluorométhylthio-3 phényl) acétamido]-2 acétate de tert-butyle fondant à 112°C.

Le (trifluorométhylthio-3 phényl)amino-2 acétate de tert-butyle peut être préparé d'une manière analogue à celle décrite à l'exemple 18 pour la préparation du (dichloro-2,3 phényl)amino-2 acétate de tert-butyle, mais à partir de 19,3 g de trifluorométhylthio-3 aniline, de 8,4 g d'hydrogénocarbonate de sodium et de 19,5 g de bromoacétate de tert-butyle. On obtient ainsi 25,5 g de (trifluorométhylthio-3 phényl)amino-2 acétate de tert-butyle sous forme d'une huile utilisée telle quelle dans les synthèses ultérieures.

### EXEMPLE 21

A une solution de 5,5 g de [N-(méthyl-2 phényl) phtalimido-2 acétamido]-2 acétate de tert-butyle dans 90 cm3 de dichlorométhane on ajoute, à une température voisine de 0°C, 2,13 g de méthylhydrazine. Le mélange réactionnel est agité pendant 30 heures à une température voisine de 20°C puis pendant 1 heure au reflux. Après refroidissement, on ajoute 100 cm3 d'eau, agite et sépare par décantation la phase aqueuse qui est réextraite par 2 fois 60 cm3 de dichlorométhane. Les phases organiques sont réunies, lavées par 2 fois 15 cm3 d'eau, séchées sur sulfate de magnésium, filtrées puis concentrées à sec sous pression réduite (2,7 kPa) à 40°. on obtient ainsi l'[amino-2 N-(méthyl-2 phényl) acétamido]-2 acétate de tert-butyle sous forme d'une huile qui est dissoute dans 40 cm3 de tétrahydrofuranne anhydre. On ajoute à cette solution 1,77 g d'isocyanate de méthyl-3 phényle puis aigte le mélange réactionnel pendant 1 heure à une température voisine de 20°C et concentre à sec sous pression réduite (2,7 kPa) à 40°C. On obtient après recristallisation dans l'éther de pétrole, 1,05 g de [N-(méthyl-2 phényl) [(méthyl-3 phényl)-3 uréido]-2 acétamido]-2 acétate de tert-butyle fondant à 133°C.

Le [N-(méthyl-2 phényl) phtalimido-2 acétamido]-2 acétate de tert-butyle peut être préparé d'une manière analogue à celle décrite à l'exemple 1 pour la préparation du [N-(chloro-3 phényl) phtalimido-2 acétamido]-2 acétate de tert-butyle, mais à partir de 5,2 g de (méthyl-2 phényl)amino-2 acétate de tert-butyle, de 2,6 g de triéthylamine et de 5,3 g de chlorure de phtalimido-2 acétyle. On obtient ainsi, après recristallisation dans l'acétate d'éthyle, 5,5 g de [N-(méthyl-2 phényl) phtalimido-2 acétamido]-2 acétate de tert-butyle fondant à 140°C.

Le (méthyl-2 phényl)amino-2 acétate de tert-butyle peut être préparé d'une manière analogue à celle décrite à l'exemple 18 pour la préparation du (dichloro-2,3 phényl)amino-2 acétate de tert-butyle, mais à partir de 6,4 g de méthyl-2 aniline, de 2,5 g d'hydrogénocarbonate de sodium et de 5,85 g de bromoacétate de tert-butyle. On obtient ainsi 5,2 g de (méthyl-2 phényl)amino-2 acétate de tert-butyle sous forme d'une huile utilisée dans les synthèses ultérieures.

### EXEMPLE 22

On opère d'une manière analogue à celle décrite à l'exemple 17 mais à partir de 1,8 g de (chloro-4 phényl) amino-2 acétate de tert-butyle, de 1,56 g d'acide [(méthyl-3 phényl)-3 uréido]-2 acétique et de 0,89 g de chlorure de thionyle. Le produit obtenu est purifié par chromatographie sur 60 g de silice (0,04-0,063 mm) contenus dans une colonne de 2,5 cm de diamètre [éluant : cyclohexane- acétate d'éthyle (80-20 en volumes)] en utilisant une surpression d'azote de 40 kPa et en recueillant des fractions de 20 cm3. des fractions 21 à 25 sont réunies et concentrées à sec sous pression réduite (2,7 kPa) à 40°C. On obtient, après cristallisation dans l'acétonitrile, 0,5 g de [N-(chloro-4 phényl) [(méthyl-3 phényl)-3 uréido]-2 acétamido]-2 acétate de tert-butyle fondant à 125°C.

Le (chloro-4 phényl) amino-2 acétate de tert-butyle peut être préparé de la manière suivante : à une solution de 12,7 g de chloro-4 aniline dans 100 cm³ d'acétonitrile, on ajoute 9,7 g de bromoacétate de tert-butyle et le mélange est agité à reflux pendant 3 heures. Le produit insoluble est séparé par filtration et le filtrat est concentré à sec sous pression réduite (2,7 kPa) à 40°C. Le produit obtenu est purifié par chromatographie sur 150 g de silice (0,065-0,2 mm) contenus dans une colonne de 2 cm de diamètre [éluant : cyclohexane-acétate d'éthyle (70-30 en volumes)] en recueillant des fractions de 20 cm³. Après concentration sous pression réduite (2,7 kPa) à 40°C, on obtient 11,9 g de (chloro-4 phényl) amino-2 acétatede tert-butyle sous form d'une huile utilisée telle quelle dans les synthèses ultérieures.

### EXEMPLE 23

On opère d'une manière analogue à celle décrite à l'exemple 1 mais à partir de 2,3 g d'[amino-2 N-(méthoxy-3 phényl) acétamido]-2 acétate de tert-butyle et de 1,1 g d'isocyanate de méthyl-3 phényle. Le produit obtenu est purifié par chromatographie sur 150 g de silice (0,04-0,063 mm) contenus dans une colonne de 3,5 cm de diamètre [éluant : cyclohexane-acétate d'éthyle (70-30 en volumes)] en utilisant une surpression d'azote de 40 kPa et en recueillent des fractions de 20 cm3. Les fractions 10 à 16 sont réunies et concentrées à sec sous pression réduite (2,7 kPa) à 40°C. On obtient, après recristallisation dans l'oxyde de diisopropyle, 2,2 g de [N-(méthoxy-3 phényl) [(méthyl-3 phényl)-3 uréido]-2 acétamido]-2 acétate de tert-butyle fondant à environ 60°C.

L'[amino-2 N-(méthoxy-3 phényl) acétamido]-2 acétate de tert-butyle peut être préparé d'une manière analogue à celle décrite à l'exemple 1 pour la préparation de l'[(amino-2 N-(chloro-3 phényl) acétamido]-2 acétate de tert-butyle, mais à partir de 3,4 g de [N-(méthoxy-3 phényl) phtalimido-2 acétamido]-2 acétate de tert-butyle et de 0,8 g d'hydrate d'hydrazine. On obtient ainsi 2,0 g d'[amino-2 N-(méthoxy-3 phényl) acétamido]-2 acétate de tert-butyle sous forme d'une huile utilisée telle quelle dans les synthèses ultérieures.

Le [N-(méthoxy-3 phényl) phtalimido-2 acétamido]-2 acétate de tert-butyle peut être préparé de la manière suivante : à une solution de 6,3 g de [chloro-2 N-(méthoxy-3 phényl) acetamido]-2 acétate de tert-butyle dans 100 cm3 de diméthylformamide, on ajoute 7,5 g de phtalimide potassé. Le mélange est agité à une température voisine de 100°C pendant 5 heures puis versé dans 1000 cm3 d'eau. Le produit insoluble est séparé par filtration, lavé par 3 fois 60 cm³ d'eau et séché à l'air. On obtient après recristallisation dans l'éther diisopropylique, 6,7 g de [N-(méthoxy-3 phényl) phtalimido-2 acétamido]-2 acétate de tert-butyle fondant à 138°C.

Le [chloro-2 N-(méthoxy-3 phényl) acetamido]-2 acétate de tert-butyle peut être préparé de la manière suivante : à une solution de 7,9 g de (méthoxy-3 phényl)amino-2 acétate de tert-butyle et de 6,7 g de triéthylamine dans 50 cm3 de dichloro-1,2 éthane maintenue à une température voisine de 15°C, on ajoute 5,7 g de chlorure de chloracétyle. Le mélange est agité pendant 6 heures à une température voisine de 60°C. Après refroidissement, le mélange est lavé par 3 fois 100 cm3 d'eau. La phase organique est sechée sur sulfate de magnésium et concentrée à sec sous pression réduite (2,7 kPa) à 40°C. On obtient ainsi, après recristallisation dans l'éther diisopropylique, 6,3 g de [chloro-2 N-(méthoxy-3 phényl) acétamido]-2 acétate de tert-butyle fondant à 110°C.

Le (méthoxy-3 phényl)amino-2 acétate de tert-butyle peut être préparé d'une manière analogue à celle decrite à l'exemple 1 pour la préparation du (chloro-3 phényl)amino-2 acétate de tert-butyle, mais à partir de 12,4 g de méthoxy-4 aniline et de 9,75 g de bromoacétate de tert-butyle. Le produit obtenu est purifié par chromatographie sur 200 g de silice (0,063-0,200 mm) contenus dans une colonne de 7,0 cm de diamètre (éluant : dichlorométhane) en recueillant des fractions de 60 cm₃. Les fractions 10 à 20 sont réunies et concentrées à sec sous pression réduite (2,7 kPa) à 40°C. On obtient ainsi 9,8 g de (méthoxy-3 phényl)amino-2 acétate de tert-butyle sous forme d'une huile utilisée telle quelle dans les synthèses ultérieures.

### EXEMPLE 24

A une solution de 1,2 g de [N-(diméthylamino-4 phényl)[(imidazolyl-1) carboxamido]-2 acétamido]-2 acétate de tert-butyle dans 30 cm3 de toluène, on ajoute 0,83 g de méthylthio-3 aniline et le mélange est agité à reflux pendant 4 heures. Après refroidissement, on ajoute 30 cm3 d'acétate d'éthyle, puis la solution obtenue est lavée successivement par 30 cm3 d'eau, par 2 fois 30 cm3 d'une solution aqueuse 1N d'acide chlorhydrique, par 2 fois 30 cm3 d'une solution aqueuse saturée d'hydrogénocarbonate de sodium et par 30 cm3 d'une solution aqueuse saturée de chlorure de sodium. La phase organique est séchée sur sulfate de magnésium puis concentrée à sec sous pressoin réduite (2,7 kPa) à 40°C. Le résidu est purifié par chromatographie sur 100 g de silice (0,065-0,200 mm) contenus dans une colonne de 2,7 cm de diamètre (éluant : chlorure de méthylène) en recueillant des fractions de 70 cm3. Les fractions 24 à 32 sont réunies et concentrées à sec sous pression réduite (2,7 kPa) à 40°C. On obtient après recristallisation dans l'acétonitrile, 0,40 g de [N-(diméthylamino-4 phényl)[(méthylthio-3 phényl)-3 uréido]-2 acétamido]-2 acétate de tert-butyle fondant à 160°C.

Le [N-(diméthylamino-4 phényl) [(imidazolyl-1) carboxamido]-2 acétamido]-2 acétate de tert-butyle peut être préparé de la manière suivante : à une solution de 0,58 g de N,N'-diimidazole carbonyle dans 20 cm³ de tétrahydrofuranne anhydre, on ajoute une solution de 1,2 g d'[amino-2 N-(diméthylamino-4 phényl) acétamido]-2 acétate de tert-butyle dans 15 cm3 de tétrahydrofuranne anhydre. La solution est agitée pendant 3 heures à une température voisine de 20°C, puis concentrée à sec sous pression réduite (2,7 kPa) à 40°C. Le résidu est dissous dans 30 cm3 d'acétate d'éthyle et la solution obtenue est lavée successivement par 4 fois 20 cm3 d'eau et par 25 cm3 d'une solution aquesse saturée de chlorure de sodium. La phase organique est séchée sur sulfate de magnésium puis concentrée à sec sous pression réduite (2,7 kPa) à 40°C. On obtient après recristallisation dans l'acétate d'éthyle 1,2 g de [N-(diméthylamino-4 phényl) [(imidazolyl-1) carboxamido]-2 acétamido]-2 acétate de tert-butyle fondant à 110°C.

### EXEMPLE 25

A une solution de 1,1 g d'[amino-2 N-(diméthylamino-4 phényl) acétamido]-2 acétate d tert-butyle dans 35 cm3 de dichloro-1,2 éthane agitée à une température voisine de 25°C, on ajoute 0,55 g de triéthlamine, puis 0,9 g de chlorure d'indolecarbonyle-2 en solution dans 35 cm3 de dichoro-1,2 éthane. Le mélange réactionnel est agité pendant 18 heures à une température voisine de 25°C. On ajoute alors 250 cm3 de dichlorméthane puis 125 cm3 d'une solution aqueuse saturée d'hydrogénocarbonate de sodium. La phase organique est lavée par 2 fois 125 cm3 d'eau, séchée sur sulfate de magnésium et concentrée à sec sous pression réduite (2,7 kPa) à 40°C. On obtient après recristallisation dans l'acétate d'éthyle 0,35 g de N-[N-(diméthylamino-4 N-tert-butoxycarbonylméthy phényl) carbamolyméthyl] indolecarboxamide-2 fondant à 230°C.

Le chlorure d'idolecarbonyle-2 peut être préparé de la manière suivante : à une suspension de 1,85 g d'acide indolecarboxylique-2 dans 40 cm³ d'oxyde de diéthyle anhydre à une température voisine de 5°C, on ajoute 0,1 cm3 de diméthylformamide, puis 1,5 g de dichlorure d'oxalyle en solution dans 10 cm3 d'oxyde de diéthyle anhydre. Le mélange réactionnel est agité à une température voisine de 25°C pendant 2 heures. La phase éthérée est concentrée à sec sous pression réduite (2,7 pKa) à 30°C. On obtient ainsi 1,8 g de chlorure d'indolecarbonyle-2 fondant à 120°C.

### EXEMPLE 26

A une solution de 1,85 g de {[N-(méthoxy-3 phényl) N-(N-méthyl N-phénl-carbamoylméthyl) carbamoylméthyl]-3 uréido}-3 benzoate d'éthyl dans 75 cm3 d'un mélange eau-tétrahydrofuranne-dioxanne (30-40-30 en volumes), on ajoute 5 cm3 d'une solution aqueuse 1N de soude. Le mélange est agité pendant 16 heures à une température voisine de 25°C, puis concentré à environ 40 cm3 sous pression réduite (2,7kPa) à 10°C. La solution obtenue est diluée par 50 cm3 d'eau, lavée par 2 fois 50 cm3 d'acétate d'éthyle, acidifiée à pH 3 avec une solution 4N d'aide chlorhydrique et extraite par 2 fois 30 cm3 d'acétate d'éthyle. Les phases organiques sont réunies, lavées par 30 cm3 d'eau, séchées sur sulfate de magnésium et concentrées à sec sous pression réduite (2,7kPa) à 30°C. On obtient, après recristallisation dans un mélange acétonitrile-diméthylformamide (90-10 en volumes), 0,9 g d'acide {[N-(méthoxy-3 phényl) N-(N-méthyl N-phényl-carbamoylméthyl) carbamolméthyl]-3 uréido}-3 benzïque fondant à 222°C.

Le {[N-(méthoxy-3 phényl) N-(N-méthyl N-phényl-carbamoylméthyl) carbamoylméthyl]-3 uréido}-3 benzoate d'éthyle peut être préparé da la manière suivante : à une solution de 2,1 g de [N-(méthoxy-3 phényl) isocyanatoacétamido]-2 N-méthyl N-phényl-acétamide dans 80 cm3 de tétrahydrofuranne anhydre on ajoute 0,94 g d'amino-3 benzoate d'éthyl. Le mélange est agité à une température voisine de 25°C pendant 18 heures puis concentré à sec sous pression réduite (2,7kPa) à 40°C. Le produit brut obtenu est purifié par chromatographie sur 80 g de silice (0,065-0,20 mm) contenus dans une colonne de 2,5 cm de diamètre [éluant : méthanol-dichlorométhane (20-80 en volumes)] en utilisant une surpression de 40 kPa d'azote et en recueillant des fractions de 80 cm3. Les fractions 8 à 11 sont réunies et concentrées à sec sous pression réduite (2,7kPa) à 40°C. On obtient ainsi, 1,9 g de {[N-(méthoxy-3 phényl) N-(N-méthyl N-phényl-carbamoylméthyl) carbamoylméthyl]-3 uréido}-3 benzoate d'éthyle, sous forme d'une meringue utilisée telle quelle dans les synthèses ultérieures.

Le [N-(méthoxy-3 phényl) isocyanatoacétamido]-2 N-méthyl N-phényl-acétamide peut être préparé de la manière suivante : à une solution de 1,55 g de (méthoxy-3 phényl) amino-2 N-méthyl N-phényl-acétamide et de 0,5 g de pyridine dans 30 cm3 de tétrahydrofuranne anhydre maintenue à une température voisine de 5°C, on ajoute 0,87 g de chlorure d'isocyanatoacétyle en solution dans 10 cm3 de tétrahydrofuranne anhydre puis le mélange est agité pendant 3 heures à une température voisine de 25°C. Le produit insoluble est séparé par filtration et le filtrat est concentré à sec sous pression réduite (2,7kPa) à 30°C. On obtient ainse 1,9 g de [N-(méthoxy-3 phényl) isocyanatoacétamido]-2 N-méthyl N-phényl-acétamide sous forme d'une huile utilisée telle quelle dans les synthèses ultérieures.

Le (méthoxy-3 phényl) amino-2 N-méthyl N-phényl-acétamide peut être préparé de la manière suivante : à une solution de 5,2 g de [N-(méthoxy-3 phényl) trifluoroacétamido]-2 N-méthyl N-phényl-acétamide dans 80 cm3 d'éthanol, on ajoute, à une température voisine de 20°C, 14,2 cm3 d'une solution aqueuse 2N d'hydroxyde de sodium. Le mélange réactionnel est agité à reflux pendant 5 minutes puis l'éthanol est éliminé par évaporation sous pression réduite (2,7kPa) à 40°C. On ajoute au résidu 150 cm3 d'acétate d'éthyle et sépare la phase aqueuse par décantation. La phase organique est lavée par 5 fois 20 cm3 d'eau, séchée sur sulfate de magnésium, filtrée puis concentrée à sec sous pression réduite (2,7kPa) à 40°C. L'huile résiduelle est purifiée par chromatographie sur 100 g de silice (0,063-0,2mm) contenus dans une colonne de 2,5 cm de diamètre (éluant : dichlorométhane) en recueillant des fractions de 20 cm3. Les fractions ne contenant que le produit cherché sont réunies et concentrées à sec sous pression réduite (2,7kPa) à 40°C. On obtient ainsi 2,5 g de (méthoxy-3 phényl) amino-2 N-méthyl N-phényl-acétamide sous forme d'une huile utilisée telle quelle dans les synthèses ultérieures.

Le [N-(méthoxy-3 phényl) trifluoroacétamido]-2 N-méthyl N-phényl-acétamide peut être préparé de la manière suivante : à une solution de 5 g de N-(méthoxy-3 phényl) trifluoroacétamide dans 80 cm3 de tétrahydrofuranne anhydre maintenue sous atmosphère d'argon, on ajoute, à une température voisine de 10°C, 1,4 g d'une suspension huileuse (50% en poids) d'hydrure de sodium et agite la suspension obtenue pendant 30 minutes à une température voisine de 20°C. On ajoute alors une solution de 7,9 g de bromo-2 N-méthyl N-phényl-acétamide dans 50 cm3 de tétrahydrofuranne anhydre et chauffe à reflux sous agitation pendant 5 heures. Le mélange réactionnel est ensuite refroidi à une température voisine de 20°C et versé dans un mélange de 100 cm3 d'eau et de 150 cm3 d'acétate d'éthyle. La phase aqueuse est séparée par décantation et réextraite par 2 fois 100 cm3 d'acétate d'éthyle. Les phases organiques sont réunies, lavées par 3 fois 100 cm3 d'eau, séchées sur sulfate de magnésium, filtrées puis concentrées à sec sous pression réduite (2,7kPa) à 40°C. L'huile obtenue est purifiée par chromatographie sur 100 g silice (0,063-0,2mm) contenus dans une colonne de 3,5 cm de diamètre (éluant : dichlorométhane) en recueillant des fractions de 20 cm3. Les fractions ne contenant que le produit cherché sont réunies et concentrées à sec sous pression réduite (2,7 kPa) à 40°C. On obtient ainsi 5,2 g de [N-(méthoxy-3 phényl) trifluoroacétamido]-2 N-méthyl N-phényl-acétamide sous forme d'une huile utilisée telle quelle dans les synthèses ultérieures.

Le N-(méthoxy-3 phényl) trifluoroacétamide peut être préparé de la manière suivante : à une solution anhydre de 3,1 g de méthoxy-3 aniline dans 25 cm3 de pyridine on ajoute à une température voisine de -20°C, 5,25 g d'anhydride trifluoroacétique. Le mélange réactionnel est agité pendant 30 minutes à une température voisine de -20°C, puis pendant 1 heure à une température voisine de 0°C et versé dans 150 cm3 d'eau refroidie à une température voisine de 0°C. L'huile insoluble est extraire par 200 cm3 d'oxyde de diéthyle et la phase organique obtenue est lavée par 2 fois 30 cm3 d'une solution aqueuse 1N d'acide chlorhydrique puis 2 fois 30 cm3 d'eau, séchée sur sulfate de magnésium et concentrée à sec sous pression réduite (2,7kPa) à 30°C. On obtient ainsi, 5,7 g de N-(méthoxy-3 phényl) trifluoroacétamide sous forme d'une huile utilisée telle quelle dans les synthèses ultérieures.

Le bromo-2 N-méthyl N-phényl-acétamide peut être préparé de la manière suivante : à une solution de 10,7 g de N-méthylaniline dans 65 cm3 de dichlorométhane, on ajoute successivement, à une température voisine de -5°C, 11,1 g de triéthylamine et une solution de 20,4 g de bromure de bromoacétyle dans 10 cm3 de dichlorométhane. La suspension obtenue est agitée pendant 2 heures à une température voisine de 20°C puis additionnée de 25 cm3 d'eau. La phase aqueuse est séparée par décantation et réextraite par 2 fois 15 cm3 de dichlorométhane. Les phases organiques sont réunies, lavées par 3 fois 25 cm3 d'eau, séchées sur sulfate de magnésium, filtrées puis concentrées à sec sous pression réduite (2,7kPa) à 40°C. L'huile résiduelle est additionnée de 100 cm3 d'éther diéthylique anhydre ; le produit insoluble est séparé par filtration et lavé par 3 fois 15 cm3 d'éther diéthylique. Les filtrats sont réunis et concentrés à sec sous pression réduite (2,7kPa) à 40°C. On obtient ainsi 20,5 g de bromo-2 N-méthyl N-phényl-acétamide sous forme d'une huile utilisée telle quelle dans les synthèses ultérieures.

Le chlorure d'isocyanatoacétyle peut être préparé selon la méthode décrite par YOSHIO IWAKURA et Coll., J. Org. Chem., 30, 1158 (1965).

### EXEMPLE 27

A une solution de 4,8 g d'{amino-2 N-[(diméthyl-3,3 pipéridino) carbonyl-2 phényl] acétamido}-2 acétate de tert-butyle dans 40 cm3 de tétrahydrofuranne anhydre on ajoute, à une température voisine de 20°C, 1,2 g d'isocyanate de méthyl-3 phényle. La solution obtenue est agitée pendant 4 heures à une température voisine de 20°C puis concentrée à sec sous pression réduite (2,7kPa) à 40°C. L'huile résiduelle est purifiée par chomatographie sur 150 g de silice (0,063-0,2mm) contenus dans une colonne de 3,5 cm de diamètre [éluant : dichlorométhane-méthanol (98-2 en volumes)] en recueillant des fractions de 50 cm3. Les fractions ne contenant que le produit cherché sont réunies et concentrées à sec sous pression réduite (2,7kPa) à 40°C. On obtient après recristallisation dans l'acétate d'éthyle, 3,2 g de {N-[(diméthyl-3,3 pipéridino) carbonyl-2 phényl] [(méthyl-3 phényl)-3 uréido]-2 acétamido}-2 acétate de tert-butyle fondant à 207°C.

L'{amino-2 N-[(diméthyl-3,3 pipéridino) carbonyl-2 phényl] acétamido}-2 acétate de tert-butyle peut être préparé de la manière suivante : à une solution de 6,3 g de {N-[(diméthyl-3,3 pipéridino) carbonyl-2 phényl] phtalimido-2 acétamido}-2 acétate de tert-butyle dans 100 cm3 de méthanol, on ajoute 1,9 g d'hydrate d'hydrazine. Le mélange réactionnel est chauffé à reflux pendant 4 heures puis concentré à sec sous pression réduite (2,7kPa) à 40°C. L'huile résiduelle est dissoute dans 200 cm3 d'acétate d'éthyle et la solution obtenue est additionnée de 50 cm3 d'eau. La phase aqueuse est séparée par décantation puis réextraite par 2 fois 30 cm3 d'acétate d'éthyle. Les phases organiques sont réunies, lavées par 2 fois 30 cm3 d'eau, séchées sur sulfate de magnésium, filtrées puis concentrées à sec sous pression réduite (2,7kPa) à 40°C. On obtient ainsi 4,8 g d'{amino-2 N-[(diméthyl-3,3 pipéridino) carbonyl-2 phényl] acétamido}-2 acétate de tert-butyle sous forme d'une huile utilisée telle quelle dans les synthèses ultérieures.

Le {N-[(diméthyl-3,3 pipéridino) carbonyl-2 phényl] phtalimido-2 acétamido}-2 acétate de tert-butyle peut être préparé de la manière suivante : à une solution de 8,4 g de N-[(diméthyl-3,3 pipéridino) carbonyl-2 phényl] phtalimido-2 acétamide en solution dans 100 cm3 de tétrahydrofuranne anhydre maintenue sous atmosphère d'argon, on ajoute, à une température voisine de 10°C, 1,05 g d'une suspension huileuse (50% en poids) d'hydrure de sodium et agite le mélange obtenu pendant 1 heure à une température voisine de 20°C. On ajoute alors une solution de 4,5 g de bromoacétate de tert-butyle dans 25 cm3 de tétrahydrofuranne anhydre et l'on agite pendant encore 3 heures à une température voisine de 20°C, puis pendant 4 heures au reflux du solvant. Le mélange réactionnel est ensuite versé dans un mélange refroidi à une température vosine de 0°C, de 30 cm3 d'eau distillée et de 200 cm3 d'acétate d'éthyle. La phase aqueuse est séparée par décantation et réextraite par 2 fois 20 cm3 d'acétate d'éthyle. Les phases organiques sont réunies lavées par 3 fois 25 cm3 d'eau, séchées sur sulfate de magnésium, filtrées puis concentrées à sec sous pression réduite (2,7kPa) à 40°C. On obtient ainsi 6,5 g de { N-[(diméthyl-3,3 pipéridino) carbonyl-2 phényl] phtalimido-2 acétamido}-2 acétate de tert-butyle sous forme d'un solide amorphe utilisé tel quel dans les synthèses ultérieures.

Le N-[(diméthyl-3,3 pipéridino) carbonyl-2 phényl] phtalimido-2 acétamide peut être préparè de la manière suivante : à une solution de 7 g de (diméthyl-3,3 pipéridino) carbonyl-2 aniline dans 150 cm3 de dichlorométhane maintenue sous atmosphère d'argon, on ajoute 3,3 g de triéthylamine puis en maintenant la température au voisinage de 20°C, une solution de 6,8 g de chlorure de phtalimido-2 acétyle dans 100 cm3 de dichlorométhane. La solution obtenue est agitée pendant 3 heures à une température voisine de 20°C, puis additionnée de 100 cm3 d'eau et de 150 cm3 de dichlorométhane. La phase aqueuse est séparée par décantation et réextraite par 2 fois 50 cm3 de dichlorométhane. Les phases organiques sont réunies, lavées par 3 fois 75 cm3 d'eau, séchées sur sulfate de magnésium, filtrées puis concentrées à sec sous pression réduite (2,7kPa) à 40C. On obtient ainsi, après recristallisation dans l'acétate d'éthyle, 10,4 g de N-[(diméthyl-3,3 pipéridino) carbonyl-2 phényl] phtalimido-2 acétamide fondant à 158°C.

La (diméthyl-3,3 pipéridino) carbonyl-2 aniline peut être préparée de la manière suivante : à une suspension de 51,6 g de chlorure stanneux dihydraté dans 64 cm3 d'une solution aqueuse 6N d'acide chlorhydrique, on ajoute 16,7 g de (diméthyl-3,3 pipéridino) carbonyl-2 nitrobenzène en maintenant la température au voisinage de 45°C. Le mélange réactionnel est ensuite chauffé à une température voisine de 85°C pendant 1 heure et 30 minutes, puis refroidi à une température voisine de 20°C et versé dans un mélange de 300 cm3 d'eau et de 250 cm3 de dichlorométhane refroidi à une température voisine de 0°C. Le mélange est alcalinisé par addition d'une solution aqueuse 11N d'ammoniac jusqu'à un pH voisin de 9. La phase aqueuse est séparée par décantation et réextraite par 2 fois 100 cm3 de dichlorométhane. Les phases organiques sont réunies, lavées par 4 fois 100 cm3 d'eau, séchées sur sulfate de magnésium, filtrées puis concentrées à sec sous pression réduite (2,7 kPa) à 40°C. On obtient ainsi 14,2 g de (diméthyl-3,3 pipéridino) carbonyl-2 aniline sous forme d'une huile utilisée telle quelle dans les synthèses ultérieures.

Le (diméthyl-3,3 pipéridino) carbonyl-2 nitronbenzène peut être préparé de la manière suivante : à une solution de 7,9 g de diméthyl-3,3 pipéridine et de 7,7 g de triéthylamine dans 100 cm3 de dichlorométhane, on ajout à une température voisine de 15°C une solution de 13,6 g de chlorure de nitro-2 benzoyle dans 25 cm3 de dichlorométhane. La suspensionest agitée pendant 2 heures à une température voisine de 20°C puis versée dans un mélange de 75 cm3 d'eau et de 100 cm3 de dichlorométhane. La phase aqueuse est séparée par décantation et réextraite par 2 fois 50 cm3 de dichlorométhane. Les phases organiques sont réunies, lavées par 3 fois 30 cm3 d'eau, puis par 30 cm3 d'une solution aqueuse 1N d'acide chlorhydrique et par 3 fois 25 cm3 d'eau, séchées sur sulfate de manésium, filtrées puis concentrées à sec sous pression réduite (2,7kPa) à 40°C. On obtient ainsi 16,8 g de (diméthyl-3,3 pipéridno) carbonyl-2 nitrobenzène sous forme d'une huile utilisée telle quelle dans les synthèses ultérieures.

### EXEMPLE 28

En opérant d'une manière analogue à celle décrite à l'exemple 27, mais à partir de 2,5 g d'{amino-2 N-[(N-méthyl-anilino) carbonyl-2 phényl] acétamido}-2 acétate de tert-butyle et de 0,83 g d'isocyanate de méthyl-3 phényle, on obtient après recristallisation dans l'acétate d'éthyle, 1,9 g de {N-[(N-méthyl-anilino) carbonyl-2 phényl] [(méthyl-3 phényl)-3 uréido]-2 acétamido)-2 acétate de tert-butyle fondant à 145°C.

L'{amino-2 N-[(N-méthyl-anilino) carbonyl-2 phényl] acétamido}-2 acétate de tert-butyle peut être préparé d'une manière analogue à celle décrite à l'exemple 27 pour la préparation de l'{amino-2 N-[(diméthyl-3,3 pipéridino) carbonyl-2 phényl] acétamido}-2 acétate de tert-butyle, mais à partir de 3,3 g de {N-[(N-méthyl-anilino) carbonyl-2 phényl] phtalimido-2 acétamido}-2 acétate de tert-butyle et de 0,93 g d'hydrate d'hydrazine. On obtient ainsi 2,5 g d'{amino-2 N-[(N-méthyl-anilino) carbonyl-2 phényl] acétamido}-2 acétate de tert-butyle sous forme d'une huile utilisée telle quelle dans les synthèses ultérieures.

Le {N-[(N-méthyl-anilino) carbonyl-2 phényl] phtalimido-2 acétamido}-2 acétate de tert-butyle peut être préparé d'un manière analogue à celle décrite à l'exemple 27 pour la préparation du {N-[(diméthyl-3,3 pipéridino) carbonyl-2 phényl] phtalimido-2 acétamido}-2 acétate de tert-butyle, mais à partir de 8,3 g de N-[(N-méthyl-anilino) carbonyl-2 phényl] phtalimido-2 acétamide, de 1,1 g d'une suspension huileuse (50% en poids) d'hydrure de sodium et de 4,5 g de bromoacétate de tert-butyle. On obtient ainsi, après recristallisation dans l'acétate d'éthyle, 3,4 g de {N-[(N-méthyl-anilino) carbonyl-2 phényl] phtalimido-2 acétamido}-2 acétate de tert-butyle fondant à 160°C.

Le N-[(N-méthyl-anilino) carbonyl-2 phényl] phtalimido-2 acétamide peut être préparé d'une manière analogue à celle décrite à l'exemple 27 pour la préparation du N-[(diméthyl-3,3 pipéridino) carbonyl-2 phényl] phtalimido-2 acétamide, mais à partir de 6,8 g de (N-méthyl-anilino) carbonyl-2 aniline, de 3,3 g de triéthylamine et de 6,8 g de chlorure de phtalimido-2 acétyle. On obtient ainsi, après recristallisation dans l'acétate d'éthyle, 10,8 g de N-[(N-méthyl-anilino) carbonyl-2 phényl] phtalimido-2 acétamide fondant à 138°C.

La (N-méthyl-anilino) carbonyl-2 aniline peut être préparée d'une manière analogue à celle décrite à l'exemple 27 pour la préparation de la (diméthyl-3,3 pipéridino) carbonyl-2 aniline, mais à partir de 50 g de chlorure stanneux dihydraté, de 70 cm3 d'une solution aquesse 6N d'acide chlorhydrique et de 17,5 g de (N-méthyl-anilino) carbonyl-2 nitrobenzène. On obtient ainsi, après recristallisation dans l'oxyde de diisopropyle, 10,3 g de (N-méthyl-anilino) carbonyl-2 aniline fondant à 127°C.

Le (N-méthyl-anilino) carbonyl-2 nitrobenzène peut être préparé d'une manière analogue à celle décrite à l'exemple 27 pour la préparation du (diméthyl-3,3 pipéridino) carbonyl-2 nitrobenzène, mais à partir de 7,2 g de N-méthyl-aniline, de 7,7 g de triéthylamine, et de 13,6 g de chlorure de nitro-2 benzoyle. On obtient ainsi 17,7 g de (N-méthyl-anilino) carbonyl-2 nitrobenzène sous forme d'une huile utilisée telle quelle dans les synthèses ultérieures.

### EXEMPLE 29

En opèrant d'une manière analogue à celle décrite à l'exemple 27, mais à partir de 2 g d'{amino-2 N-[(tétrahydro-1,2,3,4 quinolyl-1) carbonyl-2 phényl] acétamido}-2 acétate de tert-butyle et de 0,63 g d'isocyanate de méthyl-3 phényle, on obtient après recristallisation dans l'acétate d'éthyle, 1,2 g de {méthyl-3 phényl)-3 uréido]-2 N-[tétrahydro-1,2,3,4 quinolyl-1) carbonyl-2 phényl] acétamido}-2 acétate de tert-butyle fondant à 160°C.

L'{amino-2 N-[(tétrahydro-1,2,3,4 quinolyl-1) carbonyl-2 phényl] acétamido}-2 acétate de tert-butyle peut être préparé d'une manière analogue à celle décrite à l'exemple 27 pour la préparation de l'{amino-2 N-[(diméthyl-3,3 pipéridino) carbonyl-2 phényl] acétamido}-2 acétate de tert-butyle, mais à partir de 4,4 g de {phtalimido-2 N-[(tétrahydro-1,2,3,4 qinolyl-1) carbonyl-2 phényl] acétamido}-2 acétate de tert-butyle et de 1,2 g d'hydrate d'hydrazine. On obtient ainsi 2,1 g d'{amino-2 N-[tétrahydro-1,2, 3,4 quinolyl-1) carbonyl-2 phényl] acétamido}-2 acétate de tert-butyle sous forme d'une huile utilisée telle quelle dans les synthèses ultérieures.

Le {phtalimido-2 N-[(tétrahydro-1,2,3,4 quinolyl-1) carbonyl-2 phényl] acétamido}-2 acétate de tert-butyle peut être préparé d'une manière analogue à celle décrite à l'exemple 27 pour la préparation du { N-[(diméthyl-3,3 pipéridino) carbonyl-2 phényl] phtalimido-2 acétamido }-2 acétate de tert-butyle, mais à partir de 31,5 g de phtalimido-2 N-[(tétrahydro-1,2,3,4 quinoyl-1) carbonyl-2 phényl] acétamide, de 3,75 g d'une suspension huileuse (50% en poids) d'hydrure de sodium et de 16,1 g de bromoacétate de tert-butyle. On obtient ainsi, après recristallisation dans l'acétate d'éthyle, 26,5 g de {phtalimido-2 N-[(tétrahydro-1,2,3,4 quinolyl-1) carbonyl-2 phényl] acétamido}-2 acétate de tert-butyle fondant à 150°C.

Le phtalimido-2 N-[(tétrahydro-1,2,3,4 quinolyl-1) carbonyl-2 phényl] acétamide peut être préparé d'une manière analogue à celle décrite à l'exemple 27 pour la préparation du phtalimido-2 N-[(diméthyl-3,3 pipéridino) carbonyl-2 phényl] acétamide, mais à partir de 20,2 g de (tétrahydro-1,2,3,4 quinolyl-1) carbonyl-2 aniline, de 9,1 g de triéthylamine et de 19,7 g de chlorure de phtalimido-2 acétyle. On obtient ainsi, après recristallisation dans l'acétate d'éthyle , 31,6 g de phtalimido-2 N-[(tétrahydro-1,2,3,4 quinolyl-1) carbonyl-2 phényl] acétamide fondant à 130°C.

La (tétrahydro-1,2,3,4 quinolyl-1) carbonyl-2 aniline peut être préparée d'une manière analogue à celle décrite à l'exemple 27 pour la préparation de la (diméthyl-3,3 pipéridino) carbonyl-2 aniline, mais à partir de 114 g de chlorure stanneux dihydraté, de 140 cm3 d'une solution aqueuse 6N d'acide chlorhydrique et de 39,7 g de (tétrahydro-1,2,3,4 quinolyl-1) carbonyl-2 nitrobenzène. On obtient ainsi, après recristallisation dans l'acetate d'éthyle, 20,2 g de (tétrahydro-1,2,3,4 quinolyl-1) carbonyl-2 aniline fondant à 102°C.

Le (tétrahydro-1,2,3,4 quinolyl-1) carbonyl-2 nitrobenzène peut être préparé d'une manière analogue à celle décrite à l'exemple 27 pour la préparation du (diméthyl-3,3, pipéridino) carbonyl-2 nitrobenzène, mais à partir de 20 g de tétrahydro-1,2, 3,4 quinoléine, de 16,5 g de triéthylamine, et de 29,7 g de chlorure de nitro-2 benzoyle. On obtient ainsi, après recristallisation dans l'acétate d'éthyle, 39,7 g de (tétrahydro-1, 2,3,4 quinolyl-1) carbonyl-2 nitrobenzène fondant à 155°C.

### EXEMPLE 30

A une solution de 2,6 g d'[amino-2 N-(anilino-2 phényl) acétamido]-2 acétate de tert-butyle dans 20 cm3 de tétrahydrofuranne anhydre, on ajoute, à une température voisine de 20°C, 1,07 g d'isocyanate de méthyl-3 phényle. La solution obtenue est agitée pendant 2 heures à une température voisine de 20°C puis concentrée à sec sous pression réduite (2,7kPa) à 40°C. L'huile résiduelle est purifiée par chomatographie sur 150 g de silice (0,063-0,2mm) contenus dans une colonne de 2 cm de diamètre [éluant : cyclohexane-acétate d'éthyle (50-50 en volumes)] en recueillant des fractions de 20 cm3. les fractions ne contenant que le produit cherché sont réunies et concentrées à sec sous pression réduite (2,7kPa) à 40°C. On obtient après recristallisation dans l'acétate d'éthyle, 1,15 g de {N-(anilino-2 phényl) [(méthyl-3 phényl)-3 uréido]-2 acétamido}-2 acétate de tert-butyle fondant à 180°C.

L'[amino-2 N-(anilino-2 phényl) acétamido]-2 acétate de tert-butyle peut être préparé de la manière suivante : à une solution de 4,2 g de [N(anilino-2 phényl) phtalimido-2 acétamido]-2 acétate de tert-butyle dans 100 cm3 de méthanol, on ajoute 1,3 g d'hydrate d'hydrazine. Le mélange réactionnel est agité pendant 5 heures à une température voisine de 20°C, puis concentré à sec sous pression réduite (2,7kPa) à 40°C. Le résidu est traité avec 200 cm3 d'éther diéthylique et le produit insoluble est séparé par filtration. Le filtrat est concentré à sec sous pression réduite (2,7 kPa) à 40°C. On obtient ainsi 2,6 g d'[amino-2 N-(anilino-2 phényl) acétamido]-2 acétate de tert-butyle sous forme d'une huile utilisée telle quelle dans les synthèses ultérieures.

Le [N-(anilino-2 phényl) phtalimido-2 acétamido]-2 acétate de tert-butyle peut être préparé de la manière suivante : à une solution de 8,7 g de N-(anilino-2 phényl) phtalimido-2 acétamide dans 100 cm3 de tétrahydrofuranne anhydre maintenue sous atmosphère d'argon, on ajoute, à une température voisine de 10°C, 1,1 g d'une suspension huileuse (50% en poids) d'hydrure de sodium et agite la suspension obtenue pendant une heure à une température voisine de 20°C. On ajoute alors une solution de 4,6 g de bromoacétate de tert-butyle dans 20 cm3 de tétrahydrofuranne anhydre et poursuit l'agitation pendant 3 heures à une température voisine de 20°C. Le mélange réactionnel est ensuite versé dans un mélange refroidi à une température voisine de 0°C, de 100 cm3 d'eau et de 200 cm3 d'acétate d'éthyle. La phase aqueuse est séparée par décantation et réextraite par 2 fois 20 cm3 d'acétate d'éthyle. Les phases organiques sont réunies, lavées par 3 fois 25 cm3 d'eau, séchées sur sulfate de magnésium, filtrées puis concentrées à sec sous pression réduite (2,7kPa) à 40°C. On obtient ainsi 4,2 g de [N-(anilino-2 phényl) phtalimido-2 acétamido]-2 acétate de tert-butyle fondant à 205°C.

Le N-(anilino-2 phényl) phtalimido-2 acétamide peut être préparé de la manière suivante : à une solution de 9,2 g de N-phenyl diamino-1,2 benzène et de 5,1 g de triéthylamine dans 60 cm3 de dichlorométhane maintenue sous atmosphère d'argon, on ajoute, en maintenant la température au voisinage de 20°C, une solution de 11,2 g de chlorure de phtalimido-2 acétyle dans 60 cm3 de dichlorométhane. La solution obtenue est agitée pendant 2 heures à une température voisine de 20°C, puis additionnée de 100 cm3 d'eau. La phase aqueuse est séparée par décantation puis réextraite par 2 fois 50 cm3 de dichlorométhane. Les phases organiques sont réunies, lavées par 2 fois 30 cm3 d'eau, séchées sur sulfate de magnésium, filtrées puis concentrées à sec sous pression réduite (2,7kPa) à 40°C. On obtient ainsi après recristallisation dans l'acétate d'éthyle, 8,8 g de N-(anilino-2 phényl) phtalimido-2 acétamide fondant à 191°C.

### EXEMPLE 31

A une solution de 1,1 g d'(hydroxy-1 éthyl)-3 aniline dans 20 cm3 de tétrahydrofuranne anhydre, maintenue sous atmosphère d'argon, on ajoute, à une température voisine de 20°C, 3 g de [N-(méthoxy-3 phényl) isocyanatoacétamido]-2 N-méthyl N-phényl-acétamide. La solution obtenue est agitée pendant 16 heures à une température voisine de 20°C puis concentrée à sec sous pression réduite (2,7kPa) à 40°C. L'huile résiduelle est purifiée par chromatographie sur 150 g de silice (0,063-0,2 mm) contenus dans une colonne de 2,5 cm de diamètre [éluant : dichlorométhane-méthanol (98-2 en volumes)] en recueillant des fractions de 20 cm3. Les fractions ne contenant que le produit cherché sont réunies et concentrées à sec sous pression réduite (2,7kPa) à 40°C. On obtient après recristallisation dans l'oxyde de diisopropyle, 1,7 g d'{[[(hydroxy-1 éthyl)-3 phényl]-3 uréido]-2 N-(méthoxy-3 phényl) acétamido}-2 N-méthyl N-phényl-acétamide-(RS) fondant à 115°C.

### EXEMPLE 32

En opérant d'une manière analogue à celle décrite à l'exemple 31, mais à partir de 0,86 g d'(hydroxy-1 éthyl)-3 aniline et de 2,8 g de [N-(éthoxycarbonyl-2 phényl) isocyanatoacétamido]-2 N-méthyl N-phényl-acétamide, on obtient après recristallisation dans l'acétate d'éthyle, 2g d'{[[(hydroxy-1 éthyl)-3 phény]-3 uréido]-2 N-(éthoxycarbonyl-2 phényl) acétamido}-2 N-méthyl N-phényl-acétamide fondant à 195°C.

Le [N-(éthoxycarbonyl-2 phényl) isocyanatoacétamido]-2 N-méthyl N-phényl-acétamide peut être préparé d'une manière analogue à celle décrite à l'exemple 26 pour la préparation du [N-(méthoxy-3 phényl) isocyanatoacétamido]-2 N-méthyl N-phényl-acétamide, mais à partir de 1,1 g de chlorure d'isocyanatoacétyle, de 2,2 g d'(éthoxycarbonyl-2 phényl) amino-2 N-méthyl N-phényl-acétamide et de 0,65 g de pyridine. On obtient ainsi 2,8 g de [N-(éthoxycarbonyl-2 phényl) isocyanatoacétamido]-2 N-méthyl N-phényl-acétamide sous forme d'une huile utilisée telle quelle dans les synthèses ultérieures.

L'(éthoxycarbonyl-2 phényl) amino-2 N-méthyl N-phényl-acétamide peut être préparé d'une manière analogue à celle décrite à l'exemple 26 pour la préparation du (méthoxy-3 phényl) amino-2 N-méthyl N-phényl-acétamide, mais à partir de 3,1 g de [N-(éthoxycarbonyl-2 phényl) trifluoroacétamido]-2 N-méthyl N-phényl-acétamide et de 7,5 cm3 d'une solution aqueuse 2N d'hydroxyde de sodium. On obtient ainsi 2,2 g d'(éthoxycarbonyl-2 phényl) amino-2 N-méthyl N-phényl-acétamide fondant à 100°C.

Le [N-(éthoxycarbonyl-2 phényl) trifluoroacétamido]-2 N-méthyl N-phényl-acétamide peut être préparé d'une manière analogue à celle décrite à l'exemple 26 pour la préparation du [N-(méthoxy-3 phényl) trifluoroacétamido]-2 N-méthyl N-phényl-acétamide, mais à partir de 5,8 g de trifluoroacétylamino-2 benzoate d'éthyle, de 1,3 g d'une suspension huileuse (50% en poids) d'hydrure de sodium et de 7,3 g de bromo-2 N-méthyl N-phényl-acétamide. On obtient ainsi 5,7 g de [N-(éthoxycarbonyl-2 phényl) trofluoroacétamido]-2 N-méthyl N-phényl-acétamide fondant à 95°C.

Le trifluoroacétylamino-2 benzoate d'éthyle peut être préparé d'une manière analogue à celle décrite à l'exemple 26 pour la préparation du N-(méthoxy-3 phényl) trifluroacétamide, mais à partir de 4,1 g d'amino-2 benzoate d'éthyle et de 5,3 g d'anhydride trifluoroacétique. On obtient ainsi 6,4 g de trifluoroacétylamino-2 benzoate d'éthyle fondant à 78°C.

### EXEMPLE 33

En opérant d'une manière analogue à celle décrite à l'exemple 31, mais à partir de 0,86 g d'hydroxyméthyl-3 aniline et de 2,9 g de [N-(éthoxycarbonyl-2 phényl) isocyantoacétamido]-2 N-méthyl N-phenyl-acétamide, on obtient après recristallisation dans l'acétate d'éthyle 2 g de { N-(éthoxycarbonyl-2 phényl) [hydroxyméthyl-3 phényl)-3 uréido]-2 acétamido}-2 N-méthyl N-phényl-acétamide fondant à 184°C.

### EXEMPLE 34

En opérant d'une manière analogue à celle décrite à l'exemple 31, mais à partir de 1,1 g d'hydroxyméthyl-3 aniline et de 3,2 g de [N-(méthoxy-3 phényl) isocyanatoacétamido]-2 N-méthyl N-phényl-acétamide, on obtient après recristallisation dans l'acétate d'éthyle, 2,6 d'[[(hydroxyméthyl-3 phényl)-3 uréido]-2 N-(méthoxy-3 phényl) acétamido]-2 N-méthyl N-phényl-acétamide fondant à 142°C.

### EXEMPLE 35

En opérant d'une manière analogue à celle décrite à l'exemple 27, mais à partir de 3,1 g d'[amino-2 N-(éthoxycarbonyl-3 phényl) acétamido]-2 N-méthyl N-phényl-acétamide et de 1,2 g d'isocyanate de méthyl-3 phényle, on obtient après recristallisation dans l'oxyde de diisopropyle, 2 g de {N-(éthoxycarbonyl-3 phényl) [(méthyl-3 phényl)-3 uréido]-2 acétamido}-2 N-méthyl N-phényl-acétamide fondant à 88°C.

L'[amino-2 N-(éthoxycarbonyl-3 phényl) acétamido]-2 N-méthyl N-phényl-acétamide peut être préparé d'une manière analogue à celle décrite à l'exemple 27 pour la préparation de l'{amino-2 N-[(diméthyl-3,3, pipéridino) carbonyl-2 phényl] acétamido}-2 acétate de tert-butyle, mais à partir de 4,2 g de [N-(éthoxycarbonyl-3 phényl) phtalimido-2 acétamido]-2 N-méthyl N-phényl-acétamide et de 1,25 g d'hydrate d'hyrazine. On obtient ainsi 3 g d' [amino-2 N-(éthoxycarbonyl-3 phényl) acétamido]-2 N-méthyl N-phényl-acétamide sous forme d'une huile utilisée telle quelle dans les synthèses ultérieures.

Le [N-(éthoxycarbonyl-3 phényl) phtalimido-2 acétamido]-2 N-méthyl N-phényl-acétamide peut être préparé d'une manière analogue à celle décrite à l'exemple 27 pour la préparation du {N-[(diméthyl-3,3 pipéridino) carbonyl-2 phényl] phtalimido-2 acétamido}-2 acétate de tert-butyle, mais à partir de 3,9 g de N-(éthoxycarbonyl-3 phényl) phtalimido-2 acétamide, de 0,64 g d'une suspension huileuse (50% en poids) d'hydrure de sodium et de 3,3 g de bromo-2 N-méthyl N-phényl-acétamide. On obtient ainsi 4,2 g de [N-(éthoxycarbonyl-3 phényl) phtalimido-2 acétamido]-2 N-méthyl N-phényl-acétamide.

Le N-(éthoxycarbonyl-3 phényl) phtalimido-2 acétamide peut être préparé d'une manière analogue à celle décrite à l'exemple 27 pour la préparation du N-[diméthyl-3,3 pipéridino) carbonyl-2 phényl] phtalimido-2 acétamide, mais à partir de 5 g d'amino-3 benzoate d'éthyle, de 3,9 g de triéthlamine et de 8,1 g de chlorure de phtalimido-2 acétyle. On obtient ainsi, après recristallisation dans l'acétate d'éthyle, 8,7 g de N-(éthoxycarbonyl-3 phényl) phtalimido-2 acétamide fondant à 215°C.

### EXEMPLE 36

On opère d'une manière analogue à celle décrite à l'exemple 26, mais à partir de 0,5 g de {N-(N-méthyl N-phényl-carbamoylméthyl) [(méthyl-3 phényl)-3 uréido]-2 acétamido}-3 benzoate d'éthyle et de 1 cm3 d'une solution aqueuse de soude 1N. On obtient ainsi, 0,3 g d'acide {N-(N-méthyl N-phényl-carbamolyméthyl) [(méthyl-3 phényl)-3 uréido]-2 acétamido}-3 benzoïque fondant à 140°C.

### EXEMPLE 37

On opère d'une manière analogue à celle décrite à l'exemple 26, mais à partir de 2,45 g de {[N-(méthoxy-3 phényl) N-(N-méthyl N-phényl-carbamoylméthyl) carbamoylméthyl]-3 uréido}-3 phénylacétate d'éthyle et de 4,6 cm3 d'une solution aqueuse de soude 1N. On obtient ainsi, 1,6 g d'acide {[N-(méthoxy-3 phényl) N-(N-méthyl N-phényl-carbamoylméthyl) carbamoylméthyl]-3 uréido}-3 phénylacétique fondant à 120°C.

Le {[N-(méthoxy-3 phényl) N-(N-méthyl N-phényl-carbamoylméthyl) carbamoylméthyl]-3 uréido}-3 phénylacétate d'éthyle peut être préparé d'une manière analogue à celle décrite à l'exemple 26 pour la préparation du {[N-(méthoxy-3 phényl) N-(N-méthyl N-phényl-carbamoylméthyl) carbamoylméthyl]-3 uréido}-3 benzoate d'éthyle mais à partir de 2,4 g de [N-(méthoxy-3 phényl) isocyanatoacétamido]-2 N-méthyl N-phényl-acétamide et de 1,1 g d'amino-3 phénylacétate d'éthyle. Le produit brut est purifié par chromatographie sur 60 g de silice (0,063-0,2mm) contenus dans une colonne de 2,0 cm de diamètre [éluant : dichlorométhane-acétate d'éthyle (20-80 en volumes)], en recueillant de fractions de 20 cm3 . Les fractions 8 à 18 sont réunies et concentrées à sec sous pression réduite (2,7kPa) à 40°C. On obtient ainsi 2,45 g de {[N-(méthoxy-3 phényl) N-(N-méthyl N-phényl-carbamoylméthyl) carbamoylméthyl]-3 uréido}-3 phénylacétate d'éthyle sous forme d'une poudre amorphe utilisée telle quelle dans les synthèses ultèrieures.

L'amino-3 phénylacétate d'éthyle peut être préparé de la manière suivante : à une solution de 2,0 g de nitro-3 phénylacétate d'éthyle dans 20 cm3 d'éthanol, on ajoute 0,1 g de palladium sur noir à 5%. La suspension est agitée pendant 2 heures à une température voisine de 25°C sous atmosphère d'hydrogène (100kPa). Le catalyseur est séparé par filtration et le filtrat est conentré à sec sous pression réduite (2,7kPa) à 40°C. On obtient ainsi 1,7 g d'amino-3 phénylacétate d'éthyle sous forme d'une huile utilisée telle quelle dans les synthèses ultérieures.

Le nitro-3 phénylacétate d'éthyle peut être prépare selon la méthode décrite par SEGERS et A. BRUYLANTS, Bul. Soc. Chim. Belg., 64, 87 (1955).

### EXEMPLE 38

A une solution de 1,2 g de tribromure de bore dans 20 cm3 de dichlorométhane maintenue sous atmosphère d'azote à une température voisine de -55°C, on ajoute en 5 minutes une suspension de 0,8 g d'acide {[N-(méthoxy-3 phényl)N-(N-méthyl N-phényl-carbamoylméthyl) carbamoylméthyl]-3 uréido}-3 phénylacétique dans 15 cm3 de dichlorométhane. Le mélange obtenu est agité 15 minutes à une température voisine de -55°C, puis 20 heures à une température voisine de 20°C. On ajoute alors 20 cm3 d'eau puis 5 cm3 d'une solution aqueuse de soude 1N. La phase organique est séparée par décantation et la phase aqueuse est lavée par 2 fois 20 cm3 d'acétate d'éthyle, puis acidifiée avec 5 cm3 d'une solution aqueuse d'acide chlorhydrique 1N. Le précipité formé est séparé par filtration, lavé par 3 fois 5 cm3 d'eau et séché à l'air. On obtient ainsi, 0,3 g d'acide {[N-(hydroxy-3 phényl) N-(N-méthyl N-phényl-carbamoylméthyl) carbamoylméthyl]-3 uréido}-3 phénylacétique fondant à 148°C.

### EXEMPLE 39

En opèrant d'une manière analogue à celle décrite à l'exemple 1, mais à partir de 1,2 g d'[amino-2 N-(méthyl-2 phényl) acétamido]-2 N-méthyl N-phényl-acétamide et de 1,3 g d'isocyanate de méthyl-3 phényle, on obtient après recristallisation dans l'acétate d'éthyle, 2,3 g de [N-(méthyl-2 phényl) [(méthyl-3 phényl)-3 uréido]-2 acétamido]-2 N-méthyl N-phényl-acétamide fondant à 193°C.

L'[amino-2 N-(méthyl-2 phényl) acètamido]-2 N-méthyl N-phényl-acétamide peut être préparé d'une manière analogue à celle à l'exemple 4 pour la préparation de l'[amino-2 N-(trifluorométhoxy-2 phényl) acétamido]-2 acétate de tert-butyle, mais à partir de 5,3 g de [N-(méthyl-2 phényl) phtalimido-2 acétamido]-2 N-méthyl N-phényl-acétamide et 1,2 g d'hydrate d'hydrazine. On obtient ainsi 3,8 g d'[amino-2 N-(méthyl-2 phényl) acétamido]-2 N-méthyl N-phényl-acétamide sous forme d'une huile utilisée telle quelle dans les synthèses ultérieures.

Le [N-(méthyl-2 phényl) phtalimido-2 acétamido]-2 N-méthyl N-phényl-acétamide peut être préparé d'une manière analogue à celle décrite à l'exemple 4 pour la préparation du [phtlamido-2 N-(trifluorométhoxy-2 phényl) acétamido]-2 acétate de tert-butyle, mais à partir de 5,9 g de N-(méthyl-2 phényl) phtalimido-2 acétamide, de 1,15 g d'une suspension huileuse (50% en poids) d'hydrure de sodium et de 6,8 g de bromo-2 N-méthyl N-phényl-acétamide. On obtient ainsi, après recristallisation dans l'oxyde de diisopropyle, 7,1 g de [N-(méthyl-2 phényl) phtalimido-2 acétamido]-2 N-méthyl N-phényl-acétamide fondant à 120°C.

Le N-(méthyl-2 phényl) phtalidimo-2 acétamide peut être préparé d'une manière analogue à celle décrite à l'exemple 4, pour la préparation du phtalimido-2 N-(trifluorométhoxy-2 phényl) acétamide, mais à partir de 5,4 g de méthyl-2 aniline, de 6,6 g de triéthylamine et de 13,4 g de chlorure de phtalimido-2 acétyle. On obtient ainsi, après recristallisation dans l'acétate d'éthyle, 14,4 g de N-(méthyl-2 phényl) phtalimido-2 acétamide fondant à 254°C.

### EXEMPLE 40

En opérant d'une manière analogue à cell décrite à l'exemple 1, mais à partir de 2,0 g d'[amino-2 N-(méthoxy-2 phényl) acétamido]-2 N-méthyl N-phényl-acétamide et de 0,64 g d'isocyanate de méthyl-3 phényle, on obtient après recristallisation dans l'acétate d'éthyle, 1,5 g de [N-(méthoxy-2 phényl)[(méthyl-3 phényl)-3 uréido]-2 acétamido]-2 N-méthyl N-phényl-acétamide fondant à 212°C.

L'[amino-2 N-(méthoxy-2 phényl) acétamido]-2 N-méthyl N-phényl-acétamide peut être préparé d'une manière analogue à celle décrite à l'exemple 4, pour la préparation de l'[amino-2 N-(trifluorométhoxy-2 phényl) acétamido]-2 acétate de tert-butyle, mais à partir de 4,0 g de [N-(méthoxy-2 phényl) phtalimido-2 acétamido]-2 N-méthyl N-phényl-acétamide et de 1,3 g d'hydrate d'hyddrazine. On obtient ainsi 2,0 g d'[amino-2 N-(méthoxy-2 phényl) acétamido]-2 N-méthyl N-phényl-acétamide sous forme d'une huile utilisée telle quelle dans les synthèses ultérieures.

Le [N-(méthoxy-2 phényl) phtalimido-2 acétamido]-2 N-méthyl N-phényl-acétamide peut être préparé d'une manière analogue à celle décrite à l'exemple 4 pour la préparation du [phtaimido-2 N-(trifluorométhoxy-2 phényl) acétamido]-2 acétate de tert-butyle, mais à partir de 6,2 g de N-(méthoxy-2 phényl) phtalimido-2 acétamide, de 1,15 g d'une suspension huilesse (50% en poids) d'hydrure de sodium et de 6,8 g de bromo-2 N-méthyl N-phényl-acétamide.On obtient ainsi, après recristallisation dans l'acétate d'éthyle, 5,1 g de [N-(méthoxy-2 phényl) phtalimido-2 acétamido]-2 N-méthyl N-phényl-acétamide fondant à 199°C.

Le N-(méthoxy-2 phényl) phtalimido-2 acétamide peut être préparé d'une manière analogue à celle décrite à l'exemple 4 pour la préparation du phtalimido-2 N-(trifluorométhoxy-2 phényl) acétamide, mais à partir de 6,7 g de méthoxy-2 aniline, de 6,6 g de triéthylamine et de 13,4 g de chlorure de phtalimido-2 acétyle. On obtient ainsi, après recristallisation dans l'acétonitrile, 14,6 g de N-(méthoxy-2 phényl) phtalimido-2 acétamide fondant à 211°C.

### EXEMPLE 41

En opérant d'une manière analogue à celle décrite à l'exemple 1, mais à partir de 4,7 g d'[amino-2 N-(phénoxy-2 phényl) acétamido]-2 acétate de tert-butyle et de 1,9 g d'isocyanate de méthyl-3 phényle, on obtient après recristallisation dans l'acétate d'éthyle, 1,7 g de [[(méthyl-3 phényl)-3 uréido]-2 N-(phénoxy-2 phényl) acétamido]-2 acétate de tert-butyle fondant à 204°C.

L'[amino-2 N-(phénoxy-2 phényl) acétamido]-2 acétate de tert-butyle peut être préparé d'une manière analogue à celle décrite à l'exemple 4 pour la préparation de l'[amino-2 N-(trifluorométhoxy-2 phényl) acétamido]-2 acétate de tert-butyle, mais à partir de 6,3 g de [N-(phénoxy-2 phényl) phtalimido-2 acétamido]-2 acétate de tert-butyle et de 2 g d'hydrate d'hydrazine. On obtient ainsi 4,7 g d'[amino-2 N-(phénoxy-2 phényl) acétamido]-2 acétate de tert-butyle sous forme d'une huile utilisée telle quelle dans les synthèses ultérieures.

Le [N-(phénoxy-2 phényl) phtalimido-2 acétamido]-2 acétate de tert-butyle peut être préparé d'une manière analogue à celle décrite à l'exemple 4 pour la préparation du [phtalimido-2 N-(trifluorométhoxy-2 phényl) acétamido]-2 acétate de tert-butyle, mais à partir de 7,5 g de N-(phénoxy-2 phényl) phtalimido-2 acétamide , de 1,1 g d'une suspension huileuse (50% en poids) d'hydrure de sodium et de 4,3 g de bromoacétate de tert-butyle. On obtient ainsi 7,1 g de [N-(phénoxy-2 phényl) phtalimido-2 acétamido]-2 acétate de tert-butyle fondant à 145°C.

Le N-(phénony-2 phényl) phtalimido-2 acétamide peut être préparé d'une manière analogue à celle décrite à l'exemple 4 pour la préparation du phtalimido-2 N-(trifluorométhoxy-2 phényl) acétamide, mais à partir de 4,6 g de phénoxy-2 aniline, de 2,5 g de triéthylamine et de 5,6 g de chlorure de phtalimido-2 acétyle. On obtient ainsi, 8,3 g de N-(phénoxy-2 phényl) phtalimido-2 acétamide fondant à 143°C.

### EXEMPLE 42

En opérant d'une manière analogue à celle décrite à l'exemple 1, mais à partir de 5,8 g d'[amino-2 N-(biphénylyl-2 acétamido]-2 acétate de tert-butyle et de 2,3 g d'isocyanate de méthyl-3 phényle, on obtient après recristallisation dans l'acétate d'éthyle, 3,7 g de [N-(biphénylyl-2) [(méthyl-3 phényl)-3 uréido]-2 acétamido]-2 acétate de tert-butyle fondant à 177°C.

L'[amino-2 N-(biphénylyl-2) acétamido]-2 acétate de tert-butyle peut être préparé d'une manière analogue à celle décrite à l'exemple 4 pour la préparation de l'[amino-2 N-(triflurométhoxy-2 phényl) acétamido]-2 acétate de tert-butyle, mais à partir de 7,5 g de [N-(biphénylyl-2) phtalimido-2 acétamido]-2 acétate de tert-butyle et de 2,4 g d'hydrate d'hydrazine. On obtient ainsi 5,8 g d'[amino-2 N-biphénylyl-2) acétamido]-2 acétate de tert-butyle sous forme d'une huile utilisée telle quelle dans les synthèses ultérieures.

Le [N-(biphénylyl-2) phtalimido-2 acétamido]-2 acétate de tert-butyle peut être préparé d'une manière analogue à celle décrite à l'exemple 4 pour la préparation du [phtalimido-2 N-(trifluorométhoxy-2 phényl) acétamido]-2 acétate de tert-butyle mais à partir de 6,5 g de N-(biphénylyl-2) phtalimido-2 acétamide, de 0,87 g d'une suspension huileuse (50% en poids) d'hydrure de sodium et de 3,6 g de bromoacétate de tert-butyle. On obtient ainsi 8 g de [N-(biphénylyl-2) phtalimido-2 acétamido]-2 acétate de tert-butyle fondant à 145°C.

Le N-(biphénylyl-2) phtalimido-2 acétamide peut être préparé d'une manière analogue à celle décrite à l'exemple 4 pour la préparation du phtalimido-2 N-(trifluorométhoxy-2 phényl) acétamide, mais à partir de 4,2 g d'amino-2 biphényle, de 2,7 g de triéthylamine et de 5,6 g de chlorure de phtalimido-2 acétyle. On obtient ainsi 7,1 g de phtalimido-2 N-(biphénylyl-2) acétamide fondant à 204°C.

### EXEMPLE 43

En opèrant d'une manière analogue à celle décrite à l'exemple 1, mais à partir de 8,1 g d'[amino-2 N-(benzyl-2 phényl)acétamido]-2 acétate de tert-butyle et de 3 g d'isocyanate de méthyl-3 phényle, on obtient après recristallisation dans l'acétate d'éthyle, 6,6 g de [N(benzyl-2 phényl) [(méthyl-3 phényl)-3 uréido]-2 acétamido]-2 acétate de tert-butyle fondant à 183°C.

L'[amino-2 N-(benzyl-2 phényl) acétamido]-2 acétate de tert-butyle peut être préparé d'une manière analogue à celle décrite à l'exemple 4 pour la préparation de l'[amino-2 N-(trifluorométhoxy-2 phényl) acétamido]-2 acétate de tert-butyle, mais à partir de 11,2 g de [N-(benzy-2 phényl) phtalimido-2 acétamido]-2 acétate de tert-butyle et de 3,5 g d'hydrate d'hyrazine. On obtient ainsi 8,1 g d'[amino-2 N-(benzyl-2 phényl) acétamido]-2 acétate de tert-butyle sous forme d'une huile utilisée telle quelle dans les synthèses ultérieures.

Le [N-(benzyl-2 phényl) phtalimido-2 acétamido]-2 acétate de tert-butyle peut être préparé d'une manière analogue à celle décrite à l'exemple 4 pour la préparation du [phtalimido-2 N-(trifluorométhoxy-2 phényl) acétamido]-2 acétate de tert-butyle, mais à partir de 9,3 g de N-(benzyl-2 phényl) phtalimido-2 acétamide, de 1,4 g d'une suspension huileuse (50% en poids) d'hydrure de sodium et de 5,9 g de bromoacétate de tert-butyle. On obtient ainsi, 11,3 g de [N-(benzyl-2 phényl) phtalimido-2 acétamido]-2 acétate de tert-butyle fondant à 190°C.

Le N-(benzyl-2 phényl) phtalimido-2 acétamide peut être préparé d'une manière analogue à celle décrite à l'exemple 4 pour la préparation du phtalimido-2 N-(trifluorméthoxy-2 phényl) acétamide, mais à partir de 4,6 g de benzyl-2 aniline, de 3 g de triéthylamine et de 7,3 g de chlorure de phtalimido-2 acétyle. On obtient ainsi 9,3 g de N-(benzyl-2 phényl) phtalimido-2 acétamide fondant à 232°C.

### EXEMPLE 44

En opérant d'une manière analogue à celle décrite à l'exemple 1, mais à partir de 3,3 g d'[amino-2 N-(éthoxycarbonyl-3 phényl) acétamido]-2 acétate de tert-butyle et de 1,3 g d'isocyanate de méthyl-3 phényle, on obtient après recristallisation dans l'oxyde de diisopropyle, 0,85 g de [N-(éthoxycarbonyl-3 phényl) [(méthyl-3 phényl)-3 uréido]-2 acétamido]-2 acétate de tert-butyle fondant à 71°C.

L'[amino-2 N-(éthoxycarbonyl-3 phényl) acétamido]-2 acétate de tert-butyle peut être préparé d'une manière anlogue à celle décrite à l'exemple 4 pour la prépararion de l'[amino-2 N-(trifluorométhoxy-2 phényl) acétamido]-2 acétate de tert-butyle, mais à partir de 5 g de [N-(éthoxycarbonyl-3 phényl) phtalimido-2 acétamido]-2 acétate de tert-butyle et de 1,6 g d'hydrate d'hydrazine. On obtient ainsi 3,3 g d'[amino-2 N-(éthoxycarbonyl-3 phényl) acétamido]-2 acétate de tert-butyle sous forme d'une huile utilisée telle quelle dans les synthèses ultérieures.

Le N-(éthoxycarbonyl-3 phényl) [phtalimido-2 acétamido]-2 acétate de tert-butyle peut être préparé d'une manière anlogue à celle décrite à l'exemple 4 pour la préparation du [phtalimido-2 N-(trifluorméthoxy-2 phényl) acétamido]-2 acétate de tert-butyle, mais à partir de 6 g de N-(éthoxycarbonyl-3 phényl) phtalimido-2 acétamide, de 1 g d'une suspension huileuse (50% en poids) d'hydrure de sodium et de 4 g de bromoacétate de tert-butyle. On obtient ainsi 5,1 g de [N-(éthoxycarbonyl-3 phényl) phtalimido-2 acétamido]-2 acétate de tert-butyle sous forme d'une huile utilisée telle quelle dans les synthèses ultérieures.

Le N-(éthoxycarbony-3 phényl) phtalimido-2 acétamide peut être préparé d'une manière analogue à celle décrite à l'exemple 4 pour la préparation du phtalimido-2 N-(trifluorométhoxy-2 phényl) acétamide, mais à partir de 3,3 g d'amino-3 benzoate d'éthyle, de 2,4 g de triéthylamine et de 5,8 g de chlorure de phtalimido-2 acétyle. On obtient ainsi 6,2 g de N-(éthoxycarbonyl-3 phényl) phtalimido-2 acétamide fondant à 219°C.

### EXEMPLE 45

En opérant d'une manière analogue à cell décrite à l'exemple 1, mais à partir de 2,25 g d'[amino-2 N-(éthoxycarbonyl-2 phényl) acétamido]-2 N-méthyl N-phenyl-acétamide et de 0,88 g d'isocyanate de méthyl-3 phényle, on obtient après recristallisation dans l'acétate d'éthyle, 1,6 g de [N-(éthoxycarbonyl-2 phényl) [(méthyl-3 phényl)-3 uréido]-2 acétamido]-2 N-méthyl N-phényl-acétamide fondant à 201°C.

L'[amino-2 N-(éthoxycarbonyl-2 phényl) acétamido]-2 N-méthyl N-phényl-acétamide peut être préparé d'une manière analogue à celle décrite à l'exemple 4 pour la préparation de l'[amino-2 N-(trifluorométhoxy-2 phényl) acétamido]-2 acétate de tert-butyle, mais à partir de 3,0 g de [N-(éthoxycarbonyl-2 phényl) phtalimido-2 acétamido]-2 N-méthyl N-phényl-acétamide et de 0,9 g d'hydrate d'hydrazine. On obtient ainsi 2,25 g d'[amino-2 N-(éthoxycarbonyl-2 phényl) acétamido]-2 N-méthyl N-phényl-acétamide sous forme d'une huile utilisée telle quelle dans les synthèses ultérieures.

Le [N-(éthoxycarbonyl-2 phényl) phtalimido-2 acétamido]-2 N-méthyl N-phényl-acétamide peut être préparé d'une manière analogue à celle décrite à l'exemple 4 pour la préparation du [phtalimido-2 N-(trifluorométhoxy-2 phényl) acétamido]-2 acétate de tert-butyle, mais à partir de 5,4 g de N-(éthoxycarbonyl-2 phényl) phtalimido-2 acétamide, de 0,9 g d'une suspension huileuse (50%, en poids) d'hydrure de sodium et de 5,25 g de bromo-2 N-méthyl N-phényl-acétamide. On obtient ainsi après recristallisation dans l'acétate d'éthyle, 3,0 g de [N-(éthoxycarbonyl-2 phényl) phtalimido-2 acétamido]-2 N-méthyl N-phényl-acétamide fondant à 202°C.
Le N-(éthoxycarbonyl-2 phényl) phtalimido-2 acétamide peut être préparé d'une manière anlogue à celle décrite à l'exemple 4 pour la préparation du phtalimido-2 N-(trifluorométhoxy-2 phényl) acétamide, mais à partir de 4,1 g d'amino-2 benzoate d'éthyle, de 2,8 g de triéthylamine et de 5,6 g de chlorure de phtalimido-2 acétyle. On obtient ainsi, après recristallisation dans l'acétate d'éthyle, 8,7 g de N-(éthoxycarbonyl-2 phényl) phtalimido-2 acétamide fondant à 197°C.

### EXEMPLE 46

En opèrant d'une manière analogue à celle décrite à l'exemple 27, mais à partir de 2 g d'{amino-2 N-[(N-méthyl-anilino) carbonyl-2 phényl] acétamido}-2 N-méthyl N-phényl-acétamide et de 0,62 g d'isocyanate de méthyl-3 phényle, on obtient 1,45 g {N-[(N-méthyl-anilino) carbonyl-2 phényl] [(méthyl-3 phényl)-3 uréido]-2 acétamido}-2 N-méthyl N-phényl-acétamide fondant à 140°C.

L'{amino-2 N-[N-méthyl-anilino) carbonyl-2 phényl] acétamido}-2 N-méthyl N-phényl-acétamide peut être préparé d'une manière analogue à celle décrite à l'exemple 27 pour la préparation de l'{amino-2 N-[(diméthyl-3,3 pipéridino) carbonyl-2 phényl] acétamido}-2 acétate de tert-butyle, mais à partir de 2,8 g de N-méthyl {N-[(N-méthyl-anilino) carbonyl-2 phényl] phtalimido-2 acétamido}-2 N-phényl-acétamide et de 0,75 g d'hydrate d'hydrazine. On obtient ainsi 2 g d'{amino-2 N-[(N-méthyl-anilino) carbonyl-2 phényl] acètamido}-2 N-méthyl N-phényl-acétamide sous forme d'une huile utilisée telle quelle dans les synthèses ultérieures.

Le N-méthyl {N-[N-méthyl-anilino) carbonyl-2 phényl] phtalimido-2 acétamido}-2 N-phényl-acétamide peut être préparé de la manière suivante : à une suspension de 8,3 g de N-[(N-méthyl-anilino) carbonyl-2 phényl] phtalimido-2 acétamide et de 3 g de carbonate de potassium dans 50 cm3 de N,N-diméthylformamide, on ajoute, à une température voisine de 20°C, une solution de 4,6 g de bromo-2 N-méthyl N-phényl-acétamide dans 25 cm3 de N,N-diméthylformamide. Le mélange obtenu est agité pendant 120 heures à une température voisine de 20°C, puis versé dans 800 cm3 d'eau. Le produit insoluble est séparé par filtration, lavé par 4 fois 100 cm3 d'eau, séché à l'air et purifié par chromatographie sur 90 g de silice (0,04-0,063 mm) contenus dans une colonne de 3,2 cm de diamètre (éluant : dichlorométhane) en recueillant des fractions de 50 cm3. Les fractions 60 à 90 sont réunies et concentrées à sec sous pression réduite (2,7kPa) à 50°C. On obtient ainsi 2,8 g de N-méthyl {N-[(N-méthyl-anilino) carbonyl-2 phényl] phtalimido-2 acétamido}-2 N-phényl acétamide sous forme d'un solide amorphe utilisé tel quel dans les synthèses ultérieures.

### EXEMPLE 47

En opèrant d'une manière analogue à celle décrite à l'exemple 27, mais à partir de 1,45 g d'amino-2 N-(diméthyl-3,3 pipéridinocarbonylméthyl) N-[(N-méthyl-anilino) carbonyl-2 phényl] acétamide et de 0,63 g d'isocyanato-3 benzoate d'éthyle, on obtient 1 g de {{[N-(diméthyl-3,3 pipéridinocarbonylméthyl) N-[(N-méthyl-anilino) carbonyl-2 phényl] carbamoylméthyl}-3 uréido}-3 benzoate d'éthyle fondant à 225°C.

L'amino-2 N-(diméthyl-3,3 pipéridinocarbonylméthyl) N-[(N-méthyl-anilino) carbonyl-2 phényl] acétamide peut être préparé d'une manière analogue à celle décrite à l'exemple 27 pour la préparation de l'{amino-2 N-[(diméthyl-3,3 pipéridino) carbonyl-2 phényl] acétamido)-2 acétate de tert-butyle, mais à partir de 2,3 g de N-(diméthyl-3,3 pipérinocarbonylméthyl) N-[(N-méthyl-anilino) carbonyl-2 phényl] phtalimido-2 acétamide et de 0,6 g d'hydrate d'hyrazine. On obtient ainsi 1,45 g d'amino-2 N-(diméthyl-3,3 pipéridinocarbonylméthyl) N-[(N-méthyl-anilino) carbonyl-2 phényl] acétamide sous forme d'un huile utilisée telle quelle dans les synthèses ultérieures.

Le N-(diméthyl-3,3 pipéridinocarbonylméthyl) N-[(N-méthyl-anilino) carbonyl-2 phényl] phtalimido-2 acétamide peut être préparé de la manière suivante : à une solution de 6,8 g d'acide {N-[(N-méthyl-anilino) carbonyl-2 phényl] phtalimido-2 acétamido}-2 acétique et de 30 mg de N,N-diméthylamino-4 pyridine dans 150 cm3 de tétrahydrofuranne, on ajoute successivement, à une température voisine de 20°C, 2,35 g de N,N-diimidazolecarbonyle et 1,6 g de diméthyl-3,3 pipéridine. Le mélange est agité pendant 70 heures à une température voisine de 20°C puis concentré à sec sous pression réduite (2,7kPa) à 50°C. Le résidu est dissous dans 250 cm3 de dichlorométhane et la solution ainsi obtneue est lavée par 2 fois 75 cm3 d'une solution aqueuse normale d'hydroxyde de sodium puis par 100 cm3 d'eau. La phase organique est séchée sur sulfate de magnésium, filtrée et concentrée à sec sous pression réduite (2,7kPa) à 60°C. On obtient ainsi 6,6 g de N-(diméthyl-3,3 pipéridinocarbonylméthyl) N-[(N-méthyl-anilino) carbonyl-2 phényl] phtalimido-2 acétamide fondant à 180°C.

L'acide {N-[N-méthyl-anilino) carbonyl-2 phényl] phtalimido-2 acétamido}-2 acétique peut être préparé de la manière suivante : une solution de 7,9 g de {N-[(N-méthyl-anilino) carbonyl-2 phényl] phtalimido-2 acétamido}-2 acétate de tert-butyle dans 30 cm3 d'acide trifluoracétique est agitée à une temprérature voisine de 20°C pendant 24 heures puis versée dans 50 cm3 d'eau. La phase aqueuse est extraite par 3 fois 250 cm3 de dichlorométhane. Les phases organiques sont réunies, lavées par 2 fois 150 cm3 d'eau, séchées sur sulfate de magnésium, filtrées puis concentrées à sec sous pression réduite (2,7kPa) à 60°C. Le résidu est traité avec 100 cm3 d'éther de pétrole et le produit insoluble est séparé par filtration est séché sous pression réduite (15kPa) à 30°C. On obtient ainsi 6,8 g d'acide {N-[(N-méthyl-anilino) carbonyl-2 phényl] phtalimido-2 acétamido}-2 acétique sous forme d'un solide amorphe utilisé tel quel dans les synthèses ultérieures.

### EXEMPLE 48

En opérant d'une manière anlogue à celle décrite à l'exemple 27, mais à partir de 1,8 g d'[amino-2 N-(tert-butoxycarbonyl-2 phényl) acétamido]-2 acétate de tert-butyle et de 0,64 g d'isocyanate de méthyl-3 phényle, on obtient 1,3 g de {[(méthyl-3 phényl)-3 uréido]-2 N-(tert-butoxycarbonyl-2 phényl) acétamido}-2 acétate de tert-butyle fondant à 146°C.

L'[amino-2 N-(tert-butoxycarbonyl-2 phényl) acétamido]-2 acétate de tert-butyle peut être préparé d'une manière analogue à celle décrite à l'exemple 27 pour la préparation de l'{amino-2 N-[(diméthyl-3,3 pipéridino) carbonyl-2 phényl] acétamido}-2 acétate de tert-butyle, mais à partir de 2,4 g de [phtalimido-2 N-(tert-butoxycarbonyl-2 phényl) acétamido]-2 acétate de tert-butyle et de 0,7 g d'hyrate d'hydrazine. On obtient ainsi 1,8 g d'[amino-2 N-(tert-butoxycarbonyl-2 phényl) acétamido]-2 acétate de tert-butyl sous forme d'une huile utilisée telle quelle dans les synthèses ultérieures.

Le [phtalimido-2 N-(tert-butoxycarbonyl-2 phényl) acétamido]-2 acétate de tert-butyle peut être préparé d'une manière analogue à celle décrite à l'exemple 46 pour la préparation du {N-[(N-méthyl-anilino) carbonyl-2 phényl] phtalimido-2 acétamido}-2 N-méthyl N-phényl-acétamide, mais à partir de 3,8 g de (phtlimido-2 acétamido)-2 benzoate de tert-butyle, de 2,15 g de bromoacétate de tert-butyle et de 1,5 de carbonate de potassium. On obtient ainsi 2 g de [N-(tert-butoxycarbonyl-2 phényl) phtalimido]-2 acétate de tert-butyle fondant à 143°C.

Le (phtalimido-2 acétamido)-2 benzoate de tert-butyle peut être préparé d'une manière analogue à celle décrite à l'exemple 27 pour la préparation du N-[(diméthyl-3,3 pipéridino) carbonyl-2 phényl] phtalimido-2 acétamide, mais à partir de 3,3 g d'anthranilate de tert-butyle, de 4,96 g de chlorure de phtalimido-2 acétyle et de 2,24 de triéthylamine. On obtient ainsi 5,6 g de (phtalimido-2 acétamido)-2 benzoate de tert-butyle fondant à 159°C.

L'anthranilate de tert-butyle peut être préparé selon la méthode décrite par W. E. Gaines, N. B. Carson, J. Econ. Entomol., 39, 763 (1946).

### EXEMPLE 49

A une solution de 1,7 g d'{[(imidazolyl-1) carboxamido]-2 N-(méthoxy-3 phényl) acétamido}-2 N-méthyl N-phényl-acétamide dans 35 cm3 de toluène, on ajoute 1,1 g d'(amino-3 phényl)-2 éthanol. Le mélange est chauffé à reflux pendant 4 heures , puis concentré à sec sous pression réduite (2,7kPa) à 45°C. Le résidu est dissous dans 60 cm3 d'acétate d'éthyle et la solution obtenue est lavée par 20 cm3 d'une solution aqueuse d'acide chlorhydrique 2N, puis par 2 fois 25 cm3 d'eau. La phase organique est séchée sur sulfate de magnésium et concentré à sec sous pression réduite (2,7kPa) à 40°C. Le produit brut obtenu est pruifié par chromatographie sur 50 g de silice (0,065-0,205mm) contenus dans une colonne de 2,1 cm de diamètre [éluant : acétate d'éthyl-éthanol (95/5 en volumes)] en recueillant des fraction de 20 cm3. Les fractions 4 à 6 sont réunies et concentrées à sec sous pression réduite (2,7kPa) à 40°C. On obtient, après recristallisation dans un mélange oxyde de diisopropyle-acétate d'éthyl (90/10 en volumes), 0,85 g d'{{[(hydroxy-2 éthyl)-3 phényl]-3 uréido}-2 N-(méthoxy-3 phényl) acétamido}-2 N-méthyl N-phényl-acétamide fondant à 90°C.

L'{[(imidazolyl-1) carboxamido]-2 N-(méthoxy-3 phényl) acétamido}-2 N-méthyl N-phényl-acétamide peut être préparé de la manière suivante : à une solution de 3,0 g de N,N'-diimidazole carbonyle dans 30 cm3 de tétrahydrofuranne anhydre, on ajoute une solution de 3,1 g d'[amino-2 N-(méthoxy-3 phényl) acétamido]-2 N-méthyl N-phényl-acétamide dans 30 cm3 de tétrahydrofuranne anhydre. La solution est agitée pendant 16 heures à une température voisine de 25°C, puis concentrée à sec sous pression réduite (2,7kPa) à 40°C. Le résidu est dissous dans 50 cm3 d'acétate d'éthyle et la solution obtenue et lavée successivement par 4 fois 30 cm3 d'eau et par 30 cm3 d'une solution aqueuse saturée de chlorure de sodium. La phase organique est séchée sur sulfate de magnèsium puis concentrée à sec sous pression réduite (2,7kPa) à 40°C. On obtient après recristallisation dans l'acétate d'éthyle 3,5 g d'{[(imidazolyl-1) carboxamido]-2 N-(méthoxy-3 phényl) acétamido}-2 N-méthyl N-phényl-acétamide fondant à 130°C.

L'(amino-3 phényl)-2 éthanol peut être préparé selon la méthode décrit par B. CARNMALM et coll., Acta Pharm. Suedica, 11, 33 (1974).

### EXEMPLE 50

On opère d'une manière analogue à celle décrite à l'exemple 26, mais à partir de 2 g de {{[N-(méthoxy-3 phényl) N-(N-méthyl N-phényl-carbamoylméthyl) carbamoylméthyl]-3 uréido}-3 phényl}-2 propionate de méthyle-(RS) et de 3,8 cm3 d'une solution aqueuse de soude 1N. On obtient ainsi, 1,3 g d'acide {{[N-(méthoxy-3 phényl) N-(N-méthyl N-phényl-carbamoylméthyl) carbamoylméthyl]-3 uréido}-3 phényl}-2 propionique-(RS) fondant à 127°C.

Le {{[N-(méthoxy-3 phényl) N-(N-méthyl N-phényl-carbamoylméthyl) carbamoylméthyl]-3 uréido}-3 phényl}-2 propionate de méthyle-(RS) peut être préparé d'une manière analogue à celle décrite à l'exemple 49 pour la préparation de l'{{[hydroxy-2 éthyl)-3 phényl]-3 uréido}-2 N-(méthoxy-3 phényl) acétamido}-2 N-méthyl N-phényl-acétamide, mais à partir de 2,5 d'{[(imidazolyl-1) carboxamido]-2 N-(méthoxy-3 phényl) acétamido}-2 N-méthyl N-phényl-acétamide et de 2,1 g d'(amino-3 phényl)-2 propionate de méthyle-(RS). Le produit brut obtenu est purifié par chromatographie sur 50 g de silice (0,065-0,200mm) contenus dans une colonne de 2,5 cm de diamètre [éluant : chlorure de méthylène-acétate d'éthyle (60-40 en volumes) ] en recueillant des fractions de 50 cm3. Les fractions 5 à 12 sont réunies et concentrées à sec sous pression réduite (2,7kPa) à 40°C. On obtient ainsi, 2,1 g de {{[N-(méthoxy-3 phényl) N-(N-méthyl N-phényl-carbamoylméthyl) carbamoylméthyl]-3 uréido}-3 phényl}-2 propionate de méthyle-(RS) sous forme d une meringue utilisée telle quelle dans les synthèses ultérieures.

L'(amino-3 phényl)-2 propionate de méthyle-(RS) peut être préparé de la manière suivante : à une solution de 4 g de (nitro-3 phényl)-2 propionate de méthyle-(RS) dans 50 cm3 d'éthanol, on ajoute 0,3 g de palladium sur noir à 5%. La suspension est agitée pendant 2 heures à une température voisine de 25°C sous atmosphère d'hydrogène (100kPa). le catalyseur est ensuite séparé par filtration et le filtrat est concentré à sec sous pression réduite (2,7kPa) à 40°C. On obtient ainsi, 3,3 g d'(amino-3 phényl)-2 propionate de méthyle-(RS) sous forme d'une huile utilisée telle quelle dans les synthèses ultérieures.

Le (nitro-3 phényl)-2 propionate de méthyle-(RS) peut être préparé de la manière suivante : on fait buller pendant 3 heures de l'acide chlorhydrique dans une solution de 5 g de (nitro-3 phényl)-2 propionitrile-(RS) dans 40 cm3 de méthanol. Le mélange obtenu est agité à reflux pendant 30 minutes, et le produit insoluble est séparé par filtration. Le filtrat est concentré à sec sous pression réduite (2,7kPa) à 40°C. Le produit brut est purifié par chromatographie sur 80 g de silice (0,065-0,200mm) contenus dans une colonne de 3,5 cm de diamètre [éluant : éther de pétrole-acétate d'éthyle (80/20 en volumes)] en recueillant des fractions de 100 cm3. Les fractions 1 et 2 sont réunies et concentrées à sec sous pression réduite (2,7kPa) à 40°C. On obtient ainsi 4,0 g de (nitro-3 phényl)-2 propionate de méthyle-(RS) sous forme d'une huile utilisée telle quelle dans les synthèses ultérieures.

Le (nitro-3 phényl)-2 propionitrile-(RS) peut être préparé selon la méthode décrite par E. Felder et coll., J. Med Chem., 13, 559 (1970).

### EXEMPLE 51

On opère d'une manière analogue à celle décrite à l'exemple 49 mais à partir de 1,7 g d'{[(imidazolyl-1) carboxamido]-2 N-(méthoxy-3 phényl) acétamido}-2 N-méthyl N-phényl-acétamide et de 1,4 g d'(amino-3 benzyl)-5 tétrazole. Le produit brut obtenu est purifié par chromatographie sur 40 g de silice (0,065-0,200mm) contenus dans une colonne de 2 cm de diamètre [éluant : chlorure de méthylène-éthanol (90/10 en volumes)] en recueillant des fractions de 25 cm3. Les fractions 5 à 123 sont réunies et concentrées à sec sous pression réduite (2,7kPa) à 40°C. On obtient ainsi, après recristallisation dans un mélange acétate d'éthyle-oxyde de diisopropyle (10/90 en volumes), 0,2 g de {N-(méthoxy-3 phényl) {{[(tétrazolyl-5) méthyl]-3 phényl}-3 uréido}-2 acétamido}-2 N-méthyl N-phényl-acétamide fondant à 120°C.

L'(amino-3 benzyl)-5 tétrazole peut être préparé de la manière suivante : à une solution de 3,9 g de (nitro-3 benzyl)-5 tétrazole dans 80 cm3 d'éthanol, on ajoute 0,3 g de palladium sur noir à 5%. La suspension est agitée pendant 2 heures à une température voisine de 25°C sous atmosphère d'hydrogène (100kPa), Le catalyseur est séparé par filtration et le filtrat est concentré à sec sous pression réduite (2,7kPa) à 40°C. On obtient ainsi, 3,1 g d'(amino-3 benzyl)-5 tétrazole fondant à 140°C.

Le (nitro-3 benzyl)-5 tétrazole peut être préparé de la manière suivante : à une solution de 1,6 g de nitro-3 phénylacétonitrile dans 25 cm3 de diméthylformamide anhydre on ajoute 1,43 g d'azoture de sodium et 1,17 g de chlorure d'ammonium anhydre. Le mélange est agité à une température voisine de 100°C pendant 22 heures, puis concentré à sec sous pression réduite (1,2kPa)) à 80°C. Le résidu obtenu est repris par 25 cm3 d'une solution d'acide chlorhydrique 2N et le mélange obtenu est extrait par 2 fois 50 cm3 de chlorure de méthylène. Les phases organiques réunies sont séchées sur sulfate de magnésium et concentrées à sec sous pression réduite (2,7kPa) à 35°C. On obtient ainsi, 1,6 g de nitro-3 benzyl)-5 tétrazole fondant à 140°C.

Le nitro-3 phénylacétonitrile peut être préparé de la manière suivante à une solution de 20,6 g de chlorure de nitro-3 benzyle dans 120 cm3 de méthanol on ajoute 20 cm3 d'une solution aqueuse de cyanure de potassium 8,5M. Le mélange est agité à reflux pendant 4 heues puis concentré à sec sous pression réduite (2,7kPa) à 45°C. Le résidu est repris par 200 cm3 d'oxyde de diéthyle et 150 cm3 d'eau. La phase organique est séchée sur sulfate de magnésium et concentrée à sec sous pression réduite (2,7kPa) à 35°C. Le produit brut obtenu est purifié par chromatographie sur 50 g de silice (0,065-0,200mm) contenus dans une colonne de 2 cm de diamètre (éluant : chlorure de méthlyène) en recueillant de fractions de 30 cm3. Les fractions 4 à 9 sont réunies et concentrées à sec sous pression réduite (2,7kPa) à 40°C. On obtient ainsi, 11g de nitro-3 phénylacétonitrile fondant à 60°C.

### EXEMPLE 52

On opère d'une manière analogue à celle décrite à l'exemple 49, mais à partir de 1,0 g d'{[(imidazolyl-1) carboxamido]-2 N-(hydroxy-3 phényl) acétamido}-2 N-méthyl N-phényl-acétamide et de 0,6 g d'amino-3 phénylméthanol. Le produit but est purifié par chromatographie sur 40 g de silice (0,065-0,200mm) contenus dans une colonne de 2 cm de diamètre [éluant : éthanol-acétate d'éthyle (5/95 en volumes)] en recueillant des fractions de 10 cm3. Les fractions 7 à 13 sont réunies et concentrées à sec sous pression réduite (2,7kPa) à 40°C. On obtient, après recristallisation dans un mélange acétate d'éthyle-oxyde de diisopropyle (80/20 en volumes) 0,2 g d'{[(hydroxyméthyl-3 phényl)-3 uréido]-2 N-(hydroxy-3 phényl) acétamido}-2 N-méthyl N-phényl-acétamide fondant à 160°C.

L'{[(imidazolyl-1) carboxamido]-2 N-(hydroxy-3 phényl) acétamido}-2 N-méthyl N-phényl-acétamide peut être préparé d'une manière analogue à celle décrite à l'exemple 49 pour la préparation de l'{[(imidazolyl-1) carboxamido]-2 N-(méthoxy-3 phényl) acétamido}-2 N-méthyl N-phényl-acétamide, mais à partir de 0,9 g d'[amino-2 N-(hydroxy-3 phényl) acétamido]-2 N-méthyl N-phényl-acétamide et de 0,7 g de N,N'-diimidazole carbonyle. On obtient ainsi, 0,9 g d'{[(imidazolyl-1) carboxamido]-2 N-(hydroxy-3 phényl) acétamido}-2 N-méthyl N-phényl-acétamide sous forme d'une huile utilisée telle quelle dans les synthèses ultérieures.

L'[amino-2 N-(hydroxy-3 phényl) acétamido]-2 N-méthyl N-phényl-acétamide peut être préparé d'une manière analogue à celle décrite à l'exemple 4 pour la préparation de l'[amino-2 N-(trifluorméthoxy-2 phényl) acétamido]-2 acétate de tert-butyle, mais à partir de 2,0 g de [N-(hydroxy-3 phényl) phtalimido-2 acétamido]-2 N-méthyl N-phényl-acétamide et de 0,45 g d'hydrate d'hydrazine. On obtient ainsi 0,9 g d'[amino-2 N-(hydroxy-3 phényl) acétamido]-2 N-méthyl N-phényl-acétamide sous forme d'une huile utilisée telle quelle dans les synthèses ultérieures.

Le [N-(hydroxy-3 phényl) phtalimido-2 acétamido]-2 N-méthyl N-phényl-acétamide peut être préparé de la manière suivante : à 13,3 cm3 d'une solution chlorométhylènique de tribromure de bore 1M maintenue sous atmosphère d'azote à une température voisine de -50°C, on ajoute en 10 minutes une solution de 2 g de [N-(méthoxy-3 phényl) phtalimido-2 acétamido]-2 N-méthyl N-phényl-acétamide dans 20 cm3 de chlorure de méthylène. Le mélange obtenu est agité 30 minutes à une température voisine de -50°C, puis 16 heures à une température voisine de 20°C. On ajoute alors 25 cm3 d'eau puis 25 cm3 de chlorure de méthylène. La phase organique est séparée par décantation, lavée par 4 fois 30 cm3 d'eau, séchée sur sulfate de magnésium et concentrée à sec sous pression réduite (2,7kPa) à 40°C. On obtient ainsi, 0,9 g de [N-(hydroxy-3 phényl) phtalimido-2 acétamido]-2 N-méthyl N-phényl-acétamide sous forme d'une huile utilisée telle quelle dans les synthèses ultérieures.

### EXEMPLE 53

On opère d'une manière analogue à celle décrite à l'exemple 1, mais à partir de 1,0 g d'[amino-2 N-(éthoxy-3 phényl) acétamido]-2 N-méthyl N-phényl-acétamide et de 0,33 g d'isocyanate de méthyl-3 phényle. Le produit brut obtenu est purifié par chromatographie sur 40 g de silice (0,065-0,200mm) contenus dans une colonne de 2,0 cm de diamètre [éluant : cyclohexane-acétate d'éthyle (30/70 en volumes)] en recueillant des fractions de 20 cm3. Les fractions 5 à 13 sont réunies et contrées à sec sous pression réduite (2,7kPa) à 40°C. On obtient, après recristallisation dans un mélange acétate d'éthyle-oxyde de diisopropyle (10/90 en volumes), 0,3 g de {N-(éthoxy-3 phényl) [(méthyl-3 phényl)-3 uréido]-2 acétamido}-2 N-méthyl N-phényl-acétamide fondant à 98°C.

L'[amino-2 N-(éthoxy-3 phényl) acétamido]-2 N-méthyl N-phényl-acétamide peut être préparé d'une manière analogue à celle décrite à l'exemple 4 pour la préparation de l'[amino-2 N-[(trifluorométhoxy-2 phényl) acétamido]-2 acétate de tert-butyle, mais à partir de 1,2 g de [N-(éthoxy-3 phényl) phtalimido-2 acétamido]-2 N-méthyl N-phényl-acétamide et de 0,28 g d'hydrate d'hyrazine. On obtient ainsi, 1 g d'[amino-2 N-(éthoxy-3 phényl) acétamido]-2 N-méthyl N-phényl-acétamide sous forme d'une huile utilisée telle quelle dans les synthèses ultérieures.

Le [N-(éthoxy-3 phényl) phtalimido-2 acétamido ]-2 N-méthyl N-phényl-acétamide peut être préparé de la manière suivante : à une solution de 2,7 g de [N-(hydroxy-3 phényl) phtalimido-2 acétamido]-2 N-méthyl N-phényl-acétamide dans 15 cm3 de N,N-diméthylformamide, on ajoute à une température voisine de 10°C, 0,24 g d'une suspension huileuse (50% en poids) d'hydrure de sodium. La suspension est agitée 30 minutes à une température voisine de 10°C puis on ajoute 1,0 g d'iodure d'éthyle. Le mélange est agité pendant 2 heures à une température voisine de 25°C puis verse dans un mélange de 150 cm3 d'eau et 200 cm3 d'acétate d'éthyle. La phase organique est séparée par décantation, lavée par 2 fois 100 cm3 d'eau, séchée sur sulfate de magnésium et concentrée à sec sous pression réduite (2,7kPa) à 35°C. Le produit brut est purifié par chromatographie sur 50 g de silice (0,063-0,2mm) contenus dans une colonne de 2,5 cm de diamètre [éluant : cyclohexane-acétate d'éthyle (40-60 en volumes)], en recueillant des fractions de 20 cm3. Les fractions 4 à 7 sont réunies et concentrées à sec sous pression réduite (2,7kPa) à 40°C. On obtient ainsi 1,2 g de [N-(éthoxy-3 phényl) phtalimido-2 acétamido]-2 N-méthyl N-phényl-acétamide sous forme d'une huile utilisée telle quelle dans les synthèses ultérieures.

### EXEMPLE 54

On opère d'une manière analogue à celle décrite à l'exemple 1, mais à partir de 0,8 g d'[amino-2 N-(méthoxy-3 phényl) acétamido]-2 N-(fluoro-2 phényl) N-méthyl-acétamide et de 0,32 g d'isocyanate de méthyl-3 phényle. Le produit obtenu est purifié par chromatographie sur 40 g de silice (0,065-0,200mm) contenus dans une colonne de 2 cm de diamètre [éluant : chlorure de méthylène-éthanol (95-5 en volumes)] en recueillant des fractions de 20 cm3. les fractions 8 à 12 sont réunies et concentrées à sec sous pression réduite (2,7kPa) à 40°C. On obtient, après recristallisation dans un mélange oxyde de diisopropyle-acétonitrile (90/10 en volumes), 0,7 g de N-(fluoro-2 phényl) (N-(méthoxy-3 phényl) [(méthyl-3 phényl)-3 uréido]-2 acétamido)-2 N-méthyl-acétamide fondant à 110°C.

L'[amino-2 N-(méthoxy-3 phényl) acétamido]-2 N-(fluoro-2 phényl) N-méthyl-acétamide peut être préparé d'une manière analogue à celle décrite à l'exemple 4 pour la préparation de l'[amino-2 N-[(trifluorométhoxy-2 phényl) acétamido]-2 acétate de tert-butyle, mais à partir de 6,2 g de N-(fluoro-2 phényl) [N-(méthoxy-3 phényl) phtalimido-2 acétamido]-2 N-méthyl-acétamide et de 1,3 g d'hydrate d'hydrazine. On obtient ainsi, 4,0 g d'[amino-2 N-(méthoxy-3 phényl) acétamido]-2 N-(fluoro-2 phényl) N-méthyl-acétamide sous forme d'une huile utilisée telle quelle dans les synthèses ultérieures.

Le N-(fluoro-2 phényl) [N-(méthoxy-3 phényl) phtalimido-2 acétamido]-2 N-méthyl-acétamide peut être préparé de la manière suivante : à une suspension de 9,2 g d'acide N-(méthoxy-3 phényl) phtalimido-2 acétamido]-2 acétique dans 150 cm3 de dichloro-1,2 éthane on ajoute 3,5 g de dichlorure d'oxalyle puis 0,2 cm3 de diméthylformamide. Le mélange est agité 2 heures à une température voisine de 25°C, puis on ajoute 3,1 g de fluoro-2 N-méthyl aniline et 2 g de pyridine en solution dans 20 cm3 de dichloro-1,2 éthane. La solution est agitée 2 heures à une température voisine de 25°C, puis lavée par 2 fois 100 cm3 d'eau. La phase organique est séchée sur sulfate de magnésium et concentrée à sec sous pression réduite (2,7kPa) à 40°C. Le produit brut obtenu est purifié par chromatographie sur 80 g de silice (0,065-0,200mm) contenus dans une colonne de 4,0 cm de diamètre [éluant : chlorure de méthylène-méthanol (98-2 en volumes)] en receuillant des fractions de 30 cm3. Les fractions 5 à 13 sont réunies et concentrées à sec sous pression réduite (2,7kPa) à 40°C. On obtient ainsi, 6,2 g de N-(fluoro-2 phényl) [N-(méthoxy-3 phényl) phtalimido-2 acétamido]-2 N-méthyl-acétamide sous forme d'une meringue utilisée telle quelle dans les synthèses ultérieures.

L'acide [N-(méthoxy-3 phényl) phtalimido-2 acétamido]-2 acétique peut être préparé de la manière suivante : à une solution de 19 g de [N-(méthoxy-3 phényl) phtalimido-2 acétamido]-2 acétate de tert-butyle dans 220 cm3 de dichlorométhane, on ajoute 32,9 g d'acide trifluoroacétique. La solution obtenue est agitée à reflux pendant 4 heures, puis concentrée à sec sous pression réduite (2,7kPa) à 40°C. On obtient, après recristallisation dans l'éther diisopropylique, 16 g d'acide [N-(méthoxy-3 phényl) phtalimido-2 acétamido]-2 acétique fondant à 198°C.

La fluoro-2 N-méthyl-aniline peut être préparée de la manière suivante : à une suspension de 4,9 g d'hydrure de lithium-aluminium dans 100 cm3 de tétrahydrofuranne anhydre maintenue à une température voisine de 25°C, on ajoute en 15 minutes une solution de 12,2 g de fluoro-2 formanilide dans 100 cm3 de tétrahydrofuranne anhydre. Le mélange est agité à une température voisine de 25°C pendant 3 heures. Après refroidissement à une température voisine de 5°C, on ajoute successivement 5,7 cm3 d'eau, 4,2 cm3 d'une solution aqueuse 5N de soude puis 19 cm3 d'eau. La suspension obtenue est agitée pendant 30 minutes et on ajoute 150 cm3 d'oxyde de diéthyle. Le produit insoluble est séparé par filtration et le filtrat est concentré à sec sous pression réduite (2,7kPa) à 40°C. Le résidu est dissous dans 60 cm3 de dichlorométhane et la solution est séchée sur sulfate de magnésium puis concentrée à sec sous pression réduite (2,7kPa) à 40°C. On obtient ainsi 7,2 g de fluoro-2 N-méthyl-aniline sous forme d'une huile utilisée telle quelle dans les synthèses ultérieures.

Le fluoro-2 formanilide peut être préparé de la manière suivante : à une solution de 10,8 g de méthylate de sodium dans 100 cm3 de diméthylformide anhydre, on ajoute 11 g de fluoro-2 aniline. Le mélange est chauffé à reflux pendant 2 heures en distillant le méthanol formé, puis concentré à sec sous pression réduite (0,01kPa) à 60°C. Le résidu est repris par 1 litre d'eau et 300 cm3 d'oxyde de diéthyle. La phase organique est séparée par décantation, séchée sur sulfate de magnésium et concentrée à sec sous pression réduite (2,7kPa) à 30°C. On obtient ainsi 12,2 g de fluoro-2 formanilide sous forme d'une huile utilisée telle quelle dans les synthèses ultérieures.

### EXEMPLE 55

On opère d'une manière analogue à celle décrite à l'exemple 26, mais à partir de 3,1 g de {{N-[(dihydro-3,4 2H-benothiazine-1,4 yl-4)-2 oxo-2 éthyl] N-(méthoxy-3 phényl) carbamoylméthyl}-3 uréido}-3 benzoate d'éthyle et de 5,5 cm3 d'une solution aqueuse de soude 1N. On obtient ainsi, 1,6 g d'acide {{N-[(dihydro-3,4 2H-benzothiazine-1,4 yl-4)-2 oxo-2 éthyl] N-(méthoxy-3 phényl) carbamoylméthyl}-3 uréido}-3 benzoïque fondant à 165°C.

Le {{N-[(dihydro-3,4 2H-benzothiazine-1,4 yl-4)-2 oxo-2 éthyl] N-(méthoxy-3 phényl) carbamoylméthyl}-3 uréido}-3 benzoate d'éthyle peut être préparé d'une manière analogue à celle décrite à l'exemple 49 pour la préparation de l'{{[(hydroxy-2 éthyl)-3 phényl]-3 uréido}-2 N-(méthoxy-3 phényl) acétamido)-2 N-méthyl N-phényl-acétamide, mais à partir de 3,0 g de N-[(dihydro-3,4 2H-benzothiazine-1,4 yl-4)-2 oxo-2 éthyl] [(imidazolyl-1) carboxamido]-2 N-(méthoxy-3 phényl) acétamide et de 2,2 g d'amino-3 benzoate d'éthyle. Le produit obtenu est purifié par chromatographie sur 150 g de silice (0,065-0,200mm) contenus dans une colonne de 5 cm de diamètre [éluant : chlorure de méthylène-acétate d'éthyle (70-30 en volumes)] en recueillant des fractions de 30 cm3. Les fractions 24 à 36 sont réunies et concentrées à sec sous pression réduite (2,7kPa) à 40°C. On obtient ainsi, 3,2 g de {{N-[(dihydro-3,4 2H-benzothiazine-1,4 yl-4)-2 oxo-2 éthyl] N-(méthoxy-3 phényl) carbamoylméthyl}-3 uréido}-3 benzoate d'éthyle sous forme d'une meringue utilisée telle quelle dans les synthèses ultérieures.

Le N-[(dihydro-3,4 2H-benzothiazine-1,4 yl-4)-2 oxo-2 éthyl] [(imidazolyl-1) carboxamido]-2 N-(méthoxy-3 phényl) acétamide peut être préparé d'une manière analogue à celle décrite à l'exemple 49 pour la préparation de l'{[(imidazolyl-1) carboxamido]-2 N-(méthoxy-3 phényl) acétamido}-2 N-méthyl N-phényl-acétamide, mais à partir de 3,6 g d'amino-2 N-[(dihydro-3,4 2H-benzothiazine-1,4 yl-4)-2 oxo-2 éthyl] N-(méthoxy-3 phényl) acétamide et de 2,6 g de N,N'-dimidazole carbonyle. On obtient ainsi, 3,1 g de N-[(dihydro-3,4 2H-benzothiazine-1,4 yl-4)-2 oxo-2 éthyl] [(imidazolyl-1) carboxamido]-2 N-(méthoxy-3 phényl) acétamide sous forme d'une poudre amorphe utilisée telle quelle dans les synthèses ultérieures.

L'amino-2 N-[(dihydro-3,4 2H-benzothiazine-1,4 yl-4)-2 oxo-2 éthyl] N-(méthoxy-3 phényl) acétamide peut être préparé d'une manière analogue à celle décrite à l'exemple 4, pour la préparation de l'[amino-2 N-(trifluorométhoxy-2) phényl acétamido]-2 acétate de tert-butyle, mais à partir de 8,0 g de N-[(dihydro-3,4 2H-benzothiazine-1,4 yl-4)-2 oxo-2 éthyl] N-(méthoxy-3 phényl) phtalimido-2 acétamide et de 1,6 g d'hydrate d'hydrazine. On obtient ainsi, 5,9 g d'amino-2 N-[(dihydro-3,4 2H-benzothiazine-1,4 yl-4)-2 oxo-2 éthyl] N-(méthoxy-3 phényl) acétamide sous forme d'une huile utilisée telle quelle dans les synthèses ultérieures.

Le N-[(dihydro-3,4 2H-benzothiazine-1,4 yl-4)-2 oxo-2 éthyl] N-(méthoxy-3 phényl) phtalimido-2 acétamide peut être préparé d'une manière analogue à celle décrite à l'exemple 54 pour la préparation du N-(fluoro-2 phényl) [N-(méthoxy-3 phényl) phtalimido-2 acétamido]-2 N-méthyl-acétamide, mais à partir de 9,2 g d'acide [N-(méthoxy-3 phényl) phtalimido-2 acétamido]-2 acétique, de 3,5 g de dichlorure d'oxalyle, de 3,8 g de dihydro-3,4 2H-benzothizine-1,4 et de 2 g de pyridine. On obtient après recristallisation dans un mélange acétonitrile-oxyde de diisopropyle (35/65 en volumes), 8,2 g de N-[(dihydro-3,4 2H-benzothiazine-1,4 yl-4)-2 oxo-2 éthyl] N-(méthoxy-3 phényl) phtalimido-2 acétamide fondant à 150°C.

La dihydro-3,4 2H-benzothiazine-1,4 peut êtr préparée selon la méthode décrite par C. C. J. CULVENOR et coll., J. Chem. Soc., 278 (1949).

### EXEMPLE 56

On opère d'une manière analogue à celle décrite à l'exemple 26 mais à partir de 0,7 g de {{N-(méthoxy-3 phényl) N-[(tétrahydro-1,2,3,4 quinolyl-1)-2 oxo-2 éthyl] carbamoylméthyl)-3 uréido}-3 benzoate d'éthyle et de 1,3 cm3 d'une solution aqueuse de soude 1N. On obtient ainsi, 0,2 g d'acide {{N-(méthoxy-3 phényl) N-[(tétrahydro-1,2,3,4 quinolyl-1)-2 oxo-2 éthyl] carbamoylméthyl}-3 uréido}-3 benzoïque fondant à 190°C.

Le {{N-(méthoxy-3 phényl) N-[(tétrahydro-1,2,3,4 quinolyl-1)-2 oxo-2 éthyl] carbamoylméthyl}-3 uréido}-3 benzoate d'éthyle peut être préparé d'une manière analogue à celle décrite à l'exemple 49 pour la préparation de l'{{[(hydroxy-2 éthyl)-3 phényl]-3 uréido}-2 N-(méthoxy-3 phényl) acétamido}-2 N-méthyl N-phényl-acétamide, mais à partir de 1,2 g d'[(imidazolyl-1) carboxamido]-2 N-(méthoxy-3 phényl) N-[(tétrahydro-1,2,3,4 quinolyl-1)-2 oxo-2 éthyl] acétamide et de 0,85 g d'amino-3 benzoate d'éthyle. Le produit obtenu est purifié par chromatographie sur 40 g de silice (0,065-0,200mm) contenus dans une colonne de 2 cm de diamètre [éluant : chlorure de méthylène-acétate d'éthyl (70-30 en volumes)] en recueillant des fractions de 20 cm3. Les fractions 31 à 40 sont réunies et concentrées à sec sous pression réduite (2,4kPa) à 40°C. On obtient ainsi, 0,7 g de {{N-(méthoxy-3 phényl) N-[(tétrahydro-1,2,3,4 quinolyl-1)-2 oxo-2 éthyl] carbamoylmétyl}-3 uréido}-3 benzoate d'éthyle sous forme d'une meringue utilisée telle quelle dans les synthèses ultérieues.

L'[(imidazolyl-1) carboxamido]-2 N-(méthoxy-3 phényl) N-[(tétrahydro-1,2,3,4 quinolyl-1)-2 oxo-2 éthyl] acétamide peut être préparé d'une manière analogue à celle décrite à l'exemple 49 pour la préparation de l'{[(imidazolyl-1) carboxamido]-2 N-(méthoxy-3 phényl) acétamido}-2 N-méthyl N-phényl-acétamide, mais à partir de 5,0 g d'amino-2 N-(méthoxy-3 phényl) N-[(tétrahydro-1,2,3,4 quinolyl-1)-2 oxo-2 éthyl] acétamide et de 4,2 g de N,N'-diimidazole carbonyle. On obtient ainsi, 2,3 g d'[(imidazolyl-1) carboxamido]-2 N-(méthoxy-3 phényl) N-[(tétrahydro-1,2,3,4 quinolyl-1)-2 oxo-2 éthyl] acétamide sous forme d'une meringue utilisée telle quelle dans les synthèses ultérieures.

L'amino-2 N-(méthoxy-3 phényl) N-[(tétrahydro-1,2,3,4 quinolyl-1)-2 oxo-2 éthyl] acétamide peut être préparé d'une manière analogue à celle décrite à l'exemple 4, pour la préparation de l'[amino-2 N-(trifluorométhoxy-2) phényl acétamido]-2 acétate de tert-butyle, mais à partir de 8,5 g de N-(méthoxy-3 phényl) phtalimido-2 N-[(tétrahydro-1,2,3,4 quinolyl-1)-2 oxo-2 éthyl] acétamide et de 1,7 g d'hydrate d'hydrazine. on obtient ainsi, 5 g d'amino-2 N-(méthoxy-3 phényl) N-[(tétrahydro-1,2,3,4 quinolyl-1)-2 oxo-2 éthyl] acétamide sous forme d'une huile utilisée telle quelle dans les synthèses ultérieures.

Le N-(méthoxy-3 phényl) phtalimido-2 N-[(tétrahydro-1,2,3,4 quinolyl-1)-2 oxo-2 éthyl] acétamide peut être préparé d'une manière analogue à celle décrite à l'exemple 54 pour la préparation du N-(fluro-2 phényl) [N-(méthoxy-3 phényl) phtalimido-2 acétamido]-2 N-méthyl-acétamide, mais à partir de 8,0 g d'acide [N-(méthoxy-3 phényl) phatalimido-2 acétamido]-2 acétique, de 3,0 g de dichlorure d'oxalyle, de 3,5 g de tétrahydro-1,2,3,4 quinoléine et de 1,8 g de pyridine. On obtient ainsi, 8,8 g de N-(méthoxy-3 phényl) phtalimido-2 N-[(tétrahydro-1,2,3,4 quinolyl-1)-2 oxo-2 éthyl] acétamide sous forme d'une huile utilisée telle quelle dans les synthèses ultérieures.

La présente invention concerne également les médicaments constitués par au moins un composé de formule (I) à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour adminstration parentérale, peuvent être de préférence des solutions aqueuses des solutions non aqueuses, des suspensions ou des émulsions. Comme solvant ou véhicule, on peut employer de l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersents et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour adminstration rectale sont les suppositoires ou les capsules rectales qui contiennent , outre le produit actif, des excipients tels que le beurre de cacao, des glycérides semi-synthétiques ou des polyéthylèneglycols.

Les compositions pour administration topique peuvent être par exemple des crèmes, des pommades, des lotions, des collyres, des collutoires, des gouttes nasales ou des aérosols.

En thérapeutique humaine, les composés selon l'invention sont particulièrement utiles dans le traitement et la prévention des désordres liés à la CCK et à la gastrine au niveau du système nerveux et de l'appareil gastrointestinal. Ces composés peuvent donc être utilisés dans le traitement et la prévention des psychoses, des troubles anxieux, de la maladie de Parkinson, de la diskinésie tardive, du syndrome du colon irritable, de la pancréatite aiguë, des ulcères, des désordres de la motilité intestinale, de certaines tumeurs de l'oesophage inférieur, du colon et de l'intestin, et comme potentialisateur de l'activité analgésique des médicaments analgésiques narcotiques et non narcotiques et comme régulateur de l'appétit.

Les doses dépendent de l'effet recherché, de la durée du traitement et de la voie d'administration utilisée ; elles sont généralement comprises entre 0,05 g et 1 g par jour par voie orale pour un adulte avec des doses unitaires allant de 10 mg à 500 mg de substance active.

D'une façon générale, le médicin traitant déterminera la posologie appropriée en fonction de l'âge, du poids et de tous les autres facteurs propres au sujet à traiter.

Les exemples suivants illustrent les compositions selon l'invention :

### EXEMPLE A

On prépare, selon la technique habituelle, des gélules dosées à 50 mg de produit ayant la composition suivante :

| | |
|---|---|
| - acide {[N-(méthoxy-3 phényl) N-(N-méthyl N-phényl-carbamoylméthyl) carbamoylméthyl]-3 uréido}-3 benzoïque | 50 mg |
| - cellulose | 18 mg |
| - lactose | 55 mg |
| - silice colloïdale | 1 mg |
| - carboxyméthylamidon sodique | 10 mg |
| - talc | 10 mg |
| - stéarate de magnésium | 1 mg |

### EXEMPLE B

On prépare, selon la techhique habituelle, des comprimés dosés à 50 mg de produit actif ayant la composition suivante :

| | |
|---|---|
| - {N-[(diméthyl-3,3 pipéridino) carbonyl-2 phényl] [(méthyl-3 phényl)-3 uréido]-2 acétamido}-2 acétate de tert-butyle | 50 mg |
| - lactose | 104 mg |
| - cellulose | 40 mg |
| - polyvidone | 10 mg |
| - carboxyméthylamidon sodique | 22 mg |
| - talc | 10 mg |
| - stéarate de magnésium | 2 mg |
| - silice colloïdale | 2 mg |
| - mélange d'hydroxyméthylcellulose, glycérine, oxyde de titane (72-3,5-24,5) | q.s.p. 1 comprimé pelliculé terminé à 245 mg |

### EXEMPLE C

On prépare un solution injectable contenant 10 mg de produit actif ayant la composition suivante :

| | |
|---|---|
| - acide {[N-(méthoxy-3 phényl) N-(N-méthyl N-phényl-carbamoylméthyl) carbamoylméthyl]-3 uréido}-3 phénylacétique | 10 mg |
| - acide benzoïque | 80 mg |
| - alcool benzylique | 0,06 cm³ |
| - benzoate de sodium | 80 mg |
| - éthanol à 95 % | 0,4 cm³ |
| - hydroxyde de sodium | 24 mg |
| - propylène glycol | 1,6 cm³ |
| - eau | q.s.p. 4 cm³ |

## Revendications (Revendications pour l'(les) Etat(s) contractant(s) suivant(s): AT, BE, CH, DE, DK, FR, GB, IT, LI, LU, NL, SE)

1. Composés de formule : 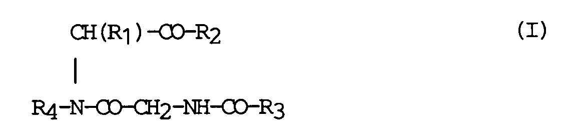 dans laquelle
- R₁ représente un atome d'hydrogène, un radical alkyle, alcoxycarbonyle ou phényle (éventuellement substitué par un ou plusieurs substituants choisis parmi les atomes d'halogène et les radicaux alkyle, alcoxy, alkylthio, nitro et amino),
- R₂ représente un radical alcoxy, cycloalkyloxy (éventuellement) substitué par au moins un radical alkyle), cycloalkylalkyloxy, phénylalkyloxy, polyfluoroalkyloxy, cinnamyloxy, -NR₅R₆
- R₃ représente un radical phénylamino (dont le noyau phényle est éventuellement substitué par un ou plusieurs substituants choisis parmi les atomes d'halogène et les radicaux alkyle, alcoxy, alkylthio, carboxy, hydroxy, mono ou polyhydroxyalkyle, nitro, amino, acyle, cyano, sulfamoyle, carbamoyle, benzoyle, alcoxycarbonyle, phénylhydroxyméthyle, pipéridino, hydroxyiminoalkyle, alcoxyiminoalkyle, alkylsulfinyle, tétrazolyl-5, tétrazolyl-5 alkyle, sulfo, -alk-O-CO-alk, -alk-O-alk, -alk-COOX, -O-alk-COOX, -alk'-COOX, -CH=CH-COOX, -CO-COOX, -alk-SO₃H et -S-alk-COOX), phényle (éventuellement substitué par un ou plusieurs substituants choisis parmi les atomes d'halogène et les radicaux alkyle, alcoxy et alkylthio), naphtyle, indolyle ou quinolyle,
- R₄ représente un radical phényle substitué par un ou plusieurs substituants choisis parmi les atomes d'halogène et les radicaux alkyle, alcoxy, hydroxy, trifluorométhyle, nitro, alkylthio, alcoxycarbonyle, carboxy, acylamino, méthylènedioxy, trifluorométhoxy, trifluorométhylthio, phénoxy, phényle, benzyle, phénylamino et -CO-NR₁₂R₁₃,
- R₅ et R₆ identiques ou différents représentent un atome d'hydrogène, un radical alkyle, phényle (éventuellement substitué par un ou plusieurs substituants choisis parmi les atomes d'halogène et les radicaux alkyle, alcoxy et alkylthio), indanyle, cycloalkylalkyle, cycloalkyle, phénylalkyle ou bien R₅ et R₆ forment ensemble avec l'atome d'azote auquel ils sont rattachés un hétérocycle mono ou polycyclique saturé ou insaturé contenant 4 à 9 atomes de carbone et un ou plusieurs hétéroatomes (O,N,S) et éventuellement substitué par un ou plusieurs radicaux alkyle, alcoxy, alcoxycarbonyle, dialkylcarbamoyle, phényle ou en combinaison avec un atome de carbone de l'hétérocycle par un cycle spiromonocyclique à 4 ou 5 chaînons et contenant éventuellement un ou plusieurs hétéroatomes (O,S,N),
- R₁₂ représente un atome d'hydrogène ou un radical alkyle,
- R₁₃ représente un radical phényle,
ou bien NR₁₂R₁₃ représente un radical pipéridino éventuellement substitué par un radical alkyle, tétrahydro-1,2,3,4 quinolyl-1, pyrrolidinyl-1 éventuellement substitué par au moins un radical phényle ou pipérazinyl-1 éventuellement substitué en -4 par un radical alkyle
- alk représente un radical alkyle ou alkylène,
- alk' représente un radical hydroxyalkylène,
- X représente un atome d'hydrogène ou un radical alkyle,
étant entendu que les radicaux alkyle, alkyène, alcoxy et les portions alkyle, alkylène et alcoxy contiennent 1 à 4 atomes de carbone en chaîne droite ou ramifiée, que les radicaux et portions cyclcoalkyle contiennent 3 à 6 atomes de carbone, et que les radicaux acyle contiennent 2 à 4 atomes de carbone ainsi que leurs racémiques et leurs énantiomères lorsqu'ils contiennent un ou plusieurs centres asymétriques.

2. Composés de formule (I) selon la revendication 1 pour lesquels R₅ et R₆ forment ensemble avec l'atome d'azote auquel ils sont rattachés un cycle pipéridino (éventuellement substitué par au moins un radical alkyle, phényle, alcoxycarbonyle ou dialkylcarbamoyle), un cycle perhydroazépinyl-1, indolinyl-1, tétrahydro-1,2,3,6 pyridyl-1, tétrahydro-1,2,3,4 quinolyl-1, pyrrolidinyl-1, dihydro-3,4 2H-benzoxazine-1,4 yl-4, dihydro-3,4 2H-benzothiazine-1,4 yl-4, N-alkyl tétrahydro-1,2,3,4 quinoxalinyl-1, perhydroquinoly-1, tétrahydro-1,2,3,4 isoquinolyl-2, aza-8 spiro [4,5] décanyl-8, aza-8 dioxa-1,4 spiro [4,5] décanyl-8, phényl-2 ou -3 pyrrolidinyl-1 ou thiomorpholino (éventuellement substitué par au moins un radical alkyle).

3. Composés de formule (I) selon les revendications 1 et 2 pour lesquels les atomes d'halogène sont des atomes de chlore, de brome et de fluor.

4. Composés de formule (I) selon l'une des revendications 1 à 3 pour lesquels R₁ représente un atome d'hydrogène, R₂ représente un radical alcoxy ou -NR₅R₆, R₃ représente un radical phénylamino (dont le noyau phényle est substitué par un ou plusieurs substituants choisis parmi les radicaux alkyle, monohydroxyalkyle, carboxy et -alk-COOH) et R₄ représente un radical phényle substitué par un ou plusieurs substituants choisis parmi les atomes d'halogène et les radicaux alcoxy, hydroxy, alcoxycarbonyle et -CO-NR₁₂-R₁₃.

5. Procédé de préparation des composés de formule (I) selon la revendication 1 pour lesquels R₃ représente un radical phénylamino (dont le noyau phényle est éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, -alk-O-alk, tétrazolyl-5 et tétrazolyl-5 alkyle) caractérisé en ce que l'on fait réagir un isocyanate de formule :
OCN-R₉ (II)
dans laquelle R₉ représente un radical phényle (éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, -alk-O-alk, tétrazolyl-5 et tétrazolyl-5 alkyle) sur un dérivé aminé de formule : 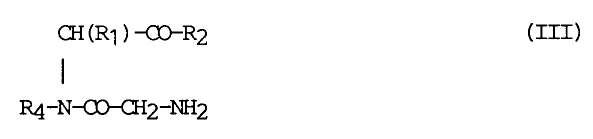 dans laquelle R₁, R₂ et R₄ ont les mêmes significations que dans la revendication 1, puis isole le produit.

6. Procédé de préparation des composés de formule (I) selon la revendication 1 pour lesquels R₃ représente un radical phénylamino (dont le noyau phényle est éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, tétrazolyl-5, tétrazolyl-5 alkyle et -alk-O-alk) caractérisé en ce que l'on fait réagir une amine de formule :
R₄-NH-CH(R₁)-CO-R₂ (VI)
dans laquelle R₁, R₂ et R₄ ont les mêmes significations que dans la revendication 1, sur un acide de formule :
HOOC-CH₂-NH-CO-R₃ (XV)
dans laquelle R₃ a les mêmes significations que ci-dessus ou un dérivé réactif de cet acide, puis isole le produit.

7. Procédé de préparation des composés de formule (I) selon la revendication 1 pour lesquels R₃ représente un radical phénylamino dont le noyau phényle est éventuellement substitué caractérisé en ce que l'on fait réagir un dérivé de formule : 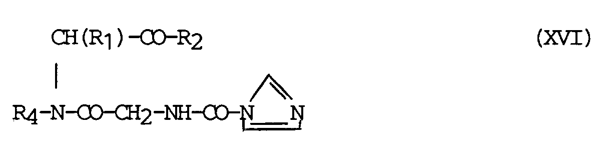 dans laquelle R₁, R₂ et R₄ ont les mêmes significations que dans la revendication 1, sur une amine de formule :
H₂N-R₁₁ (XVII)
dans laquelle R₁₁ représente un radical phényle (éventuellement substitué par un ou plusieurs substituants choisis parmi les atomes d'halogène et les radicaux alkyle, alcoxy, alkylthio, carboxy, hydroxy, mono ou polyhydroxyalkyle, nitro, amino, acyle, cyano, sulfamoyle, carbamoyle, benzoyle, alcoxycarbonyle, phénylhydroxyméthyle, pipéridino, hydroxyiminoalkyle, alcoxyiminoalkyle, alkylsulfinyle, tétrazolyl-5, tétrazolyl-5 alkyle, sulfo, -alk-O-CO-alk, -alk-O-alk, -alk-COOX, -O-alk-COOX, -alk'-COOX, -CH=CH-COOX, -CO-COOX, -alk-SO₃H et -S-alk-COOX) puis isole le produit.

8. Procédé de préparation des composés de formule (I) selon la revendication 1 pour lesquels R₃ représente un radical phénylamino (dont le noyau phényle est éventuellement substitué) caractérisé en ce que l'on fait réagir une amine de formule :
R₁₁-NH₂ (XVII)
dans laquelle R₁₁ a les mêmes significations que dans la revendication 7 sur un isocyanate de formule : 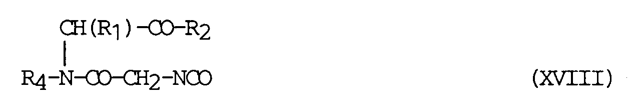 dans laquelle R₁, R₂ et R₄ ont les mêmes significations que dans la revendicaton 1, puis isole le produit.

9. Procédé de préparation des composé de formule (I) selon la revendication 1 pour lesquels R₃ représente un radical phénylamino dont le noyau phényle est substitué par au moins un radical carboxy,-alk-COOH, -O-alk-COOH, -alk'-COOH, -CH=CH-COOH, -CO-COOH ou -S-alk-COOH et/ou R₄ représente un radical phényle substitué pur un radical carboxy à l'exeception des composés comportant un radical alcoxycarbonyle caractérisé en ce que l'on hydrolyse un ester correspondant puis isole le produit.

10. Procédé de préparation des composés de formule (I) selon la revendication 1 pour lesquels R₄ représente un radical phényle substitué par un radical hydroxy à l'exception des composés comportant un radical alcoxy, alcoxycarbonyle, alkylthio, cycloalkyloxy, cycloalkylalkyloxy, phénylalkyloxy, trifluorométhoxy, cinnamyloxy, caractérisé en ce que l'on hydrolyse un composé correspondant pour lequel R₄ représente un radical phényle substitué par un radical alcoxy puis isole le produit.

11. Procédé de préparation des composés de formule (I) selon la revendication 1 pour lesquels R₃ représente un radical phényle éventuellement substitué, naphtyle, indolyle ou quinolyle, caractérisé en ce que l'on fait réagir un amine de formule : 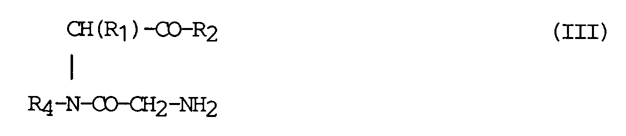 dans laquelle R₁, R₂ et R₄ ont les mêmes significations que dans la revendication 1 sur un dérivé de formule :
HOOC-R₃ (XIX)
dans laquelle R₃ a les mêmes significations que ci-dessus puis isole le produit.

12. Médicaments caractérisés en ce qu'ils contiennent en tant que principe actif au moins un composé de formule (I) selon la revendication 1.

13. Médicaments caractérisés en ce qu'ils contiennent en tant que principe actif au moins un composé selon l'une des revendications 2, 3 et 4.

14. Médicaments selon les revendications 12 et 13 pour le traitement ou la prévention de désordres liés à la CCK et à la gastrine au niveau du système nerveux et de l'appareil gastroin-testinal.

## Revendications (Revendications pour l'(les) Etat(s) contractant(s) suivant(s): ES, GR)

1. Procédé de préparation des composés de formule : 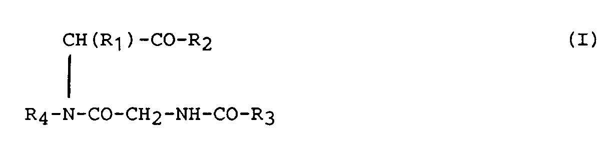 dans laquelle
- R₁ représente un atome d'hydrogène, un radical alkyle, alcoxycarbonyle ou phényle (éventuellement substitué par un ou plusieurs substituants choisis parmi les atomes d'halogène et les radicaux alkyle, alcoxy, alkylthio, nitro et amino),
- R₂ représente un radical alcoxy, cycloalkyloxy (éventuellement substitué par au moins un radical alkyle), cycloalkylalkyloxy, phénylalkyloxy, polyfluoroalkyloxy, cinnamyloxy, -NR₅R₆,
- R₃ représente un radical phénylamino (dont le noyau phényle est éventuellement substitué par un ou plusieurs substituants choisis parmi les atomes d'halogène et les radicaux alkyle, alcoxy, alkylthio, carboxy, hydroxy, mono ou polyhydroxyalkyle, nitro, amino, acyle, cyano, sulfamoyle, carbamoyle, benzoyle, alcoxycarbonyle, phénylhydroxyméthyle, pipéridino, hydroxyiminoalkyle, alcoxyiminoalkyle, alkylsulfinyle, tétrazolyl-5, tétrazolyl-5 alkyle, sulfo, -alk-O-CO-alk, -alk-O-alk, -alk-COOX, -O-alk-COOX, -alk'-COOX, -CH=CH-COOX, -CO-COOX, -alk-SO₃H et -S-alk-COOX), phényle (éventuellement substitué par un ou plusieurs substituants choisis parmi les atomes d'halogène et les radicaux alkyle, alcoxy et alkylthio), naphtyle, indolyle ou quinolyle,
- R₄ représente un radical phényle substitué par un ou plusieurs substituants choisis parmi les atomes d'halogène et les radicaux alkyle, alcoxy, hydroxy, trifluorométhoxy, trifluorométhyle, nitro, alkylthio, alcoxycarbonyle, carboxy, acylamino, méthylènedioxy, trifluorométhylthio, phénoxy, phényle, benzyle, phénylamino et -CO-NR₁₂R₁₃,
- R₅ et R₆ identiques ou différents représentent un atome d'hydrogène, un radical alkyle, phényle (éventuellement substitué par un ou plusieurs substituants choisis parmi les atomes d'halogène et les radicaux alkyle, alcoxy et alkylthio), indanyle, cycloalkylalkyle, cycloalkyle, phénylalkyle ou bien R₅ et R₆ forment ensemble avec l'atome d'azote auxquels ils sont rattachés un hétérocycle mono ou polycyclique saturé ou insaturé contenant 4 à 9 atomes de carbone et un ou plusieurs hétéroatomes (O,N,S) et éventuellement substitué par un ou plusieurs radicaux alkyle, alcoxy, alcoxycarbonyle, dialkylcarbamoyle, phényle ou en combinaison avec un atome de carbone de l'hétérocycle par un cycle spiromonocyclique à 4 ou 5 chaînons et contenant éventuellement un ou plusieurs hétéroatomes (O,S,N),
- R₁₂ représente un atome d'hydrogène ou un radical alkyle,
- R₁₃ représente un radical phényle,
ou bien NR₁₂R₁₃ représente un radical pipéridino éventuellement substitué par un radical alkyle, tétrahydro-1,2,3,4 quinolyl-1, pyrrolidinyl-1 éventuellement substitué par au moins un radical phényle ou pipérazinyl-1 éventuellement substitué en -4 par un radical alkyle,
- alk représente un radical alkyle ou alkylène,
- alk' représente un radical hydroxyalkylène,
- X représente un atome d'hydrogène ou un radical alkyle,
étant entendu que les radicaux alkyle, alkylène, alcoxy et les portions alkyle, alkylène et alcoxy contiennent 1 à 4 atomes de carbone en chaîne droite ou ramifiée, que les radicaux et portions cycloalkyle contiennent 3 à 6 atomes de carbone, et que les radicaux acyle contiennent 2 à 4 atomes de carbone ainsi que leurs racémiques et leurs énantiomères lorsqu'ils contiennent un ou plusieurs centres asymétriques,
caractérisé en ce que :
A - pour la préparation des composés pour lesquels R₃ représente un radical phénylamino (dont le noyau phényle est éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, -alk-O-alk, tétrazolyl-5 et tétrazolyl-5 alkyle) on fait réagir un isocyanate de formule :
OCN-R₉ (II)
dans laquelle R₉ représente un radical phényle (éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, -alk-O-alk, tétrazolyl-5 et tétrazolyl-5 alkyle) sur un dérivé aminé de formule : 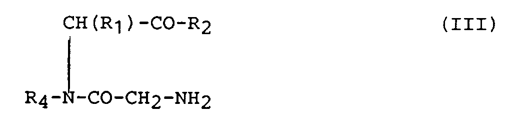 dans laquelle R₁, R₂ et R₄ ont les mêmes significations que dans la formule (I), puis isole le produit,
B - pour la préparation des composés pour lesquels R₃ représente un radical phénylamino (dont le noyau phényle est éventuellement substitué par un ou plusieurs substituants choisis parmi les atomes d'halogène et les radicaux alkyle, alcoxy, alkylthio, nitro, acyle, cyano, sulfamoyle, benzoyle, alcoxycarbonyle, tétrazolyl-5, tétrazolyl-5 alkyle et -alk-O-alk) on fait réagir une amine de formule :
R₄-NH-CH(R₁)-CO-R₂ (VI)
dans laquelle R₁, R₂ et R₄ ont les mêmes significations que dans la formule (I), sur un acide de formule :
HOOC-CH₂-NH-CO-R₃ (XV)
dans laquelle R₃ a les mêmes significations que précédemment puis isole le produit,
C - pour la préparation des composés pour lesquels R₃ représente un radical phénylamino dont le noyau phényle est éventuellement substitué, on fait réagir un dérivé de formule : 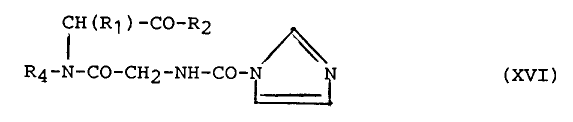 dans laquelle R₁, R₂, R₃ ont les mêmes significations que dans la formule (I), sur une amine de formule :
H₂N-R₁₁ (XVII)
dans laquelle R₁₁ représente un radical phényle (éventuellement substitué par un ou plusieurs substituants choisis parmi les atomes d'halogène et les radicaux alkyle, alcoxy, alkylthio, carboxy, hydroxy, mono ou polyhydroxyalkyle, nitro, amino, acyle, cyano, sulfamoyle, carbamoyle, benzoyle, alcoxycarbonyle, phénylhydroxyméthyle, pipéridino, hydroxyiminoalkyle, alcoxyiminoalkyle, alkylsulfinyle, sulfo, tétrazolyl-5, tétrazolyl-5 alkyle, -alk-O-CO-alk, -alk-O-alk, -alk-COOX -O-alk-COOX, -alk'-COOX, -CH=CH-COOX, -CO-COOX, -alk-SO₃H et -S-alk-COOX) puis isole le produit,
D - pour la préparation des composés pour lesquels R₃ représente un radical phénylamino dont le noyau phényle est éventuellement substitué, on fait réagir une amine de formule :
R₁₁-NH₂ (XVII)
dans laquelle R₁₁ a les mêmes significations que ci-dessus, sur un isocyanate de formule : 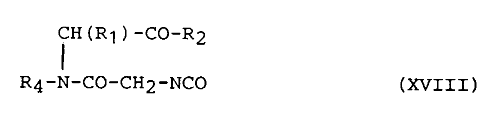 dans laquelle R₁, R₂, R₃ et R₄ ont les mêmes significations que dans la formule (I), puis isole le produit,
E - pour la préparation des composés pour lesquels R₃ représente un radical phénylamino dont le noyau phényle est substitué par au moins un radical carboxy, -alk-COOH, -O-alk-COOH, -alk'-COOH, -CH=CH-COOH, -CO-COOH, -C(=NOH)-COOH ou -S-alk-COOH et/ou R₄ représente un radical phényle substitué par un radical carboxy à l'exception des composés comportant un radical alcoxycarbonyle, on hydrolyse un ester correspondant puis isole le produit,
F - pour la préparation des composés pour lesquels R₄ représente un radical phényle substitué par un radical hydroxy à l'exception des composés comportant un radical alcoxy, alcoxycarbonyle, alkylthio, cycloalkyloxy, cycloalkylalkyloxy, phénylalkyloxy, trifluorométhoxy, cinnamyloxy, on hydrolyse un composé correspondant pour lequel R₄ représente un radical alcoxy puis isole le produit,
G - pour la préparation des composés pour lesquels R₃ représente un radical phényle éventuellement substitué, naphtyle, indolyle ou quinolyle, on fait réagir une amine de formule : 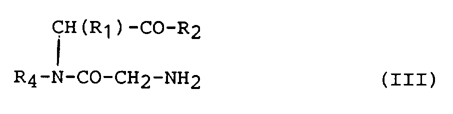 dans laquelle R₁, R₂ et R₄ ont les mêmes significations que dans la formule (I), sur un dérivé de formule :
HOOC-R₃ (XIX)
dans laquelle R₃ a les mêmes significations que précédemment puis isole le produit.

2. Procédé selon la revendication 1 pour la préparation des composés pour lesquels R₅ et R₆ forment ensemble avec l'atome d'azote auquel ils sont rattachés un cycle pipéridino (éventuellement substitué par au moins un radical alkyle, phényle, alcoxycarbonyle ou diakylcarbamoyle), un cycle perhydroazépinyl-1 indolinyl-1, tétrahydro-1,2,3,6 pyridyl-1, tétrahydro-1,2,3,4 quinolyl-1, pyrrolidinyl-1, dihydro-3,4 2H-benzoxazine-1,4, yl-4, dihydro-3,4 2H-benzothiazine-1,4 yl-4, N-alkyl tétrahydro-1,2,3,4 quinoxalinyl-1, perhydroquinolyl-1, tétrahydro-1,2,3,4 isoquinolyl-2, aza-8 spiro [4,5] décanyl-8, aza-8 dioxa-1,4 spiro [4,5] décanyl-8, phényl-2 ou -3 pyrrolidinyl-1 ou thiomorpholino (éventuellement substitué par au moins un radical alkyle).

3. Procédé selon la revendication 1 ou 2 pour la préparation des composés pour lesquels les atomes d'halogène sont des atomes de chlore, de brome ou de fluor.

4. Procédé selon l'une des revendications 1, 2, ou 3 pour la préparation des composés pour lesquels R₁ représente un atome d'hydrogène, R₂ représente un radical phénylamino (dont le noyau phényle est substitué par un ou plusieurs substituants choisis parmi les radicaux alkyle, monohydroxyalkyle, carboxy et -alk-COOH) et R₄ représente un radical phényle substitué par un ou plusieurs substituants choisis parmi les atomes d'halogène et les radicaux alcoxy, hydroxy, alcoxycarbonyle et -CO-NR₁₂R₁₃.

## Claims (Claims for the following Contracting State(s): AT, BE, CH, DE, DK, FR, GB, IT, LI, LU, NL, SE)

1. Compounds of formula: 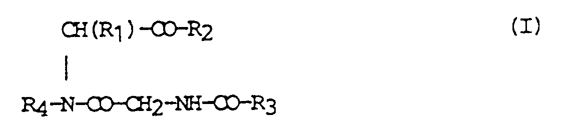 in which
- R₁ represents a hydrogen atom, an alkyl radical, an alkoxycarbonyl radical or phenyl radical (optionally substituted by one or more substituents chosen from halogen atoms and alkyl, alkoxy, alkylthio, nitro and amino radicals),
- R₂ represents an alkoxy radical, a cycloalkyloxy radical (optionally substituted by at least one alkyl radical) or a cycloalkylalkyloxy, penylalkyloxy, polyfluoroalkyloxy, cinnamyloxy, or -NR₅R₆ radical,
- R₃ represents a phenylamino radical ( whose phenyl nucleus is optionally substituted by one or more substituents chosen from halogen atoms and alkyl, alkoxy, alkylthio, carboxy, hydroxy, mono- or polyhydroxyalkyl, nitro, amino, acyl, cyano, sulphamoyl, carbamoyl, benzoyl, alkoxycarbonyl, phenylhydroxymethyl, piperidino, hydroxyiminoalkyl, alkoxyiminoalkyl, alkylsulphinyl, 5-tetrazolyl, 5-tetrazolylalkyl, sulpho, -alk-O-CO-alk, -alk-O-alk, -alk-COOX, -O-alk-COOX, -alk'-COOX, -CH=CH-COOX, CO-COOX, -alk-SO₃H and -S-alk-COOX radicals), a phenyl radical (optionally substituted by one or more substituents chosen from halogen atoms and alkyl, alkoxy and alkylthio radicals), or a naphthyl, indolyl or quinolyl radical,
- R₄ represents a phenyl radical substituted by one or more substituents chosen from halogen atoms and alkyl, alkoxy, hydroxy, trifluoromethyl, nitro, alkylthio, alkoxycarbonyl, carboxy, acylamino, methylenedioxy, trifluoromethoxy, trifluoromethylthio, phenoxy, phenyl, benzyl, phenylamino and -CO-NR₁₂R₁₃ radicals,
- R₅ and R₆, which are identical or different, represent a hydrogen atom, an alkyl radical, a phenyl radical (optionally substituted by one or more substituents chosen from halogen atoms and alkyl, alkoxy and alkylthio radicals) or an indanyl, cycloalkylalkyl, cycloalkyl or phenylalkyl radical or alternatively R₅ and R₆ form, together with the nitrogen atom to which they are attached, a saturated or unsaturated mono- or polycyclic heterocycle containing 4 to 9 carbon atoms and one or more heteroatoms (O,N,S) and optionally substituted by one or more alkyl, alkoxy, alkoxycarbonyl, dialkylcarbamoyl or phenyl radicals, or, in combination with a carbon atom of the heterocycle, by a 4 or 5-membered spiromonocyclic ring and optionally containing one or more heteroatoms (O,S,N),
- R₁₂ represents a hydrogen atom or an alkyl radical,
- R₁₃ represents a phenyl radical,
or alternatively NR₁₂R₁₃ represents a piperidino radical optionally substituted by an alkyl radical, a 1,2,3,4-tetrahydro-1-quinolyl radical, 1-pyrrolidinyl radical optionally substituted by at least one phenyl radical or 1-piperazinyl radical optionally substituted in the 4-position by an alkyl radical,
- alk represents an alkyl or alkylene radical,
- alk' represents a hydroxyalkylene radical,
- X represents a hydrogen atom or an alkyl radical, it being understood that the alkyl, alkylene and alkoxy radicals and the alkyl, alkylene and alkoxy portions contain 1 to 4 carbon atoms in a straight or branched chain, that the cycloalkyl radicals and portions contain 3 to 6 carbon atoms, and that the acyl radicals contain 2 to 4 carbon atoms, as well as their racemates and their enantiomers when they contain one or more asymmetric centres.

2. Compounds of formula (I) according to Claim 1, for which R₅ and R₆ form, together with the nitrogen atom to which they are attached, a piperidino ring (optionally substituted by at least one alkyl, phenyl, alkoxycarbonyl or dialkylcarbamoyl radical), a 1-perhydroazepinyl, 1-indolinyl, 1,2,3,6-tetrahydro-1-pyridyl, 1,2,3,4-tetrahydro-1-quinolyl, 1-pyrrolidinyl, 3,4-dihydro-2H-1,4-benzoxazin-4-yl, 3,4-dihydro-2H-1,4-benzothiazin-4-yl, N-alkyl-1,2,3,4-tetrahydro-1-quinoxalinyl, 1-perhydroquinolyl, 1,2,3,4-tetrahydro-2-isoquinolyl, 8-azaspiro[4.5]decan-8-yl, 8-aza-1,4-dioxaspiro[4.5]decan-8-yl, 2- or 3-phenyl-1-pyrrolidinyl or thiomorpholino ring (optionally substituted by at least one alkyl radical).

3. Compounds of formula (I) according to Claims 1 and 2 for which the halogen atoms are chlorine, bromine and fluorine atoms.

4. Compounds of formula (I) according to one of Claims 1 to 3 for which R₁ represents a hydrogen atom, R₂ represents an alkoxy or -NR₅R₆ radical, R₃ represents a phenylamino radical (whose phenyl nucleus is substituted by one or more substituents chosen from alkyl, monohydroxyalkyl, carboxy and -alk-COOH radicals) and R₄ represents a phenyl radical substituted by one or more substituents chosen from halogen atoms and alkoxy, hydroxy, alkoxycarbonyl and -CO-NR₁₂-R₁₃ radicals.

5. Process for preparing the compounds of formula (I) according to Claim 1 for which R₃ represents a phenylamino radical (whose phenyl nucleus is optionally substituted by one or more substituents chosen from halogen atoms and alkyl, alkoxy, alkylthio, nitro, acyl, cyano, sulphamoyl, benzoyl, alkoxycarbonyl, -alk-O-alk, 5-tetrazolyl and 5-tetrazolylalkyl radicals), characterized in that an isocyanate of formula:
OCN-R₉ (II)
in which R₉ represents a phenyl radical (optionally substituted by one or more substituents chosen from halogen atoms and alkyl, alkoxy, alkylthio, nitro, acyl, cyano, sulphamoyl, benzoyl, alkoxycarbonyl, -alk-O-alk, 5-tetrazoyl and 5-tetrazolylalkyl radicals) is reacted with an amine-containing derivative of formula: 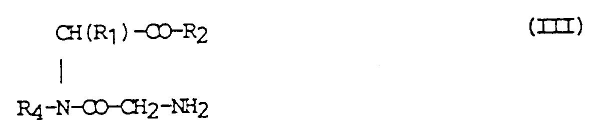 in which R₁, R₂ and R₄ have the same meanings as in Claim 1, and the product is then isolated.

6. Process for preparing the compounds of formula (I) according to Claim 1 for which R₃ represents a phenylamino radical (whose phenyl nucleus is optionally substituted by one or more substituents chosen from halogen atoms and alkyl, alkoxy, alkylthio, nitro, acyl, cyano, sulphamoyl, benzoyl, alcoxycarbonyl, 5-tetrazolyl, 5-tetrazolylalkyl and -alk-O-alk radicals) characterized in that an amine of formula:
R₄NH-CH(R₁)-CO-R₂ (VI)
in which R₁, R₂ and R₄ have the same meanings as in Claim 1, is reacted with an acid of formula:
HOOC-CH₂-NH-CO-R₃ (XV)
in which R₃ has the same meanings as above or a reactive derivative of this acid, and the product is then isolated.

7. Process for preparing the compounds of formula (I) according to Claim 1 for which R₃ represents a phenylamino radical whose phenyl nucleus is optionally substituted, characterized in that a derivative of formula: 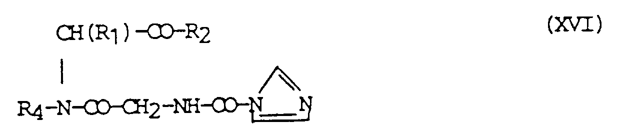 in which R₁, R₂ and R₄ have the same meanings as in Claim 1, is reacted with an amine of formula:
H₂N-R₁₁ (XVII)
in which R₁₁ represents a phenyl radical (optionally substituted by one or more substituents chosen from halogen atoms and alkyl, alkoxy, alkylthio, carboxy, hydroxy, mono- or polyhydroxyalkyl, nitro, amino, acyl, cyano, sulphamoyl, carbamoyl, benzoyl, alkoxycarbonyl, phenylhydroxymethyl, piperidino, hydroxyiminoalkyl, alkoxyiminoalkyl, alkylsulphinyl, 5,tetrazolyl, 5-tetrazolylalkyl, sulpho, -alk-O-CO-alk, -alk-O-alk, -alk-COOX, -O-alk-COOX, alk'-COOX, -CH=CH-COOX, -CO-COOX, -alk-SO₃H and -S-alk-COOX radicals), and the product is then isolated.

8. Process for preparing the compounds of formula (I) according to Claim 1, for which R₃ represents a phenylamino radical (whose phenyl nucleus is optionally substituted) characterized in that an amine of formula:
R₁₁NH₂ (XVII)
in which R₁₁ has the same meanings as in Claim 7, is reacted with an isocyanate of formula: 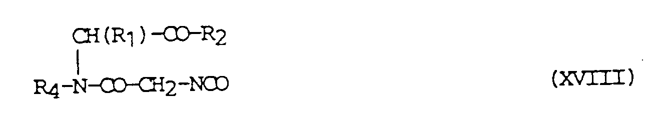 in which R₁, R₂ and R₄ have the same meanings as in Claim 1, and the product is then isolated.

9. Process for preparing the compounds of formula (I) according to Claim 1 for which R₃ represents a phenylamino radical whose phenyl nucleus is substituted by at least one carboxy,-alk-COOH, -O-alk-COOH, -alk'-COOH, -CH=CH-COOH, -CO-COOH or -S-alk-COOH radical and/or R₄ represents a phenyl radical substituted by a carboxy radical with the exception of the compounds containing an alkoxycarbonyl radical, characterized in that a corresponding ester is hydrolysed and the product is then isolated.

10. Process for preparing the compounds of formula (I) according to Claim 1 for which R₄ represents a phenyl radical substituted by a hydroxy radical with the exception of the compounds containing an alkoxy, alkoxycarbonyl, alkylthio, cycloalkyloxy, cycloalkylalkyloxy, phenylalkyloxy, trifluoromethoxy, or cinnamyloxy radical, characterized in that a corresponding compound, for which R₄ represents a phenyl radical substituted by an alkoxy radical, is hydrolysed and the product is then isolated.

11. Process for preparing the compounds of formula (I) according to Claim 1 for which R₃ represents an optionally substituted phenyl radical or a naphthyl, indolyl or quinolyl radical, characterized in that an amine of formula: 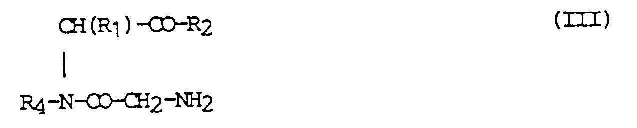 in which R₁, R₂ and R₄ have the same meanings as in Claim 1, is reacted with a derivative of formula:
HOOC-R₃ (XIX)
in which R₃ has the same meanings as above, and the product is then isolated.

12. Medicinal products characterized in that they contain, as active ingredient, at least one compound of formula (I) according to Claim 1.

13. Medicinal products characterized in that they contain, as active ingredient, at least one compound according to one of Claims 2, 3 and 4.

14. Medicinal products according to Claims 12 and 13, for the treatment or prevention of disorders linked to CCK and to gastrin in the nervous system and the gastro-intestinal apparatus.

## Claims (Claims for the following Contracting State(s): ES, GR)

1. Process for preparing the compounds of formula: 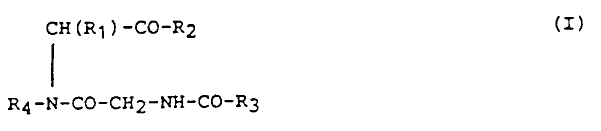 in which
- R₁ represents a hydrogen atom, an alkyl radical, an alkoxycarbonyl radical or phenyl radical (optionally substituted by one or more substituents chosen from halogen atoms and alkyl, alkoxy, alkylthio, nitro and amino radicals),
- R₂ represents an alkoxy radical, a cycloalkyloxy radical (optionally substituted by at least one alkyl radical) or a cycloalkylalkyloxy, phenylalkyloxy, polyfluoroalkyloxy, cinnamyloxy, or -NR₅R₆ radical
- R₃ represents a phenylamino radical ( whose phenyl nucleus is optionally substituted by one or more substituents chosen from halogen atoms and alkyl, alkoxy, alkylthio, carboxy, hydroxy, mono- or polyhydroxyalkyl, nitro, amino, acyl, cyano, sulphamoyl, carbamoyl, benzoyl, alkoxycarbonyl, phenylhydroxymethyl, piperidino, hydroxyiminoalkyl, alkoxyiminoalkyl, alkylsulphinyl, 5-tetrazolyl, 5-tetrazolylalkyl, sulpho, -alk-O-CO-alk, -alk-O-alk, -alk-COOX, -O-alk-COOX, -alk'-COOX, -CH=CH-COOX, CO-COOX, -alk-SO₃H and -S-alk-COOX radicals), a phenyl radical (optionally substituted by one or more substituents chosen from halogen atoms and alkyl, alkoxy and alkylthio radicals), or a naphthyl, indolyl or quinolyl radical,
- R₄ represents a phenyl radical substituted by one or more substituents chosen from halogen atoms and alkyl, alkoxy, hydroxy, trifluoromethoxy, trifluoromethyl, nitro, alkylthio, alkoxycarbonyl, carboxy, acylamino, methylenedioxy, trifluoromethylthio, phenoxy, phenyl, benzyl, phenylamino and -CO-NR₁₂R₁₃ radicals,
- R₅ and R₆, which are identical or different, represent a hydrogen atom, an alkyl radical, a phenyl radical (optionally substituted by one or more substituents chosen from halogen atoms and alkyl, alkoxy and alkylthio radicals) or an indanyl, cycloalkylalkyl, cycloalkyl or phenylalkyl radical or alternatively R₅ and R₆ from, together with the nitrogen atom to which they are attached, a saturated or unsaturated mono- or polycyclic heterocycle containing 4 to 9 carbon atoms and one or more heteroatoms (O,N,S) and optionally substituted by one or more alkyl, alkoxy, alkoxycarbonyl, dialkylcarbamoyl or phenyl radicals, or, in combination with a carbon atom of the heterocycle, by a 4 or 5-membered spiromonocyclic ring and optionally containing one or more heteroatoms (O,S,N),
- R₁₂ represents a hydrogen atom or an alkyl radical,
- R₁₃ represents a phenyl radical,
or alternatively NR₁₂R₁₃ represents a piperidino radical optionally substituted by an alkyl radical, a 1,2,3,4-tetrahydro-1-quinolyl radical, a 1-pyrrolidinyl radical optionally substituted by at least one phenyl radical or 1-piperazinyl radical optionally substituted in the 4-position by an alkyl radical,
- alk represents an alkyl or alkylene radical,
- alk' represents a hydroxyalkylene radical,
- X represents a hydrogen atom or an alkyl radical, it being understood that the alkyl, alkylene and alkoxy radicals and the alkyl, alkylene and alkoxy portions contain 1 to 4 carbon atoms in a straight or branched chain, that the cycloalkyl radicals and portions contain 3 to 6 carbon atoms, and that the acyl radicals contain 2 to 4 carbon atoms, as well as their racemates and their enantiomers when they contain one or more asymmetric centres,
characterized in that:
A - for the preparation of the compounds for which R₃ represents a phenylamino radical (whose phenyl nucleus is optionally substituted by one or more substituents chosen from halogen atoms and alkyl, alkoxy, alkylthio, nitro, acyl, cyano, sulphamoyl, benzoyl, alkoxycarbonyl, -alk-O-alk, 5-tetrazolyl and 5-tetrazolylalkyl radicals), an isocyanate of formula:
OCN-R₉ (II)
in which R₉ represents a phenyl radical (optionally substituted by one or more substituents chosen from halogen atoms and alkyl, alkoxy, alkylthio, nitro, acyl, cyano, sulphamoyl, benzoyl, alkoxycarbonyl, -alk-O-alk, 5-tetrazoyl and 5-tetrazolylalkyl radicals) is reacted with an amine-containing derivative of formula: 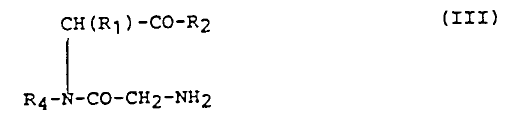 in which R₁, R₂ and R₄ have the same meanings as in formula (I), and their product is then isolated,
B - for the preparation of the compounds for which R₃ represents a phenylamino radical (whose phenyl nucleus is optionally substituted by one or more substituents chosen from halogen atoms and alkyl, alkoxy, alkylthio, nitro, acyl, cyano, sulphamoyl, benzoyl, alcoxycarbonyl, 5-tetrazolyl, 5-tetrazolylalkyl and -alk-O-alk radicals) an amine of formula:
R₄-NH-CH(R₁)-CO-R₂ (VI)
in which R₁, R₂ and R₄ have the same meanings as in formula (I), is reacted with an acid of formula:
HOOC-CH₂-NH-CO-R₃ (XV)
in which R₃ has the same meanings as above and the product is then isolated,
C - for the preparation of the compounds for which R₃ represents a phenylamino radical whose phenyl nucleus is optionally substituted, a derivative of formula: 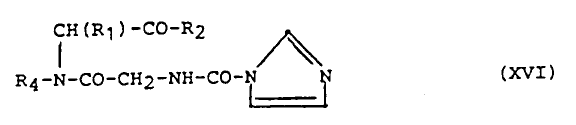 in which R₁, R₂ and R₃ have the same meanings as in formula (I), is reacted with an amine of formula:
H₂N-R₁₁ (XVII)
in which R₁₁ represents a phenyl radical (optionally substituted by one or more substituents chosen from halogen atoms and alkyl, alkoxy, alkylthio, carboxy, hydroxy, mono- or polyhydroxyalkyl, nitro, amino, acyl, cyano, sulphamoyl, carbamoyl, benzoyl, alkoxycarbonyl, phenylhydroxymethyl, piperidino, hydroxyiminoalkyl, alkoxyiminoalkyl, alkylsulphinyl, sulpho, 5-tetrazolyl, 5-tetrazolylalkyl, -alk-O-CO-alk, -alk-O-alk, -alk-COOX, -O-alk-COOX, alk'-COOX, -CH=CH-=COOX, -CO-COOX, -alk-SO₃H and -S-alk-COOX radicals), and the product is then isolated,
D - for the preparation of the compounds for which R₃ represents a phenylamino radical whose phenyl nucleus is optionally substituted, an amine of formula:
R₁₁-NH₂ (XVII)
in which R₁₁ has the same meanings as above, is reacted with an isocyanate of formula: 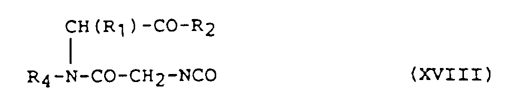 in which R₁, R₂, R₃ and R₄ have the same meanings as in formula (I), and the product is then isolated,
E - for the preparation of the compounds for which R₃ represents a phenylamino radical whose phenyl nucleus is substituted by at least one carboxy, -alk-COOH, -O-alk-COOH, -alk'-COOH, -CH=CH-COOH, -CO-COOH, -C(=NOH)-COOH or -S-alk-COOH radical and/or R₄ represents a phenyl radical substituted by a carboxy radical with the exception of the compounds containing an alkoxycarbonyl radical, a corresponding ester is hydrolysed and the product is then isolated,
F - for the preparation of the compounds for which R₄ represents a phenyl radical substituted by a hydroxy radical with the exception of the compounds containing an alkoxy, alkoxycarbonyl, alkylthio, cycloalkyloxy, cycloalkylalkyloxy, phenylalkyloxy, trifluoromethoxy, or cinnamyloxy radical, a corresponding compound, for which R₄ represents an alkoxy radical is hydrolysed and the product is then isolated,
G - for the preparation of the compounds for which R₃ represents an optionally substituted phenyl radical or a naphthyl, indolyl or quinolyl radical, an amine of formula: 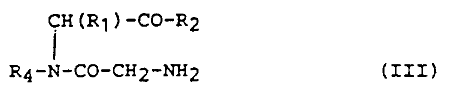 in which R₁, R₂ and R₄ have the same meanings as in formula (I), is reacted with a derivative of formula:
HOOC-R₃ (XIX)
in which R₃ has the same meanings as above, and the product is then isolated.

2. Process according to Claim 1 for the preparation of the compounds for which R₅ and R₆ form, together with the nitrogen atom to which they are attached, a piperidino ring (optionally substituted by at least one alkyl, phenyl, alkoxycarbonyl or dialkylcarbamoyl radical), a 1-perhydroazepinyl, 1-indolinyl, 1,2,3,6-tetrahydro-1-pyridyl, 1,2,3,4-tetrahydro-1-quinolyl, 1-pyrrolidinyl, 3,4-dihydro-2H-1,4-benzoxazin-4-yl, 3,4-dihydro-2H-1,4-benzothiazin-4-yl, N-alkyl-1,2,3,4-tetrahydro-1-quinoxalinyl,1-perhydroquinolyl, 1,2,3,4-tetrahydro-2-isoquinolyl, 8-azaspiro[4.5]decan-8-yl, 8-aza-1,4-dioxaspiro[4.5]decan-8-yl, 2- or 3-phenyl-1-pyrrolidinyl or thiomorpholino ring (optionally substituted by at least one alkyl radical).

3. Process according to Claim 1 or 2 for the preparation of the compounds for which the halogen atoms are chlorine, bromine or fluorine atoms.

4. Process according to one of Claims 1,2, or 3 for the preparation of the compounds for which R₁ represents a hydrogen atom, R₂ represents a phenylamino radical (whose phenyl nucleus is substituted by one or more substituents chosen from alkyl, monohydroxyalkyl, carboxy and -alk-COOH radicals) and R₄ represents a phenyl radical substituted by one or more substituents chosen from halogen atoms and alkoxy, hydroxy, alkoxycarbonyl and -CO-NR₁₂R₁₃ radicals.

## Patentansprüche (Patentansprüche für folgende(n) Vertragsstaat(en): AT, BE, CH, DE, DK, FR, GB, IT, LI, LU, NL, SE)

1. Verbindungen der Formel 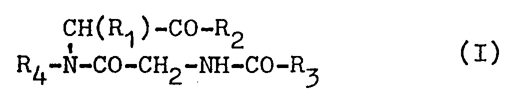 worin
R₁ für ein Wasserstoffatom, einen Alkyl-, Alkoxycarbonyl- oder Phenylrest (gegebenenfalls substituiert durch einen oder mehrere Substituenten, ausgewählt unter Halogenatomen und den Alkyl-, Alkoxy-, Alkylthio-, Nitro- und Aminogruppen) steht,
R₂ einen Alkoxy-, Cycloalkoxy- (gegebenenfalls substituiert durch zumindest einen Alkylrest), Cycloalkalkoxy-, Phenylalkoxy-, Polyfluoralkoxy-, Cinnamyloxy-Rest, einen Rest -NR₅R₆ bedeutet,
R₃ einen Phenylaminorest (dessen Phenylkern gegebenenfalls durch einen oder mehrere Substituenten, ausgewählt unter den Halogenatomen und den Alkyl-, Alkoxy-, Alkylthio-, Carboxy-, Hydroxy-, Mono- oder Polyhydroxyalkyl-, Nitro-, Amino-, Acyl-, Cyano-, Sulfamoyl-, Carbamoyl-, Benzoyl-, Alkoxycarbonyl-, Phenylhydroxymethyl-, Piperidino-, Hydroxyiminoalkyl-, Alkoxyiminoalkyl-, Alkylsulfinyl-, Tetrazol-5-yl-, Tetrazol-5-yl-alkyl-, Sulforesten, den Resten alk-O-CO-alk, -alk-O-alk, -alk-COOX, -O-alk-COOX, -alk'-COOX, -CH=CH-COOX, -CO-COOX, -alk-SO₃H und -S-alk-COOX, substituiert ist), einen Phenylrest (gegebenenfalls substituiert durch einen oder mehrere Substituenten, ausgewählt unter den Halogenatomen, den Alkyl-, Alkoxy- und Alkylthioresten), einen Naphthyl-, Indolyl- oder Chinolylrest bedeutet,
R₄ einen Phenylrest, der durch einen oder mehrere Substituenten, ausgewählt unter den Halogenatomen und den Alkyl-, Alkoxy-, Hydroxy-, Trifluormethyl-, Nitro-, Alkylthio-, Alkoxycarbonyl-, Carboxy-, Acylamino-, Methylendioxy-, Trifluormethoxy-, Trifluormethylthio-, Phenoxy-, Phenyl-, Benzyl-, Phenylaminoresten und dem Rest -CO-NR₁₂R₁₃, substituiert ist, bedeutet,
R₅ und R₆, identisch oder verschieden, ein Wasserstoffatom, einen Alkyl-, Phenyl- (gegebenenfalls substituiert durch einen oder mehrere Substituenten, ausgewählt unter den Halogenatomen und den Alkyl-, Alkoxy- und Alkylthioresten), Indanyl-, Cycloalkalkyl-, Cycloalkyl-, Phenylalkylrest bedeuten oder R₅ und R₆ gemeinsam mit dem Stickstoffatom, an das sie gebunden sind, einen gesättigten oder ungesättigten, mono- oder polycyclischen Heterocyclus mit 4 bis 9 Kohlenstoffatomen und einem oder mehreren Heteroatomen (O, N, S) bilden, der gegebenenfalls durch einen oder mehrere Alkyl-, Alkoxy-, Alkoxycarbonyl-, Dialkylcarbamoyl-, Phenylreste oder in Kombination mit einem Kohlenstoffatom des Heterocyclus durch einen spiromonocyclischen Ring mit 4 oder 5 Gliedern, der gegebenenfalls ein oder mehrere Heteroatome (O, S, N) aufweist, substituiert ist,
R₁₂ ein Wasserstoffatom oder einen Alkylrest bedeutet,
R₁₃ einen Phenylrest bedeutet,
oder NR₁₂R₁₃ einen Piperidino-, gegebenenfalls substituiert durch einen Alkylrest, 1,2,3,4-Tetrahydro-chinol-1-yl-, pyrrolidine-1-yl, gegebenenfalls substituiert durch zumindest einen Phenylrest, oder Piperazin-1-yl-Rest, gegebenenfalls substituiert in 4-Stellung durch einen Alkylrest, bedeutet,
alk für einen Alkyl- oder Alkylenrest steht,
alk' für einen Hydroxyalkylenrest steht,
X ein Wasserstoffatom oder einen Alkylrest bedeutet,
mit der Maßgabe, daß die Alkyl-, Alkylen-, Alkoxyreste und die Alkyl-, Alkylen- und Alkoxyteile 1 bis 4 Kohlenstoffatome in gerader oder verzweigter Kette aufweisen, daß die Cycloalkylreste und -teile 3 bis 6 Kohlenstoffatome besitzen und daß die Acylreste 2 bis 4 Kohlenstoffatome besitzen, sowie deren racemische Formen und deren Enantiomere, wenn sie ein oder mehrere Asymmetriezentren aufweisen.

2. Verbindungen der Formel (I) gemäß Anspruch 1, worin R₅ und R₆ mit dem Stickstoffatom, an das sie gebunden sind, einen Piperidinoring (gegebenenfalls substituiert durch zumindest einen Alkyl-, Phenyl-, Alkoxycarbonyl- oder Dialkylcarbamoylrest), einen Perhydroazepin -1-yl-, Indolin-1-yl-, 1,2,3,6-Tetrahydro-pyrid-1-yl-, 1,2,3,4-Tetrahydro-chinol-1-yl-, Pyrrolidin-1-yl-, 3,4-Dihydro-2H-1,4-benzoxazin-4-yl-, 3,4-Dihydro-2H-1,4-benzothiazin-4-yl-, N-Alkyl-1,2,3,4-tetrahydro-chinoxalin-1-yl-, Perhydrochinol-1-yl-, 1,2,3,4-Tetrahydro-isochinol-2-yl-, 8-Aza-spiro[4.5]-decan-8-yl-, 8-Aza-1,4-dioxa-spiro[4,5] -decan-8-yl-, 2- oder 3-Phenylpyrrolidin-1-yl- oder Thiomorpholinoring (gegebenenfalls substituiert durch zumindest einen Alkylrest) bilden.

3. Verbindungen der Formel (I) gemäß Anspruch 1 und 2, worin die Halogenatome Chlor-, Brom- und Fluoratome sind.

4. Verbindungen der Formel (I) gemäß einem der Ansprüche 1 bis 3, worin R₁ ein Wasserstoffatom bedeutet, R₂ einen Alkoxyrest oder einen Rest -NR₅R₆ wiedergibt, R₃ für einen Phenylaminorest steht (dessen Phenylkern durch einen oder mehrere Substituenten, ausgewählt unter den Alkyl-, Monohydroxyalkyl-, Carboxy-Resten und dem Rest -alk-COOH, substituiert ist) und R₄ einen Phenylrest darstellt, der durch einen oder mehrere Substituenten, ausgewählt unter den Halogenatomen und den Alkoxy-, Hydroxy-, Alkoxycarbonylresten und dem Rest -CO-NR₁₂R₁₃, substituiert ist.

5. Verfahren zur Herstellung der Verbindungen der Formel (I) gemäß Anspruch 1, worin R₃ einen Phenylaminorest bedeutet (dessen Phenylkern gegebenenfalls durch einen oder mehrere Substituenten, ausgewählt unter den Halogenatomen und den Alkyl, Alkoxy-, Alkylthio-, Nitro-, Acyl-, Cyano-, Sulfamoyl-, Benzoyl-, Alkoxycarbonylresten, dem Rest -alk-O-alk, dem Tetrazol-5-yl- und dem Tetrazol-5-yl-alkylrest, substituiert ist), dadurch gekennzeichnet, daß man ein Isocyanat der Formel
OCN-R₉ (II)
worin R₉ einen Phenylrest (gegebenenfalls substituiert durch einen oder mehrere Substituenten, ausgewählt unter den Halogenatomen und den Alkyl-, Alkoxy-, Alkylthio-, Nitro-, Acyl-, Cyano-, Sulfamoyl-, Benzoyl-, Alkoxycarbonylresten, dem Rest -alk-O-alk, dem Tetrazol-5-yl- und dem Tetrazol-5-yl-alkylrest) darstellt, mit einem Aminoderivat der Formel 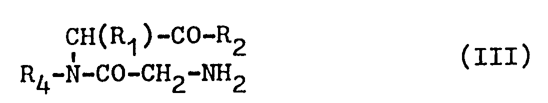 worin R₁, R₂ und R₄ die in Anspruch 1 angegebenen Bedeutungen besitzen, umsetzt und danach das Produkt isoliert.

6. Verfahren zur Herstellung der Verbindungen der Formel (I) gemäß Anspruch 1, worin R₃ einen Phenylaminorest bedeutet (dessen Phenylkern gegebenenfalls durch einen oder mehrere Substituenten, ausgewählt unter den Halogenatomen und den Alkyl-, Alkoxy-, Alkylthio-, Nitro-, Acyl-, Cyano-, Sulfamoyl-, Benzoyl-, Alkoxycarbonyl-, Tetrazol-5-yl-, Tetrazol-5-yl-alkylresten und dem Rest -alk-O-alk, substituiert ist), dadurch gekennzeichnet, daß man ein Amin der Formel
R₄-NH-CH(R₁)-CO-R₂ (VI)
worin R₁, R₂ und R₄ die in Anspruch 1 angegebenen Bedeutungen besitzen, mit einer Säure der Formel
HOOC-CH₂-NH-CO-R₃ (XV)
worin R₃ die vorstehend angegebenen Bedeutungen besitzt, oder einem reaktiven Derivat dieser Säure umsetzt und nachfolgend das Produkt isoliert.

7. Verfahren zur Herstellung der Verbindungen der Formel (I) gemäß Anspruch 1, worin R₃ einen Phenylaminorest bedeutet, dessen Phenylkern gegebenenfalls substituiert ist, dadurch gekennzeichnet, daß man ein Derivat der Formel 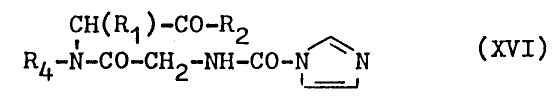 worin R₁, R₂ und R₄ die in Anspruch 1 angegebenen Bedeutungen besitzen, mit einem Amin der Formel
H₂N-R₁₁ (XVII)
worin R₁₁ einen Phenylrest (gegebenenfalls substituiert durch einen oder mehrere Substituenten, ausgewählt unter den Halogenatomen und den Alkyl-, Alkoxy-, Alkylthio-, Carboxy-, Hydroxy-, Mono- oder Poly-hydroxyalkyl-, Nitro-, Amino-, Acyl-, Cyano-, Sulfamoyl-, Carbamoyl-, Benzoyl-, Alkoxycarbonyl-, Phenylhydroxymethyl-, Piperidino-, Hydroxyiminoalkyl-, Alkoxyiminoalkyl-, Alkylsulfinyl-, Tetrazol-5-yl-, Tetrazol-5-yl-alkyl-, Sulforesten, den Resten -alk-O-CO-alk, -alk-O-alk, -alk-COOX, -O-alk-COOX, -alk'-COOX, -CH=CH-COOX, -CO-COOX, -alk-SO₃H und -S-alk-COOX) bedeutet, umsetzt und dann das Produkt isoliert.

8. Verfahren zur Herstellung der Verbindungen der Formel (I) gemäß Anspruch 1, worin R₃ einen Phenylaminorest bedeutet (dessen Phenylkern gegebenenfalls substituiert ist), dadurch gekennzeichnet, daß man ein Amin der Formel
R₁₁-NH₂ (XVII)
worin R₁₁ die in Anspruch 7 angegebenen Bedeutungen besitzt, mit einem Isocyanat der Formel 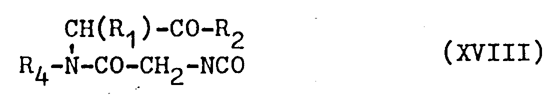 worin R₁, R₂ und R₄ die in Anspruch 1 angegebenen Bedeutungen besitzen, umsetzt und dann das Produkt isoliert.

9. Verfahren zur Herstellung der Verbindungen der Formel (I) gemäß Anspruch 1, worin R₃ einen Phenylaminorest bedeutet, dessen Phenylkern durch zumindest einen Carboxyrest, einen Rest -alk-COOH, -O-alk-COOH, -alk'-COOH, -CH=CH-COOH, -CO-COOH oder -S-alk-COOH substituiert ist, und/oder R₄ eine durch einen Carboxyrest substituierte Phenylgruppe bedeutet, mit Ausnahme der einen Alkoxycarbonylrest aufweisenden Verbindungen, dadurch gekennzeichnet, daß man einen entsprechenden Ester hydrolysiert und dann das Produkt isoliert.

10. Verfahren zur Herstellung der Verbindungen der Formel (I) gemäß Anspruch 1, worin R₄ einen durch einen Hydroxyrest substituierten Phenylrest bedeutet, mit Ausnahme der Verbindungen, die einen Alkoxy-, Alkoxycarbonyl-, Alkylthio-, Cycloalkoxy-, Cycloalkalkoxy-, Phenylalkoxy-, Trifluormethoxy-, Cinnamyloxy -Rest aufweisen, dadurch gekennzeichnet, daß man eine entsprechende Verbindung, worin R₄ einen durch einen Alkoxyrest substituierten Phenylrest bedeutet, hydrolysiert und dann das Produkt isoliert.

11. Verfahren zur Herstellung der Verbindungen der Formel (I) gemäß Anspruch 1, worin R₃ einen Phenylrest, der gegebenenfalls substituiert ist, einen Naphthyl-, Indolyl- oder Chinolylrest bedeutet, dadurch gekennzeichnet, daß man ein Amin der Formel 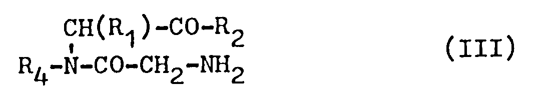 worin R₁, R₂ und R₄ die in Anspruch 1 angegebenen Bedeutungen besitzen, mit einem Derivat der Formel
HOOC-R₃ (XIX)
worin R₃ die vorstehend angegebenen Bedeutungen besitzt, umsetzt und dann das Produkt isoliert.

12. Arzneimittel, dadurch gekennzeichnet, daß sie als Wirkstoff zumindest eine Verbindung der Formel (I) gemäß Anspruch 1 enthalten.

13. Arzneimittel, dadurch gekennzeichnet, daß sie als Wirkstoff zumindest eine Verbindung gemäß einem der Ansprüche 2,3 und 4 enthalten.

14. Arzneimittel gemäß Anspruch 12 und 13 zur Behandlung oder Verhütung von mit dem CCK und dem Gastrin verbundenen Störungen im Bereich des Nervensystems und des Gastrointestinaltrakts.

## Patentansprüche (Patentansprüche für folgende(n) Vertragsstaat(en): ES, GR)

1. Verfahren zur Herstellung der Verbindungen der Formel 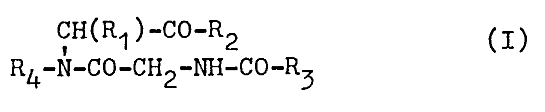 worin
R₁ für ein Wasserstoffatom, einen Alkyl-, Alkoxycarbonyl- oder Phenylrest (gegebenenfalls substituiert durch einen oder mehrere Substituenten, ausgewählt unter Halogenatomen und den Alkyl-, Alkoxy-, Alkylthio-, Nitro- und Aminogruppen) steht,
R₂ einen Alkoxy-, Cycloalkoxy- (gegebenenfalls substituiert durch zumindest einen Alkylrest), Cycloalkalkoxy-, Phenylalkoxy-, Polyfluoralkoxy-, Cinnamyloxy-Rest, einen Rest -NR₅R₆ bedeutet,
R₃ einen Phenylaminorest (dessen Phenylkern gegebenenfalls durch einen oder mehrere Substituenten, ausgewählt unter den Halogenatomen und den Alkyl-, Alkoxy-, Alkylthio-, Carboxy-, Hydroxy-, Mono- oder Polyhydroxyalkyl-, Nitro-, Amino-, Acyl-, Cyano-, Sulfamoyl-, Carbamoyl-, Benzoyl-, Alkoxycarbonyl-, Phenylhydroxymethyl-, Piperidino-, Hydroxyiminoalkyl-, Alkoxyiminoalkyl-, Alkylsulfinyl-, Tetrazol-5-yl-, Tetrazol-5-yl-alkyl-, Sulforesten, den Resten alk-O-CO-alk, -alk-O-alk, -alk-COOX, -O-alk-COOX, -alk'-COOX, -CH=CH-COOX, -CO-COOX, -alk-SO₃H und -S-alk-COOX, substituiert ist), einen Phenylrest (gegebenenfalls substituiert durch einen oder mehrere Substituenten, ausgewählt unter den Halogenatomen, den Alkyl-, Alkoxy- und Alkylthioresten), einen Naphthyl-, Indolyl- oder Chinolylrest bedeutet,
R₄ einen Phenylrest, der durch einen oder mehrere Substituenten, ausgewählt unter den Halogenatomen und den Alkyl-, Alkoxy-, Hydroxy-, Trifluormethoxy-, Trifluormethyl-, Nitro-, Alkylthio-, Alkoxycarbonyl-, Carboxy-, Acylamino-, Methylendioxy-, Trifluormethylthio-, Phenoxy-, Phenyl-, Benzyl-, Phenylaminoresten und dem Rest -CO-NR₁₂R₁₃, substituiert ist, bedeutet,
R₅ und R₆, identisch oder verschieden, ein Wasserstoffatom, einen Alkyl-, Phenyl- (gegebenenfalls substituiert durch einen oder mehrere Substituenten, ausgewählt unter den Halogenatomen und den Alkyl-, Alkoxy- und Alkylthioresten), Indanyl-, Cycloalkalkyl-, Cycloalkyl-, Phenylalkylrest bedeuten oder R₅ und R₆ gemeinsam mit dem Stickstoffatom, an das sie gebunden sind, einen gesättigten oder ungesättigten, mono- oder polycyclischen Heterocyclus mit 4 bis 9 Kohlenstoffatomen und einem oder mehreren Heteroatomen (O, N, S) bilden, der gegebenenfalls durch einen oder mehrere Alkyl-, Alkoxy-, Alkoxycarbonyl-, Dialkylcarbamoyl-, Phenylreste oder in Kombination mit einem Kohlenstoffatom des Heterocyclus durch einen spiromonocyclischen Ring mit 4 oder 5 Gliedern, der gegebenenfalls ein oder mehrere Heteroatome (O, S, N) aufweist, substituiert ist,
R₁₂ ein Wasserstoffatom oder einen Alkylrest bedeutet,
R₁₃ einen Phenylrest bedeutet,
oder NR₁₂R₁₃ einen Piperidino-, gegebenenfalls substituiert durch einen Alkylrest, 1,2,3,4-Tetrahydro-chinol-1-yl-, Pyrrolidin-1-yl-, gegebenenfalls substituiert durch zumindest einen Phenylrest; oder Piperazin-1-yl-Rest, gegebenenfalls substituiert in 4-Stellung durch einen Alkylrest, bedeutet,
alk für einen Alkyl- oder Alkylenrest steht,
alk' für einen Hydroxyalkylenrest steht,
X ein Wasserstoffatom oder einen Alkylrest bedeutet,
mit der Maßgabe, daß die Alkyl-, Alkylen-, Alkoxyreste und die Alkyl-, Alkylen- und Alkoxyteile 1 bis 4 Kohlenstoffatome in gerader oder verzweigter Kette aufweisen, daß die Cycloalkylreste und -teile 3 bis 6 Kohlenstoffatome besitzen und daß die Acylreste 2 bis 4 Kohlenstoffatome besitzen, sowie von deren racemischen Formen und deren Enantiomeren, wenn sie ein oder mehrere Asymmetriezentren aufweisen, dadurch gekennzeichnet, daß man
A - zur Herstellung der Verbindungen, worin R₃ einen Phenylaminorest bedeutet (dessen Phenylkern gegebenenfalls durch einen oder mehrere Substituenten, ausgewählt unter den Halogenatomen und den Alkyl-, Alkoxy-, Alkylthio-, Nitro-, Acyl-, Cyano-, Sulfamoyl-, Benzoyl-, Alkoxycarbonyl-, -alk-O-alk-, Tetrazol-5-yl- und Tetrazol-5-yl-alkylresten, substituiert ist), ein Isocyanat der Formel
OCN-R₉ (II)
worin R₉ einen Phenylrest bedeutet (gegebenenfalls substituiert durch einen oder mehrere Substituenten, ausgewählt unter den Halogenatomen und den Alkyl-, Alkoxy-, Alkylthio-, Nitro-, Acyl-, Cyano-, Sulfamoyl-, Benzoyl-, Alkoxycarbonyl-, -alk-O-alk-, Tetrazol-5-yl- und Tetrazol-5-yl-alkylresten), mit einem Aminoderivat der Formel 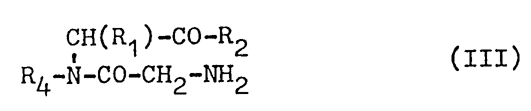 umsetzt, worin R₁, R₂ und R₄ die für Formel (I) angegebenen Bedeutungen besitzen, und anschließend das Produkt isoliert,
B - zur Herstellung der Verbindungen, worin R₃ einen Phenylaminorest bedeutet (dessen Phenylkern gegebenenfalls durch einen oder mehrere Substituenten, ausgewählt unter den Halogenatomen und den Alkyl-, Alkoxy-, Alkylthio-, Nitro-, Acyl-, Cyano-, Sulfamoyl-, Benzoyl-, Alkoxycarbonyl-, Tetrazol-5-yl-, Tetrazol-5-yl-alkyl- und -alk-O-alk-Resten, substituiert ist), ein Amin der Formel
R₄-NH-CH(R₁)-CO-R₂ (VI)
worin R₁, R₂ und R₄ die für Formel (I) angegebenen Bedeutungen besitzen, mit einer Säure der Formel
HOOC-CH₂-NH-CO-R₃ (XV)
worin R₃ die vorstehend angegebenen Bedeutungen besitzt, umsetzt, wonach man das Produkt isoliert,
C - zur Herstellung der Verbindungen, worin R₃ einen Phenylaminorest bedeutet, dessen Phenylkern gegebenenfalls substituiert ist, ein Derivat der Formel 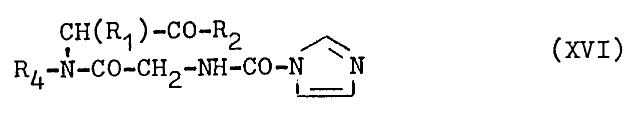 worin R₁, R₂, R₃ die für Formel (I) angegebenen Bedeutungen besitzen, mit einem Amin der Formel
H₂N-R₁₁ (XVII)
umsetzt, worin R₁₁ einen Phenylrest (gegebenenfalls substituiert durch einen oder mehrere Substituenten, ausgewählt unter den Halogenatomen und den Alkyl-, Alkoxy-, Alkylthio-, Carboxy-, Hydroxy-, Mono- oder Polyhydroxyalkyl-, Nitro-, Amino-, Acyl-, Cyano-, Sulfamoyl-, Carbamoyl-, Benzoyl-, Alkoxycarbonyl-, Phenylhydroxymethyl-, Piperidino-, Hydroxyiminoalkyl-, Alkoxyiminoalkyl-, Alkylsulfinyl-, Sulfo-, Tetrazol-5-yl-, Tetrazol-5-yl-alkylresten, den Resten -alk-O-CO-alk, -alk-O-alk, -alk-COOX, -O-alk-COOX, -alk'-COOX, -CH=CH-COOX, -CO-COOX, -alk-SO₃H und -S-alk-COOX) bedeutet, und danach das Produkt isoliert,
D - zur Herstellung der Verbindungen, worin R₃ einen Phenylaminorest bedeutet, dessen Phenylkern gegebenenfalls substituiert ist, ein Amin der Formel
R₁₁-NH₂ (XVII)
worin R₁₁ die vorstehend angegebenen Bedeutungen besitzt, mit einem Isocyanat der Formel 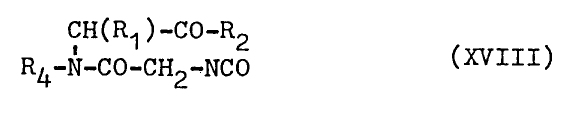 worin R₁, R₂, R₃ und R₄ die für Formel (I) angegebenen Bedeutungen besitzen, umsetzt und danach das Produkt isoliert,
E - zur Herstellung der Verbindungen, worin R₃ einen Phenylaminorest bedeutet, dessen Phenylkern durch zumindest einen Carboxyrest, einen Rest -alk-COOH, -O-alk-COOH, -alk'-COOH, -CH=CH-COOH, -CO-COOH, -C(=NOH)-COOH oder -S-alk-COOH substituiert ist und/oder R₄ einen durch einen Carboxyrest substituierten Phenylrest bedeutet, mit Ausnahme der Verbindungen, die einen Alkoxycarbonylrest aufweisen, einen entsprechenden Ester hydrolysiert und danach das Produkt isoliert,
F - zur Herstellung der Verbindungen, worin R₄ einen durch eine Hydroxygruppe substituierten Phenylrest bedeutet, mit Ausnahme der Verbindungen, die einen Alkoxy-, Alkoxycarbonyl-, Alkylthio-, Cycloalkoxy-, Cycloalkalkoxy-, Phenylalkoxy-, Trifluormethoxy-, Cinnamyloxy-Rest aufweisen, eine entsprechende Verbindung, worin R₄ einen Alkoxyrest bedeutet, hydrolysiert und anschließend das Produkt isoliert,
G - zur Herstellung der Verbindungen, worin R₃ einen gegebenenfalls substituierten Phenylrest, einen Naphthyl-, Indolyl- oder Chinolylrest bedeutet, ein Amin der Formel 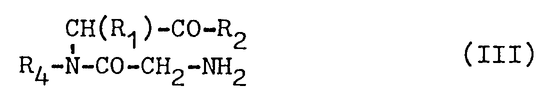 worin R₁, R₂ und R₄ die für Formel (I) angegebenen Bedeutungen besitzen, mit einem Derivat der Formel
HOOC-R₃ (XIX)
worin R₃ die vorstehend angegebenen Bedeutungen besitzt, umsetzt und danach das Produkt isoliert.

2. Verfahren gemäß Anspruch 1 zur Herstellung der Verbindungen, worin R₅ und R₆ gemeinsam mit dem Stickstoffatom, an das sie gebunden sind, einen Piperidinoring (gegebenenfalls substituiert durch zumindest einen Alkyl-, Phenyl-, Alkoxycarbonyl- oder Dialkylcarbamoylrest), einen Perhydroazepin-1-yl-, Indolin-1-yl-, 1,2,3,6-Tetrahydro-pyrid-1-yl-, 1,2,3,4-Tetrahydro-chinol-1-yl-, Pyrrolidin-1-yl-, 3,4-Dihydro-2H-1,4-benzoxazin-4-yl-, 3,4-Dihydro-2H-1,4-benzothiazin-4-yl-, N-Alkyl-1,2,3,4-tetrahydro-chinoxalin-1-yl-, Perhydrochinol-1-yl-, 1,2,3,4-Tetrahydro-isochinol-2-yl-, 8-Aza-spiro[4.5]-decan-8-yl-, 8-Aza-1,4-dioxa-spiro[4.5]-decan-8-yl-, 2- oder 3-Phenylpyrrolidin-1-yl- oder Thiomorpholinoring (gegebenenfalls substituiert durch zumindest einen Alkylrest) bilden.

3. Verfahren gemäß Anspruch 1 oder 2 zur Herstellung der Verbindungen, worin die Halogenatome Chlor-, Brom- oder Fluoratome sind.

4. Verfahren gemäß einem der Ansprüche 1, 2 oder 3 zur Herstellung der Verbindungen, worin R₁ ein Wasserstoffatom bedeutet, R₂ einen Phenylaminorest (dessen Phenylkern durch einen oder mehrere Substituenten, ausgewählt unter den Alkyl-, Monohydroxyalkyl-, Carboxyresten und dem Rest -alk-COOH, substituiert ist) bedeutet und R₄ einen Phenylrest, substituiert durch einen oder mehrere Substituenten, ausgewählt unter den Halogenatomen und den Alkoxy-, Hydroxy-, Alkoxycarbonylresten und dem Rest -CO-NR₁₂R₁₃, darstellt.
